(19) Europäisches Patentamt / European Patent Office / Office européen des brevets

(11) **EP 4 198 102 B1**

(12) **EUROPEAN PATENT SPECIFICATION**

(45) Date of publication and mention of the grant of the patent:
**01.04.2026 Bulletin 2026/14**

(21) Application number: **21911104.4**

(22) Date of filing: **24.12.2021**

(51) International Patent Classification (IPC):
***C09K 9/02*** *(2006.01)* ***G02C 7/10*** *(2006.01)*
***C07D 311/78*** *(2006.01)* ***G02B 1/04*** *(2006.01)*
***G02B 5/23*** *(2006.01)* ***C07D 311/94*** *(2006.01)*
***C07D 407/04*** *(2006.01)* ***C07D 409/04*** *(2006.01)*
***C07D 497/04*** *(2006.01)* ***C07F 7/04*** *(2006.01)*

(52) Cooperative Patent Classification (CPC):
(C-Sets available)
**C07D 311/94; C07D 407/04; C07D 409/04; C07D 497/04; C07F 7/04; C09K 9/02; G02B 1/041; G02B 5/23;** G02C 7/102 (Cont.)

(86) International application number:
**PCT/JP2021/048400**

(87) International publication number:
**WO 2022/138967 (30.06.2022 Gazette 2022/26)**

# (54) PHOTOCHROMIC COMPOUND, PHOTOCHROMIC COMPOSITION, PHOTOCHROMIC ARTICLE, AND EYEGLASSES

PHOTOCHROME VERBINDUNG, PHOTOCHROME ZUSAMMENSETZUNG, PHOTOCHROMER ARTIKEL UND BRILLE

COMPOSÉ PHOTOCHROMIQUE, COMPOSITION PHOTOCHROMIQUE, ARTICLE PHOTOCHROMIQUE ET LUNETTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2020 JP 2020215158**
**24.12.2020 JP 2020215159**
**08.02.2021 JP 2021018610**

(43) Date of publication of application:
**21.06.2023 Bulletin 2023/25**

(60) Divisional application:
**26159718.1**

(73) Proprietor: **HOYA LENS THAILAND LTD.**
**Pathumthani 12130 (TH)**

(72) Inventors:
- **KAWAKAMI, Hironori**
**Tokyo 160-8347 (JP)**
- **KOBAYASHI, Kei**
**Tokyo 160-8347 (JP)**
- **MATSUE, Aoi**
**Tokyo 160-8347 (JP)**
- **SHIMADA, Takuya**
**Tokyo 160-8347 (JP)**
- **YAMASHITA, Teruo**
**Tokyo 160-8347 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 479 171**
**WO-A1-2005/028465**
**WO-A1-2011/053615**
**WO-A1-2013/078086**
**WO-A1-2014/151543**
**JP-A- 2003 513 017**
**JP-A- 2012 092 349**
**JP-A- 2016 053 163**
**JP-A- 2018 097 173**
**US-A1- 2012 145 973**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**G02B 1/041, C08L 33/10, C08K 5/0041**

## Description

[Technical Field]

The present invention relates to a photochromic compound, a photochromic composition, a photochromic article and eyeglasses.

[Background Art]

A photochromic compound is a compound having a property of coloring under emission of light in a wavelength range having photoresponsivity and fading without light emission (photochromic properties). For example, WO 2000/15631 discloses a naphthopyran compound having photochromic properties.

EP-A-2 479 171 discloses photochromic chromene compounds having an indeno(2,1-f)naphtho(1,2-b)pyran structure as its basic skeleton, an aryl group or a heteroaryl group at the 6-position carbon atom of the structure and an electron donor group having a Hammett constant $\sigma_p$ of not more than -0.1 at the 7-position carbon atom.

WO 2013/078086 describes photochromic compounds of a specified general formula, which include at least two photochromic moieties that are linked together by a multivalent linking group which can be selected so as to be flexible and/or substantially prevent electronic interaction between any two photochromic moieties through the multivalent linking group.

[Summary of Invention]

[Technical Problem]

Examples of methods of imparting photochromic properties to optical articles such as spectacle lenses include a method of incorporating a photochromic compound into a substrate and a method of forming a layer containing a photochromic compound. Examples of properties desired for optical articles to which photochromic properties are imparted in this manner include exhibiting a high coloring concentration in a visible range (a wavelength of 380-780 nm) during coloring and exhibiting a fast fade rate after coloring by light emission.

An object of one aspect of the present invention is to provide a photochromic article with a high coloring concentration in a visible range during coloring and a high fade rate.

[Solution to Problem]

The present invention provides a photochromic compound of formula (1):

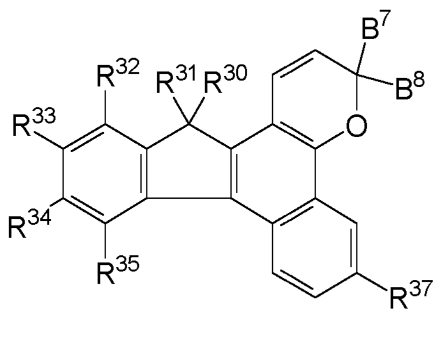

(1)

wherein

$R^{30}$ and $R^{31}$ each independently are selected from linear or branched $C_{2-10}$-alkyl, each optionally substituted,
$R^{32}$-$R^{35}$ each independently are H or a substituent other than an electron-attracting group,
$R^{37}$ is H or an electron-donating group selected from methoxy, ethoxy, phenoxy, methylsulfide, phenylsulfide, dimethylamino, pyrrolidino, piperidino, morpholino and thiomorpholino, and $B^7$ and $B^8$ each independently are H or a substituent.

Also, the present invention provides a photochromic compound of formula (1a):

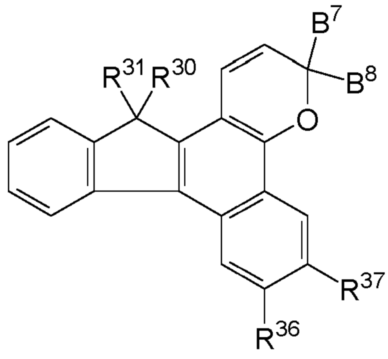

(1a)

wherein

$R^{30}$ and $R^{31}$ each independently are selected from linear or branched $C_{\geq 2}$-alkyl, $C_{\geq 3}$-cycloalkyl and $-(R^{100})_nR^{101}$ wherein $R^{100}$ is alkyleneoxy, $R^{101}$ is alkyl, n is an integer of ≥ 1; each optionally substituted,
$R^{36}$ and $R^{37}$ each independently are an electron-donating group, and
$B^7$ and $B^8$ each independently are H or a substituent.

In addition, the present invention provides a photochromic composition comprising one or more of the photochromic compounds of the present invention.

Yet further, the present invention provides a photochromic composition comprising one or more photochromic compounds of formula (1b):

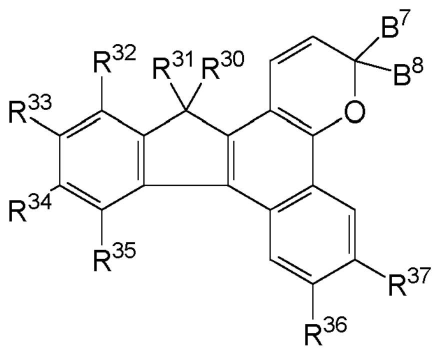

(1b)

wherein $R^{30}$ and $R^{31}$ both are ethyl, $R^{32}$-$R^{35}$ each independently are H or a substituent other than an electron-attracting group, and one of $R^{36}$ and $R^{37}$ is an electron-donating group, and the other is H or an electron-donating group, and one or more photochromic compounds of Formula (A):

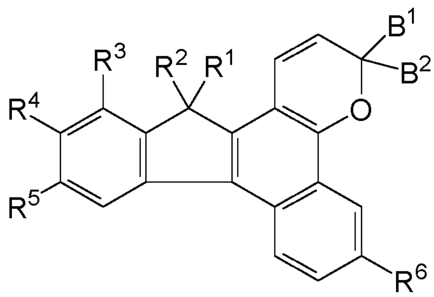

(A)

wherein $R^1$-$R^6$, $B^1$ and $B^2$ each independently are H or a substituent.

Even further, the present invention provides a photochromic article comprising a cured product obtained by curing a composition of the present invention.

Preferred embodiments of the invention are as defined in the appended dependent claims and/or in the following

detailed description.

The present photochromic compounds can be colored with a high concentration in a visible range during coloring by light emission and can exhibit a fast fade rate. According to the present photochromic compounds, it is possible to provide a photochromic article with a high coloring concentration in a visible range during coloring and a high fade rate.

[Advantageous Effects of Invention]

According to one aspect of the present invention, it is possible to provide a photochromic article with a high coloring concentration in a visible range during coloring and a high fade rate.

[Brief Description of Drawings]

[Fig. 1]
Fig. 1 shows solution spectrums obtained for a compound of Example 3.
[Fig. 2]
Fig. 2 is a graph showing the absorbance assuming an integral rate formula of a first-order reaction in order to calculate a reaction rate constant for each of a compound of Example 3 and a compound of a comparative example, that is, the change in concentration of a colored component in the system over time.

[Description of Embodiments]

As an example, a photochromic compound undergoes structural conversion into a colored component through an excited state when it receives light such as sunlight. The structure after structural conversion via light emission may be called a "colored component". On the other hand, the structure before light emission may be called a "colorless component". However, regarding the colorless component, "colorless" is not limited to being completely colorless, and includes a case in which the color is lighter than that of the colored component. The structure of General Formula 1 and the structures of general formulae of various photochromic compounds to be described below are structures of respective colorless components.

Herein, the term "photochromic article" refers to an article containing a photochromic compound. The present photochromic article contains at least one or more of the present photochromic compounds as a photochromic compound. The photochromic compound can be incorporated into a substrate of a photochromic article and/or can be incorporated into a photochromic layer in a photochromic article having a substrate and a photochromic layer. The term "photochromic layer" is a layer containing a photochromic compound.

Herein, the term "photochromic composition" refers to a composition containing a photochromic compound. The present photochromic compositions contain at least one or more of the present photochromic compounds as a photochromic compound, and can be used for producing the present photochromic article.

Herein, substituents in various general formulae whose details will be described below, and substituents when respective groups to be described below have substituents may each independently

a substituent $R^m$ selected from hydroxy and linear or branched $C_{1-18}$-alkyl groups such as a methyl, ethyl, propyl, butyl, pentyl and hexyl, mono- or multicyclic cycloaliphatic alkyl groups such as a bicyclic ring having 5-18 carbon atoms such as cyclopentyl and cyclohexyl, linear or branched $C_{1-24}$-alkoxy groups such as methoxy, ethoxy and butoxy, linear or branched $C_{1-18}$-perfluoroalkyl groups such as non-aromatic cyclic substituents having 1-24 constituent atoms and a trifluoromethyl group, linear or branched perfluoroalkoxy groups such as trifluoromethoxy, linear or branched alkylsulfide groups having 1-24 constituent atoms such as methylsulfide, ethylsulfide and butylsulfide, aryl groups such as phenyl, naphthyl, anthracenyl, fluoranthenyl, phenanthryl, pyranyl, perylenyl, styryl and fluorenyl, aryloxy groups such as phenyloxy, arylsulfide groups such as phenylsulfide, heteroaryl groups such as pyridyl, furanyl, thienyl, pyrrolyl, benzofuranyl, benzothiophenyl, indolyl, dibenzofuranyl, dibenzothiophenyl, carbazolyl, diazolyl, triazolyl, quinolinyl, phenothiazinyl, phenoxazinyl, phenazinyl, thianthryl and acridinyl, monoalkylamino groups such as amino ($-NH_2$) and monomethylamino, dialkylamino groups such as dimethylamino, monoarylamino groups such as monophenylamino, diarylamino groups such as diphenylamino, cyclic amino groups such as piperidino, morpholino, thiomorpholino, tetrahydroquinolino and tetrahydroisoquinolino, ethynyl, mercapto, silyl, a sulfonic acid group, alkylsulfonyl, formyl, carboxy, cyano and halogen such as F, Cl, Br and I; or
a substituent in which $R^m$ is additionally substituted with one or more of the same or different $R^m$'s.

As an example of the substituent in which the above $R^m$ is additionally substituted with one or more of the same or

different $R^m$'s, a structure in which the terminal carbon atom of an alkoxy group is additionally substituted with an alkoxy group, and the terminal carbon atom of the alkoxy group is additionally substituted with an alkoxy group may be exemplified. In addition, as another example of the substituent in which the above $R^m$ is additionally substituted with one or more of the same or different $R^m$'s, a structure in which two or more positions among five substitutable positions of a phenyl group are substituted with the same or different $R^m$'s may be exemplified. However, the present invention is not limited to such examples.

Herein, unless otherwise specified, the terms "number of carbon atoms" and "number of constituent atoms" refer to the numbers including the number of carbon atoms or the number of atoms of the substituent with respect to a group having a substituent. In addition, in the present invention and this specification, "unsubstituted or having one or more substituents" is synonymous with "substituted or unsubstituted".

In addition, herein, substituents in various general formulae whose details will be described below, and substituents when respective groups to be described below have substituents may each independently a solubilizing group. Herein, the "solubilizing group" refers to a substituent that can contribute to increasing the compatibility with any liquid or a specific liquid. Examples of solubilizing groups include alkyl groups containing a linear, branched or cyclic structure having 4-50 carbon atoms, linear, branched or cyclic $C_{4-50}$-alkoxy groups, linear, branched or cyclic silyl groups having 4-50 constituent atoms, those in which some of the above groups are substituted with a silicon atom, sulfur atom, nitrogen atom, or phosphorus atom, and those obtained by combining two or more of the above groups, and a substituent that can contribute to promoting thermal motion of molecules of the compound according to inclusion of this substituent is preferable. A compound having a solubilizing group as a substituent can inhibit the distance between solute molecules from decreasing and prevent the solute from solidifying, and can lower the melting point and/or glass transition temperature of the solute and create a molecule aggregation state close to that of a liquid. Therefore, the solubilizing group can liquefy a solute or increase the solubility of the compound having this substituent in a liquid. In one aspect, the solubilizing group is preferably n-butyl, n-pentyl, n-hexyl, and n-octyl which are linear alkyl, tert-butyl which is branched alkyl, or cyclopentyl and cyclohexyl which are a cyclic alkyl.

The substituent is preferably a substituent selected from methoxy, ethoxy, phenoxy, methylsulfide, ethylsulfide, phenylsulfide, trifluoromethyl, phenyl, naphthyl, dibenzofuranyl, dibenzothiophenyl, carbazolyl, phenothiazinyl, phenoxazinyl, phenazinyl, acridinyl, dimethylamino group, diphenylamino, piperidino, morpholino, thiomorpholino, cyano and solubilizing group, and more preferably a substituent selected from methoxy, phenoxy, methylsulfide, phenylsulfide, trifluoromethyl, phenyl, dimethylamino, diphenylamino, piperidino, morpholino, thiomorpholino, cyano and solubilizing group.

Herein, "electron-attracting group" refers to a substituent that attracts electrons from the side of atoms bonded more easily than hydrogen atoms. Electron-attracting groups can attract electrons as a result of substituent effects such as an inductive effect, a mesomeric effect (or a resonance effect). Specific examples of electron-attracting groups include halogen atoms (F, Cl, Br I), trifluoromethyl($-CF_3$), nitro ($-NO_2$), cyano (-CN), formyl (-CHO), acyl (-COR, R is a substituent), alkoxycarbonyl (-COOR), carboxy (-COOH), substituted sulfonyl ($-SO_2R$, R is a substituent), and sulfo ($-SO_3H$). Examples of preferable electron-attracting groups include a fluorine atom which is an electron-attracting group having high electronegativity and an electron-attracting group having a positive value for the substituent constant $\sigma_p$ at the para position based on Hammett's rule.

Herein, "electron-donating group" refers to a substituent that donates electrons to the side of atoms bonded more easily than hydrogen atoms. Electron-donating groups can be substituents that tend to donate electrons as a summation of an inductive effect, a mesomeric effect (or a resonance effect) and the like. Specific examples of electron-donating groups include hydroxy (-OH), thiol (-SH), alkoxy (-OR, R is alkyl), alkylsulfide (-SR, R is alkyl), arylsulfide, acetyl ($-OCOCH_3$), amino ($-NH_2$), alkylamide ($-NHCOCH_3$), dialkylamino ($-N(R)_2$, R each independently is alkyl), and methyl. Examples of preferable electron-donating groups include electron-donating groups having a negative value for the substituent constant $\sigma_p$ at the para position based on Hammett's rule.

Specific examples of the substituent constant $\sigma_p$ at the para position based on Hammett's rule (source: edited by Shu Iwamura, Ryoji Noyori, Takeshi Nakai, and Isao Kitagawa, Graduate School of Organic Chemistry (Part 1) (1988)) are shown below.

$-N(CH_3)_2$: -0.83
-OCH3: -0.27
$-t-C_4H_9$: -0.20
$-CH_3$: -0.17
$-C_2H_5$: -0.15
$-C_6H_5$: -0.01
(-H: 0)
-F: +0.06
-Cl: +0.27

-Br: +0.23
$-CO_2C_2H_5$: +0.45
$-CF_3$: +0.54
-CN: +0.66
$-SO_2CH_3$: +0.72
$-NO_2$: +0.78

[Photochromic Compound of Formula (1) or (1a)]

Hereinafter, compounds of Formula (1) and (1a) will be described in more detail:

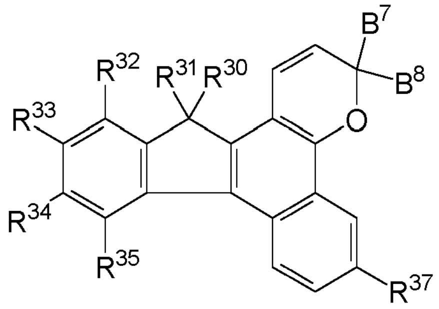

(1)

In Formula (1), $R^{30}$ and $R^{31}$ each independently are selected from linear or branched $C_{2-10}$-alkyl, each optionally substituted. When the linear or branched alkyl group has a substituent, the number of carbon atoms of the alkyl group refers to the number of carbon atoms in a moiety containing no substituent.
$R^{30}$ and $R^{31}$ each independently preferably represent ethyl, propyl, isopropyl, n-butyl, s-butyl or t-butyl. $R^{30}$ and $R^{31}$ both more preferably represent ethyl.
In Formula (1), $R^{32}$-$R^{35}$ each independently are H or a substituent other than an electron-attracting group. Two or more substituents may be bonded to form a ring.
In one aspect, $R^{32}$-$R^{35}$ all may be H. In another aspect, $R^{32}$-$R^{35}$ may each independently be H, optionally substituted aryl or optionally substituted alkoxy. For such aryl groups and alkoxy groups, the above description regarding the substituent $R^m$ can be referred to. In one aspect, $R^{32}$-$R^{35}$ may each independently H, optionally substituted phenyl or methoxy.
In one aspect, in $R^{32}$-$R^{35}$, $R^{33}$ may represent a substituent other than an electron-attracting group, and $R^{32}$, $R^{34}$ and $R^{35}$ may be H. In this case, $R^{33}$ may be optionally substituted aryl or optionally substituted alkoxy, and may be, for example, optionally substituted phenyl or methoxy.
In Formula (1), $R^{37}$ is H or an electron-donating group selected from methoxy, ethoxy, phenoxy, methylsulfide, phenylsulfide, dimethylamino, pyrrolidino, piperidino, morpholino and thiomorpholino.
In Formula (1), $B^7$ and $B^8$ each independently are H or a substituent, and preferably each independently are a phenyl, naphthyl, fluorenyl, benzofluorenyl, fluoranthenyl, dibenzofuranyl or dibenzothiophenyl, each optionally substituted. More preferably, $B^7$ and $B^8$ each independently are substituted phenyl. Such substituted phenyl groups may include one or more substituents selected from methoxy, methylsulfide, amino, dimethylamino, piperidino, morpholino, thiomorpholino, phenyl, fluorine, chlorine, bromine, iodine, trifluoromethyl and cyano.

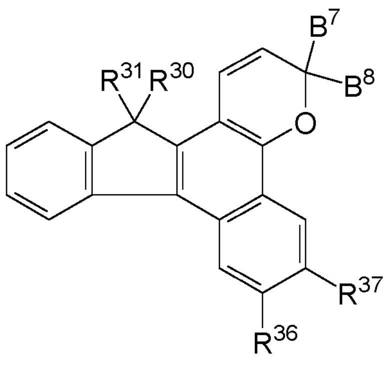

(1a)

In Formula (1a), $R^{30}$ and $R^{31}$ each independently are selected from linear or branched $C_{\geq 2}$-alkyl (for example,

$C_{2-20}$-alkyl or $C_{2-10}$-alkyl), $C_{\geq 3}$-cycloalkyl (for example, $C_{3-20}$-cycloalkyl) and $-(R^{100})_nR^{101}$ wherein $R^{100}$ is alkyleneoxy, $R^{101}$ is alkyl, n is an integer of ≥ 1; each optionally substituted.

In $-(R^{100})_nR^{101}$ the total number of carbon atoms of n alkyleneoxy groups represented by $R^{100}$ and alkyl groups represented by $R^{101}$ is 2 or more (for example, 2-50). When the linear or branched $C_{\geq 2}$-alkyl group has a substituent, the number of carbon atoms of the alkyl group refers to the number of carbon atoms in a moiety containing no substituent. When the cyclic $C_{\geq 3}$-alkyl group has a substituent, the number of carbon atoms of the alkyl group refers to the number of carbon atoms in a moiety containing no substituent. When the group represented by "$-(R^{100})_nR^{101}$" has a substituent, a total number of carbon atoms of n alkyleneoxy groups represented by $R^{100}$ and alkyl groups represented by $R^{101}$ refers to the number of carbon atoms in a moiety containing no substituent.

$R^{30}$ and $R^{31}$ each independently preferably represent ethyl, propyl, isopropyl, n-butyl, s-butyl or t-butyl. $R^{30}$ and $R^{31}$ both more preferably represent ethyl.

In Formula (1a), $R^{36}$ and $R^{37}$ each independently are an electron-donating group. The electron-donating group that may be represented by $R^{36}$ and/or $R^{37}$ is preferably an electron-donating group selected from methoxy, ethoxy, phenoxy, methylsulfide, phenylsulfide, dimethylamino, pyrrolidino, piperidino, morpholino and thiomorpholino.

In Formula (1a), $B^7$ and $B^8$ and preferred embodiments thereof are as defined for formula (1) above.

Also, in one embodiment of the invention, a photochromic composition comprises one or more photochromic compounds of formula (1b):

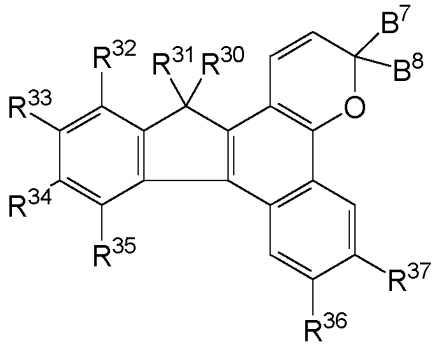

(1b)

wherein $R^{30}$ and $R^{31}$ both are ethyl, $R^{32}$-$R^{35}$ each independently are H or a substituent other than an electron-attracting group, and one of $R^{36}$ and $R^{37}$ is an electron-donating group, and the other is H or an electron-donating group. The substituent other than an electron-attracting group, and the electron-donating group are as defined above.

Examples of the photochromic compounds of the Formula (1), (1a) and (1b) include the following compounds.

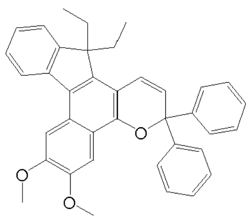

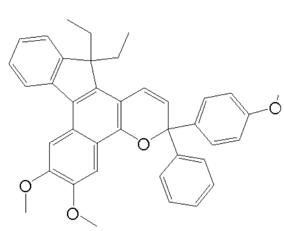

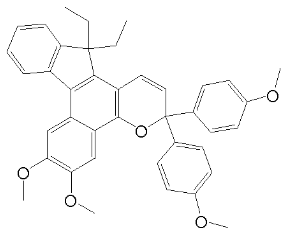

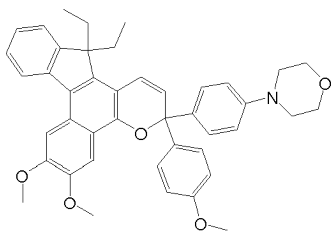

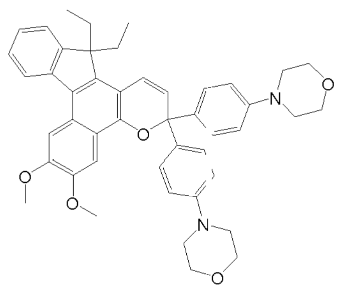

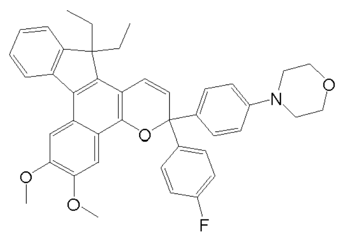

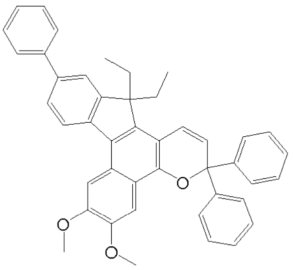
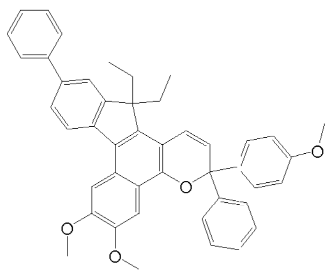
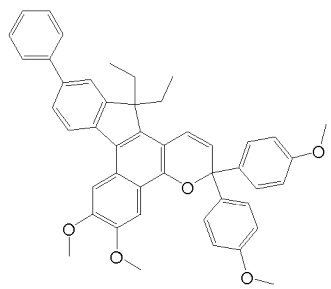

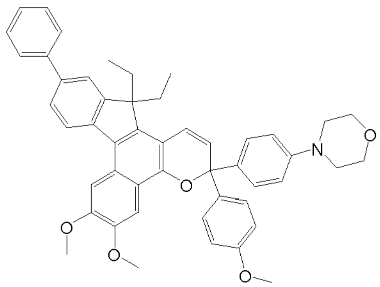
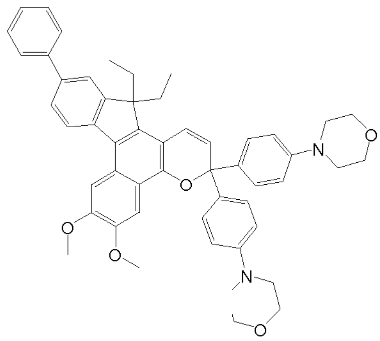
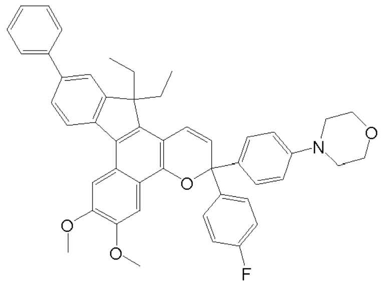

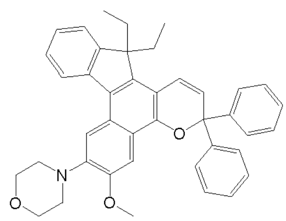
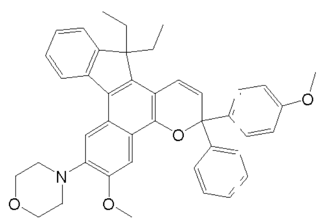
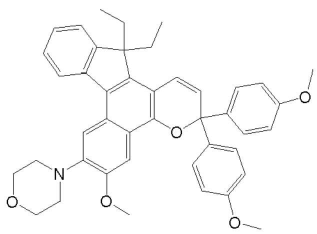

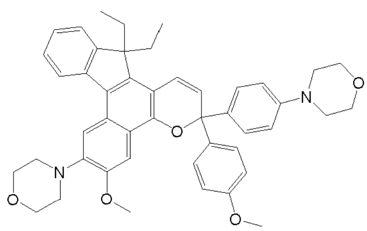
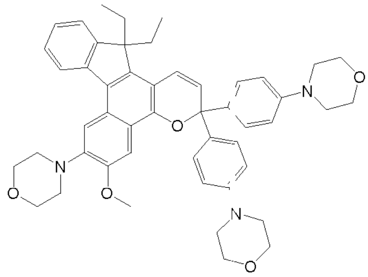
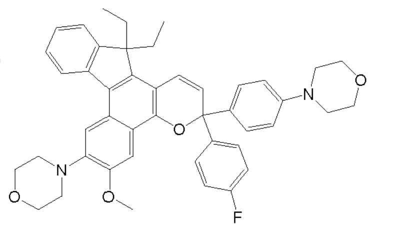

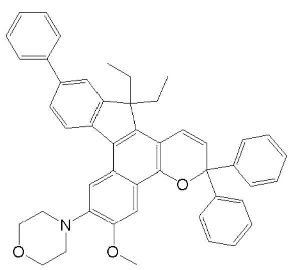
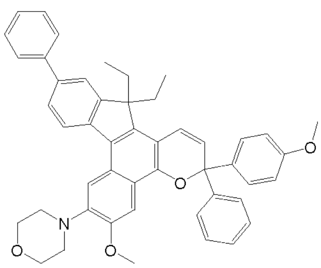
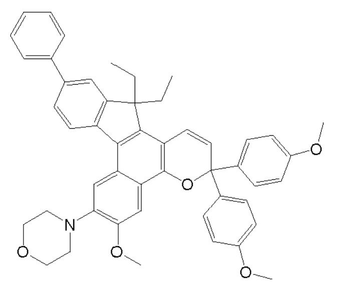

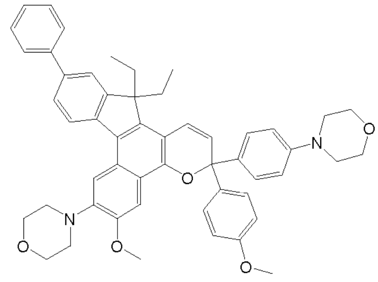
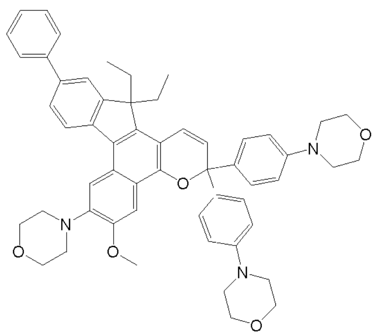
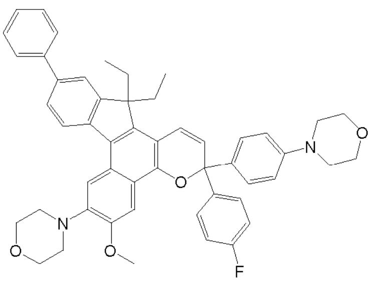

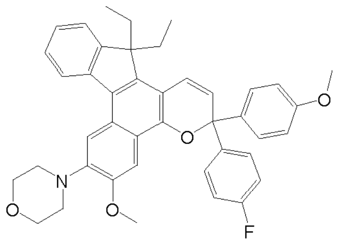
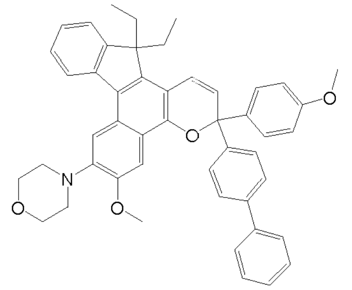
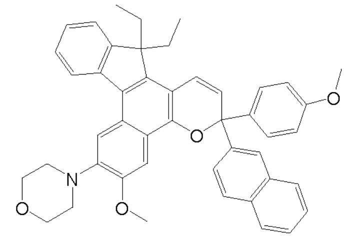

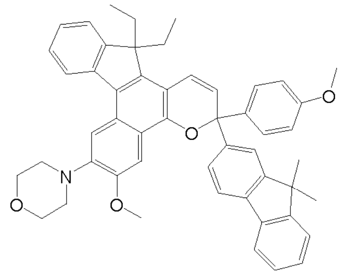
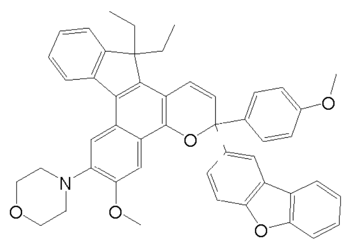
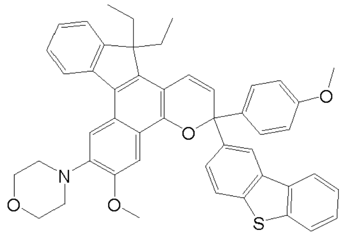

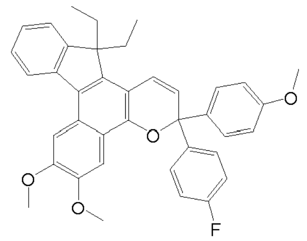
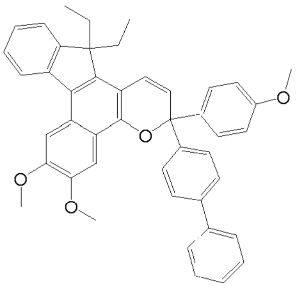
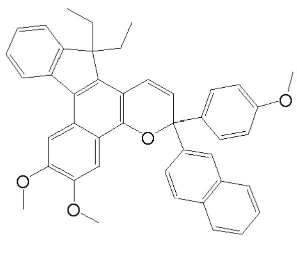

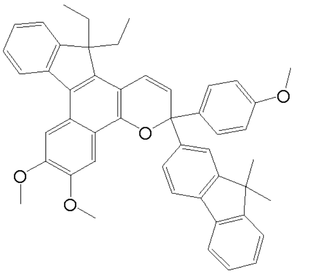
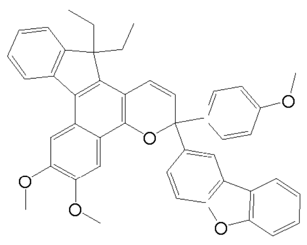
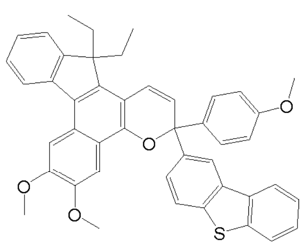

[C6]

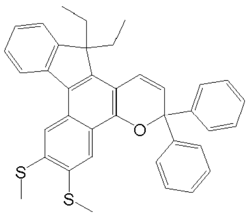
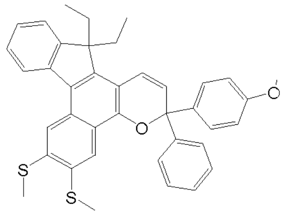
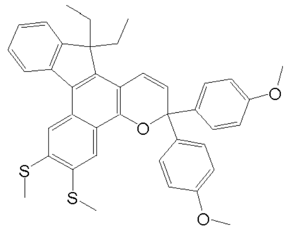
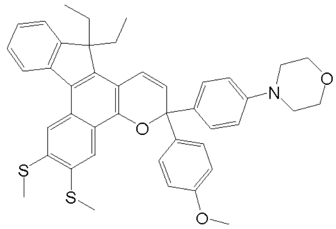
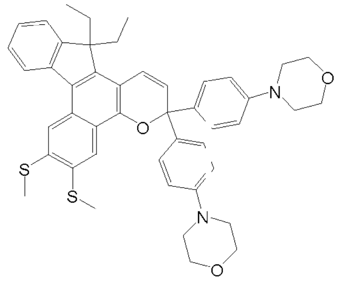
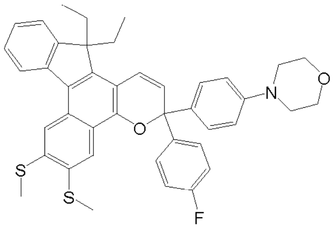
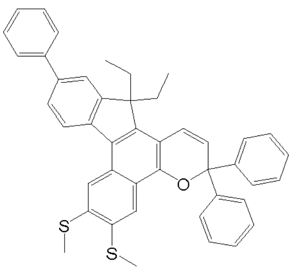
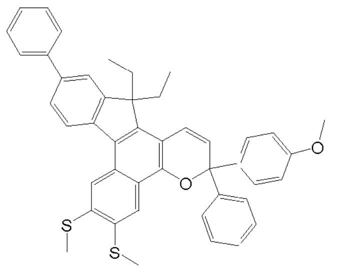
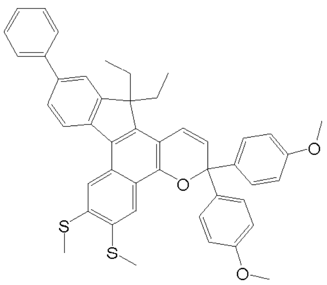
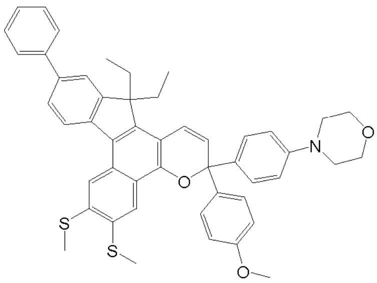
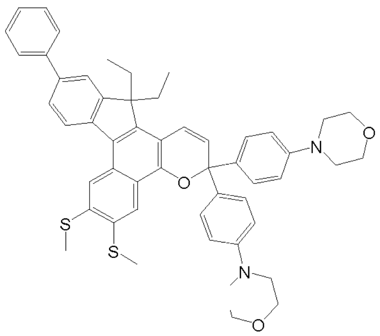
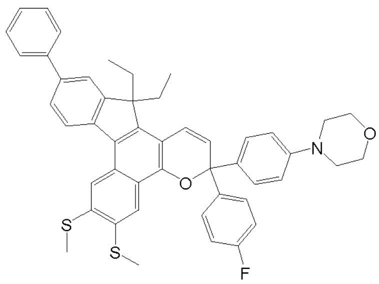

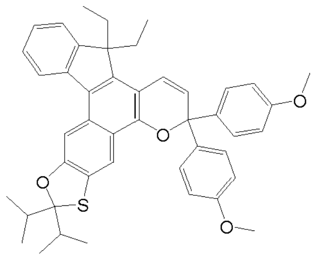
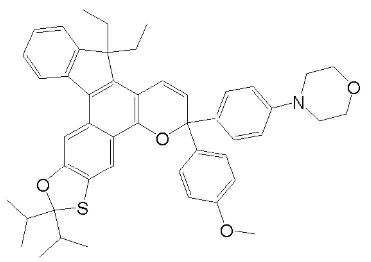
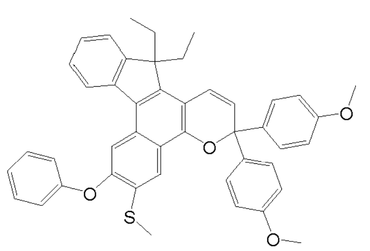

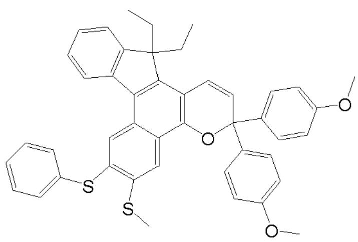
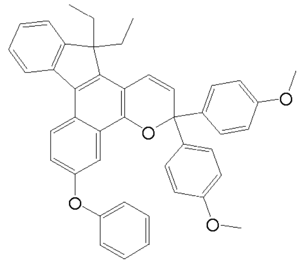
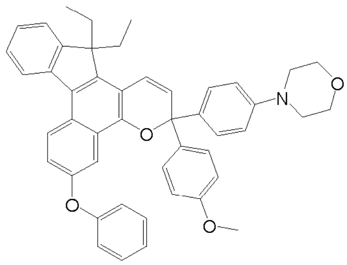

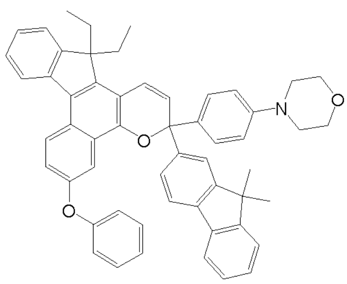
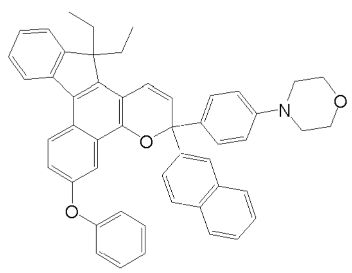
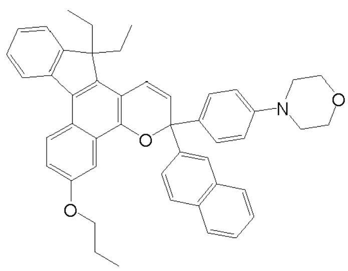

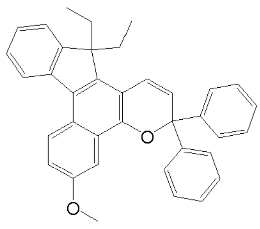

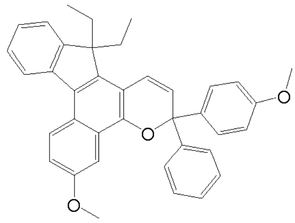

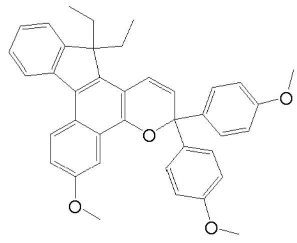

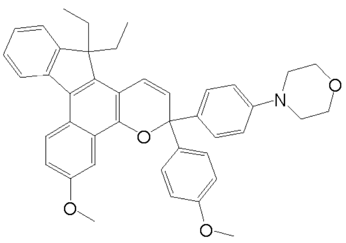

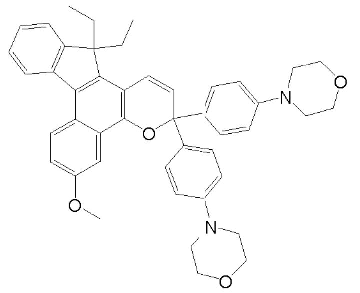

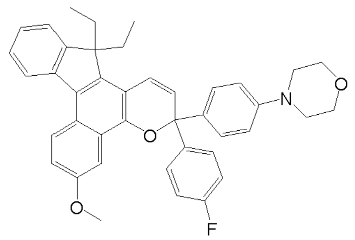

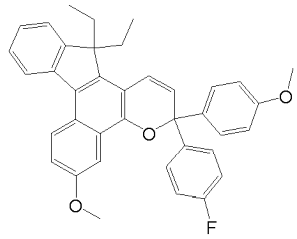

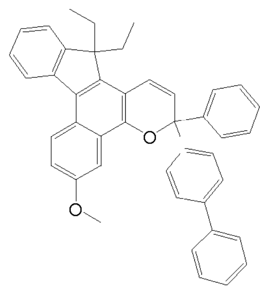

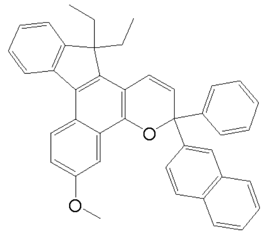

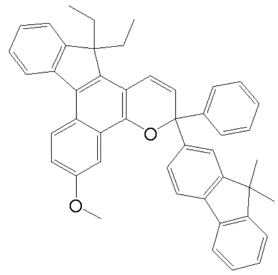

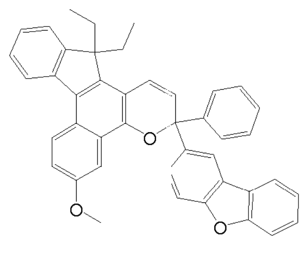

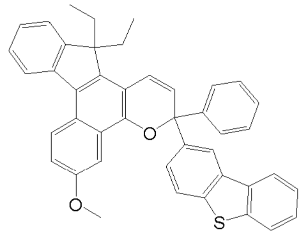

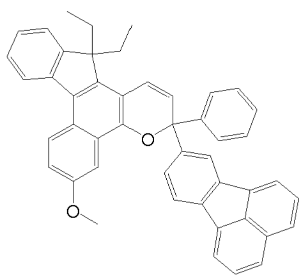

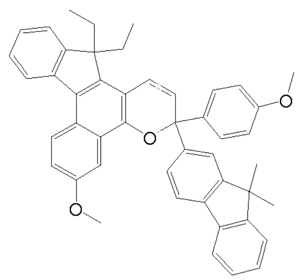

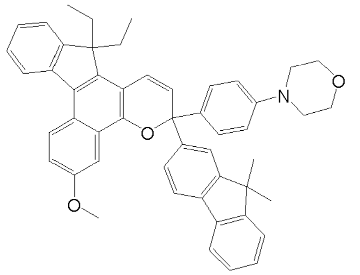

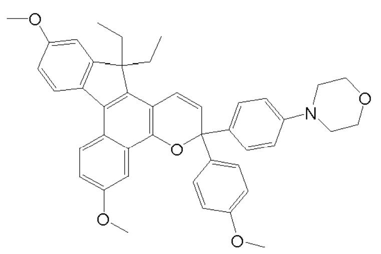

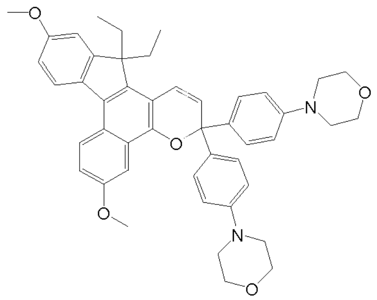

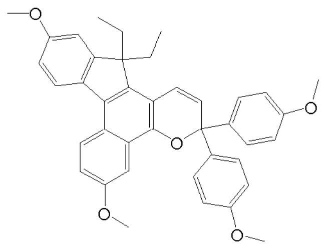

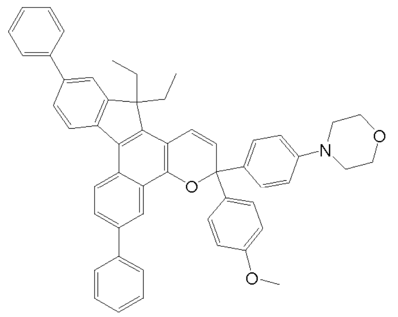

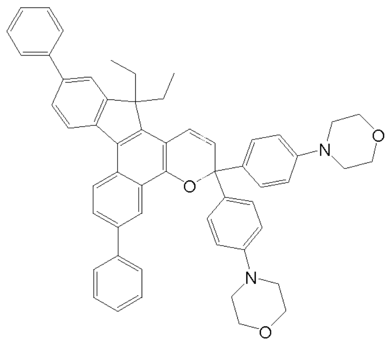

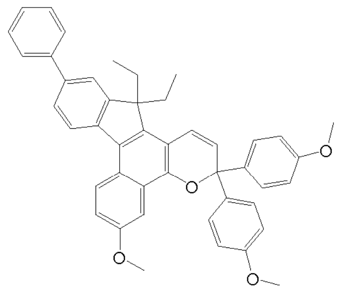

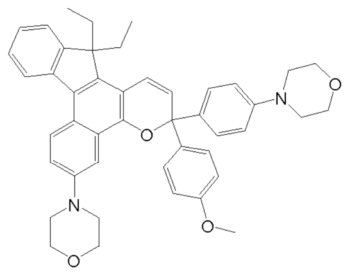

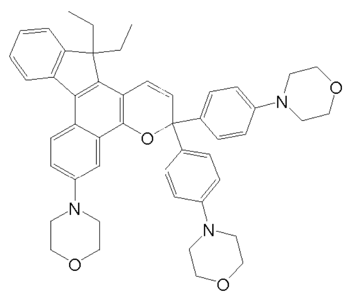

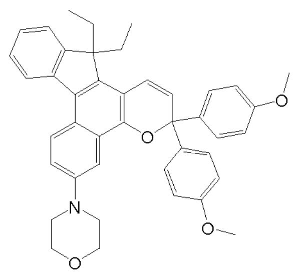

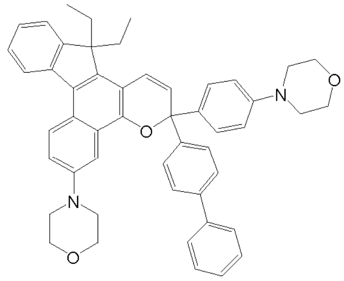

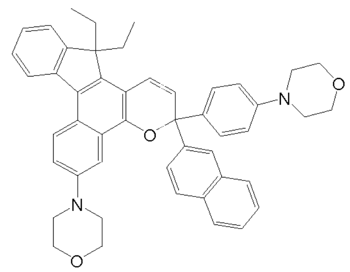

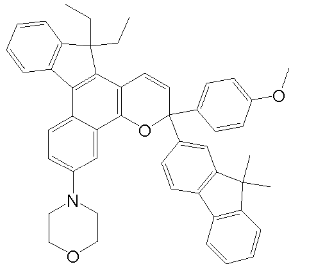

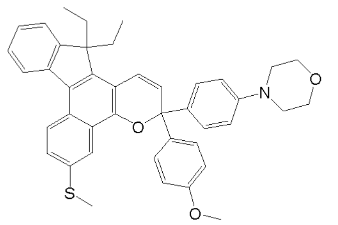
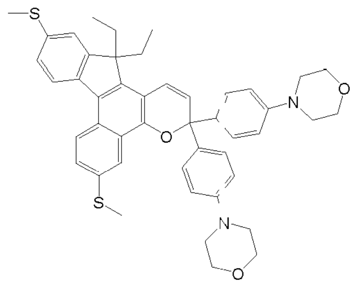
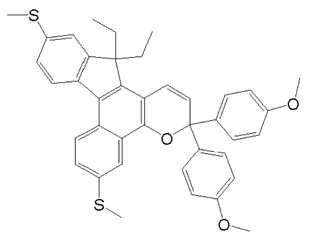

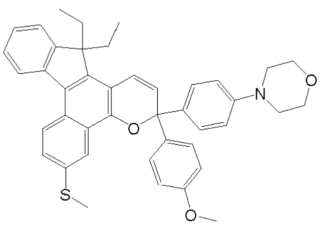
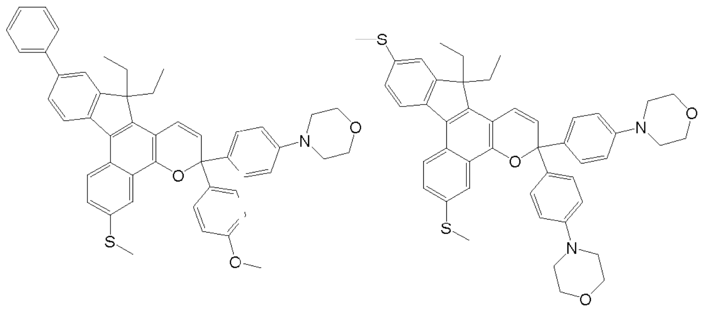

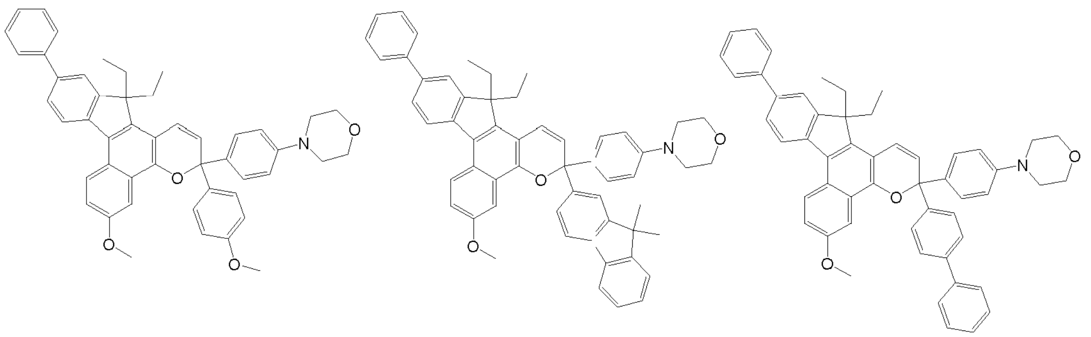

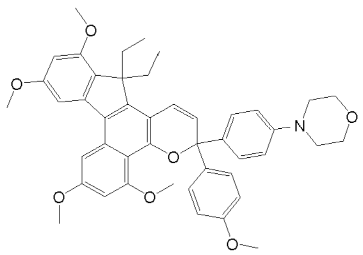
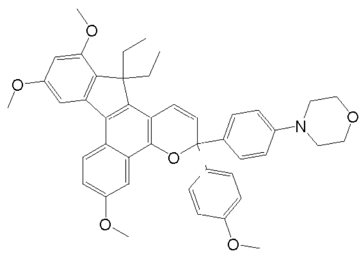
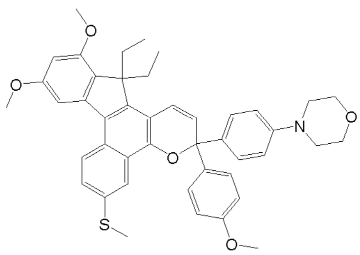

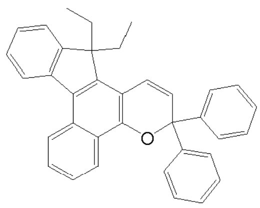
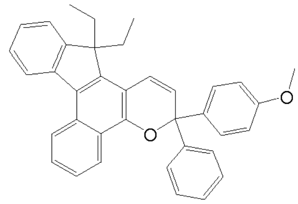
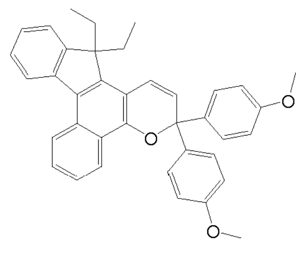

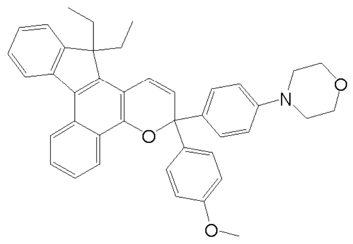
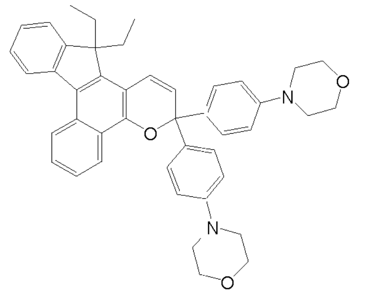
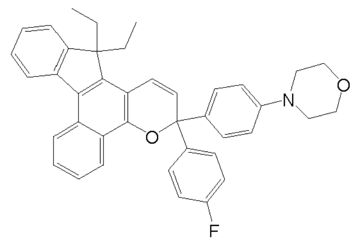

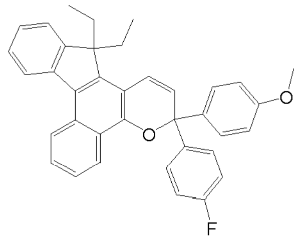
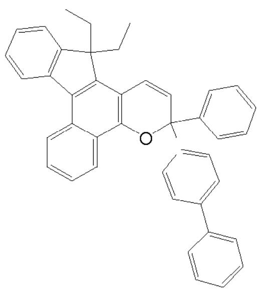
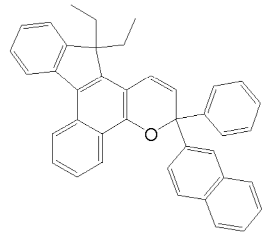

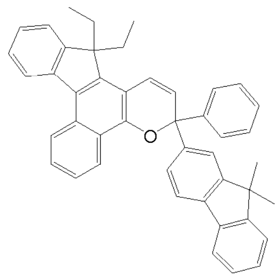
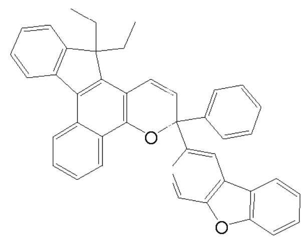
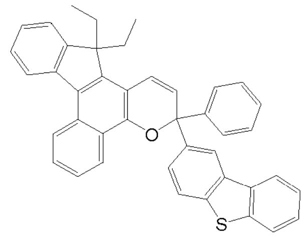

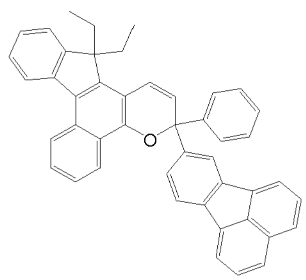
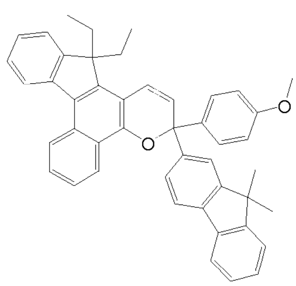
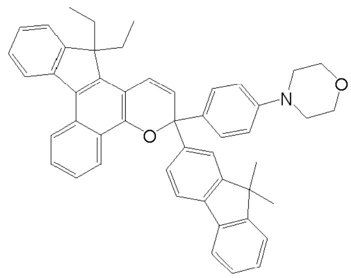

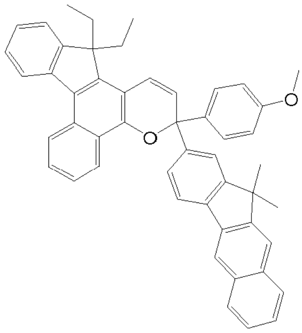

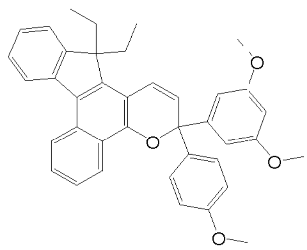

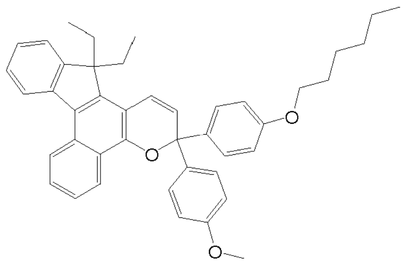

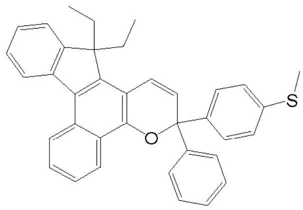

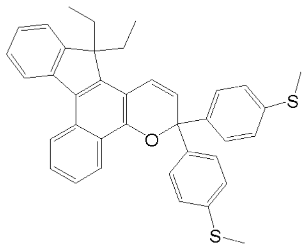

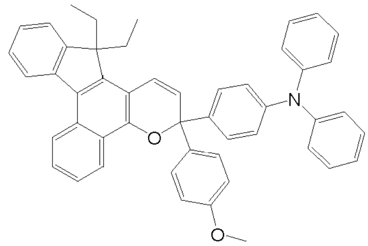

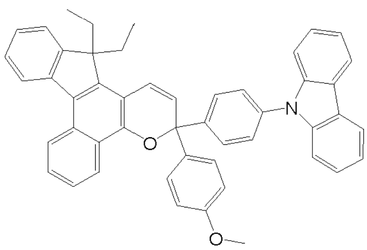

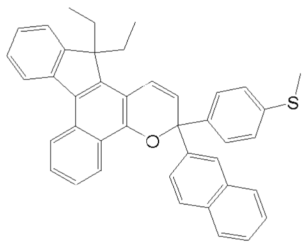

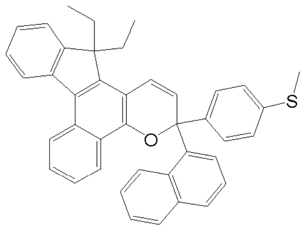

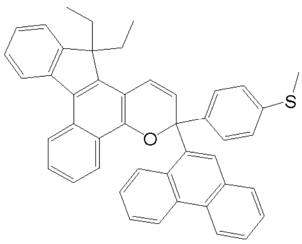

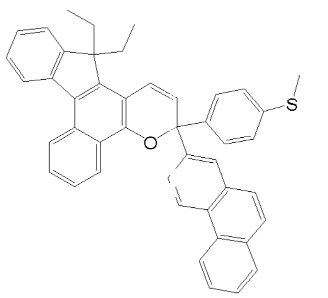

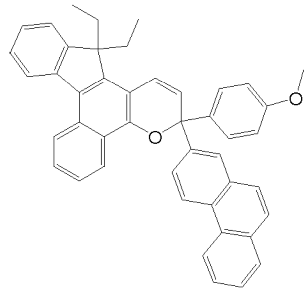

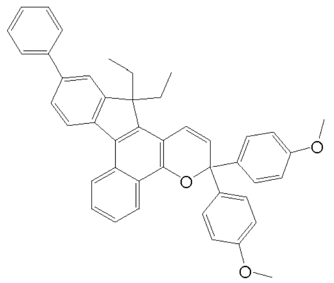
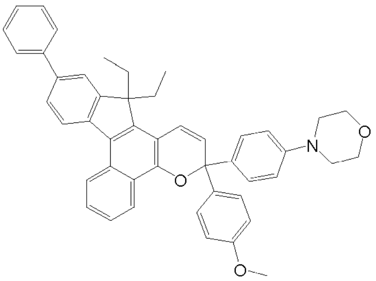
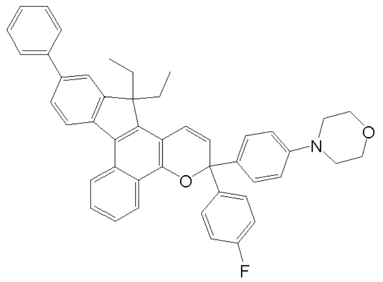
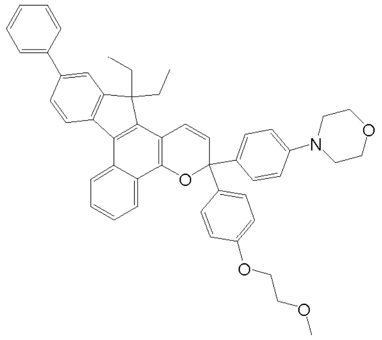
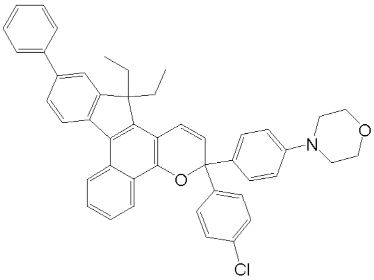
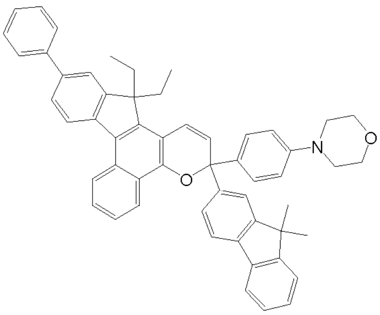
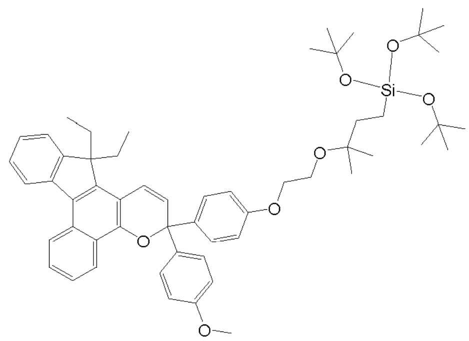
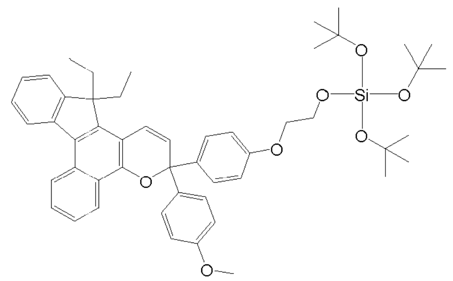
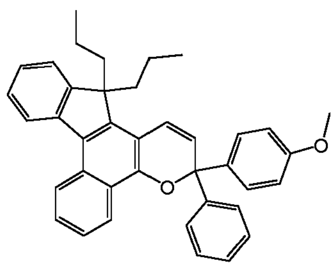
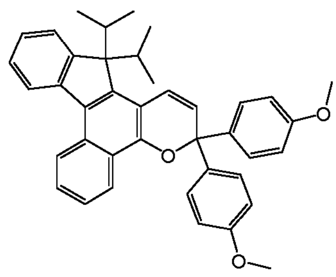
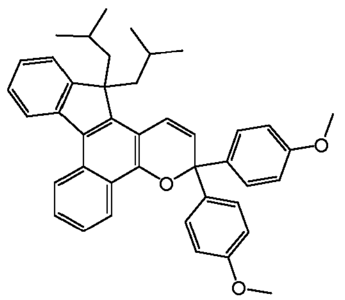
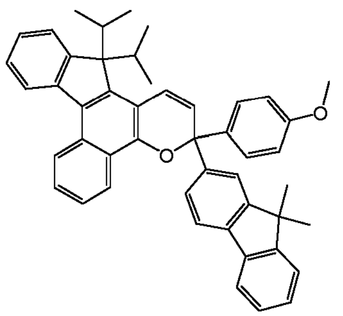
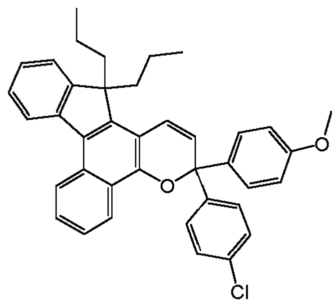
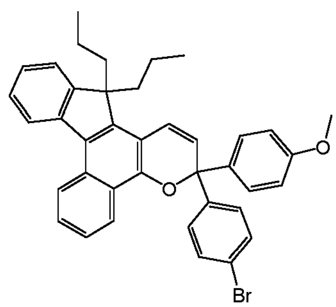

The compounds of the Formulae (1), (1a) and (1b) and the photochromic compounds represented by various general formulae to be described below can be synthesized by a known method. For the synthesis method, for example, the following documents can be referred to. JP-B-4884578, US-A-2006/0226402, US-A-2006/0228557, US-A-2008/0103301, US-A-2011/0108781, US-A-2011/0108781, US 7,527,754, U,S 7,556,751, WO 2001/60811, WO

2013/086248, WO 1996/014596 and WO 2001/019813.

[Photochromic Composition and Photochromic Article]

One aspect of the present invention relates to a photochromic composition containing one or more pf the present photochromic compounds.

In addition, one aspect of the present invention relates to a photochromic article containing one or more of the present photochromic compounds.

The photochromic composition and the photochromic article may contain only one present photochromic compound Formula (1) or (1a) or two or more (for example, two or more and four or less) thereof. In addition, examples of the photochromic compound that can be included in the photochromic composition and the photochromic article together with the present photochromic compounds include compounds of the Formulae (A), (B) and (C). Only one, two or three of compounds of Formula (A), (B) and (C) may be included.

In order to further improve the coloring concentration in a visible range during coloring, as a compound Formula (1) or (1a) which is preferably combined with a compound of Formula (B) and/or a compound of Formula (C), (a) a compound in which, in Formula (1) or (1a), $R^{30}$ and $R^{31}$ both represent ethyl, and $R^{36}$ and $R^{37}$ both are H may be exemplified.

In order to further improve the coloring concentration in a visible range during coloring, as a compound of Formula (1) or (1a) which is preferably combined with a compound of Formula (A), one or more compounds selected from (b) a compound in which, in Formula (1) or (1a), $R^{30}$ and $R^{31}$ both are ethyl, and $R^{36}$ (if present) and $R^{37}$ each independently represent an electron-donating group, and (c) a compound in which, in Formula (1), $R^{30}$ and $R^{31}$ both are ethyl, one of $R^{36}$ (if present) and $R^{37}$ is an electron-donating group and the other is H ogen atom and the other represents an may be exemplified.

In the above (c), preferably, $R^{36}$ is h and $R^{37}$ IS an electron-donating group.

Hereinafter, among compounds of Formula (1) or (1a), the compound satisfying the above (a) is called "Compound 1-a", the compound satisfying the above (b) is called "Compound 1-b", and the compound satisfying the above (c) is called "Compound 1-c". In addition, the compound of Formula (A) is called "Compound A", the compound of Formula (B is called "Compound B", and the compound of Formula (C) is called "Compound C". Compound A and Compound 1-a are collectively referred to a "group A compound", Compound B and Compound 1-b are collectively referred to a "group B compound", and Compound C and Compound 1-c are collectively referred to a "group C compound".

The present photochromic article and the present photochromic composition may contain one or more group A compounds, one or more group B compounds, and one or more group C compounds.

The group A compound contained in the photochromic article and the photochromic composition may be of only one type or two or more types (for example, two or more and four or less).

The group B compound contained in the photochromic article and the photochromic composition may be of only one type or two or more types (for example, two or more and four or less).

The group C compound contained in the photochromic article and the photochromic composition may be of only one type or two or more types (for example, two or more and four or less).

In the photochromic article and the photochromic composition, on a mass basis, a total content of the group B compound and the group C compound is preferably larger than the content of the group A compound. Based on a total content of 100 mass% of the group A compound, the group B compound and the group C compound, a total content of the group B compound and the group C compound is preferably more than 50 mass%, more preferably 60 mass% or more, still more preferably 70 mass% or more, yet more preferably 80 mass% or more, and most preferably 90 mass% or more. Based on a total content (100 mass%) of the group A compound, the B compound and the group C compound, a total content of the group B compound and the group C compound may be less than 100 mass%, 99 mass% or less, 98 mass% or less, 97 mass% or less, 96 mass% or less or 95 mass% or less.

Regarding the mixing ratio between the group B compound and the group C compound, based on a total content of 100 mass% of the group B compound and the group C compound, the content of the group B compound may be 1 mass% or more or 99 mass% or more and may be 25 mass% or less or 75 mass% or less. When the photochromic article and the photochromic composition contain two or more group B compounds, the content of the group B compounds is a total content thereof. The same applies to the contents of various components in the present invention and this specification.

The photochromic article and the photochromic composition based on a total amount of 100 mass% thereof may contain a total content of, for example, about 0.1-15.0 mass% of the group A compound, the group B compound and the group C compound. The photochromic article and the photochromic composition can contain, for example, about 0.1-15.0 mass% of the compound represented by General Formula 1 with respect to a total amount of 100 mass% thereof. However, the present invention is not limited to the above range.

Hereinafter, compounds of the Formulae (A), (B) and (C) will be described in more detail.

<Compound of Formula (A)>

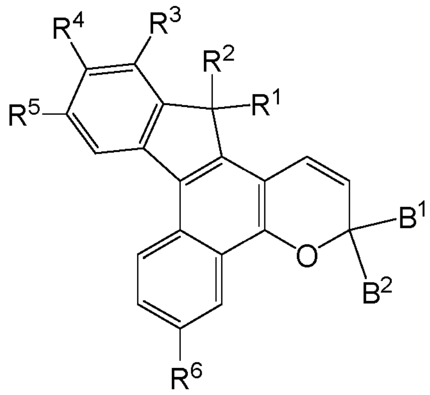

(A)

In Formula (A), $R^1$-$R^6$, $B^1$ and $B^2$ each independently are H or a substituent.

$R^1$ and $R^2$ each independently preferably are optionally substituted $C_{1-20}$-alkyl, and more preferably methyl, ethyl, propyl, butyl, pentyl or hexyl. $R^1$ and $R^2$ each independently arestill more preferably methyl or ethyl, and more preferably, $R^1$ and $R^2$ both are methyl or both are ethyl.

$B^1$ and $B^2$ each independently preferably are optionally substituted phenyl. When the phenyl group has a plurality of substituents, two or more of these substituents may be bonded to form a ring. Specific examples of the formed rings include rings included in exemplary compounds listed below. The substitution position of the substituent in the substituted phenyl group is preferably a position that is a para position with respect to a carbon atom to which $B^1$ and $B^2$ are bonded. Specific examples of substituents of substituted phenyl groups include morpholino, piperidino, halogen, alkoxy, and substituents included in exemplary compounds listed below such as the following substituents. Herein, "*" for a partial structure of a compound indicates a bonding position with an atom to which such a partial structure is bonded.

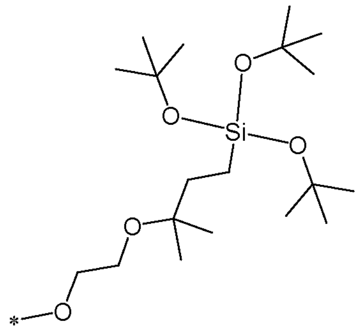

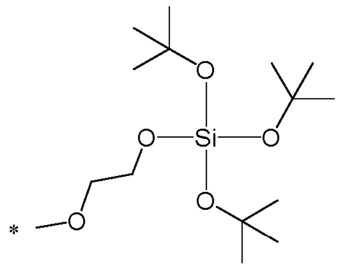

In Formula (A), $R^3$-$R^6$ each independently are H or a substituent. In one aspect, $R^3$-$R^6$ all may be H. In another aspect, $R^3$-$R^6$ each independently are H or an electron-attracting group, provided that one or more of $R^3$-$R^6$ are an electron-attracting group. The electron-attracting group is preferably halogen, $C_{1-6}$-perfluoroalkyl, perfluorophenyl, perfluoroalkylphenyl or cyano. The halogen atom is preferably F. The $C_{1-6}$-perfluoroalkyl group is preferably trifluoromethyl.

In one aspect, the compound of Formula (A) may be the following compound.

A compound in which, among $R^3$-$R^6$, only $R^4$ is an electron-attracting group, and $R^3$, $R^5$ and $R^6$ are H.

A compound in which, among $R^3$-$R^6$, $R^4$ and $R^6$ are the same or different electron-attracting groups, and $R^3$ and $R^5$ are H.

A compound in which, among $R^3$-$R^6$, $R^3$ and $R^5$ are the same or different electron-attracting groups, and $R^4$ and $R^6$ are H.

Examples of compounds of Formula (A) include the following compounds.

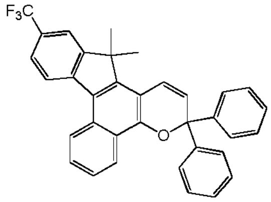
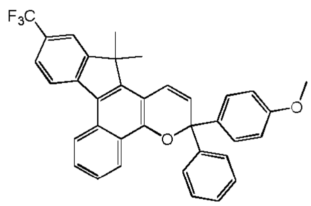
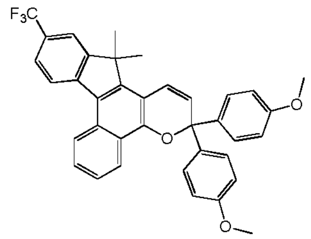
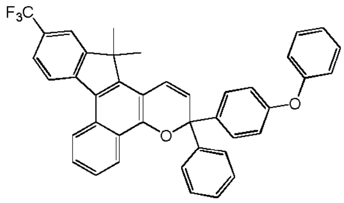
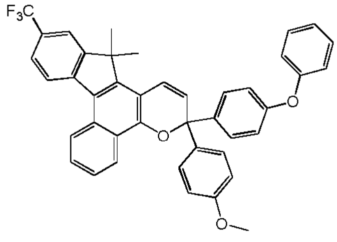
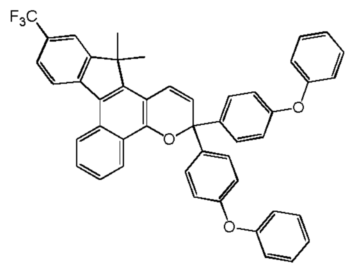
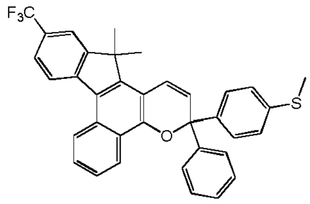
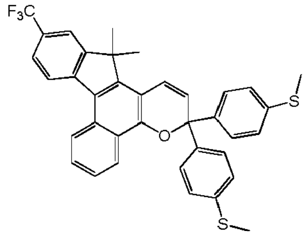
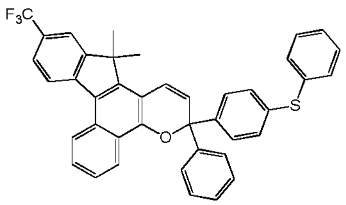
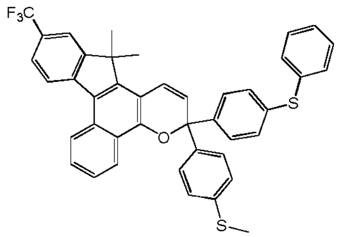
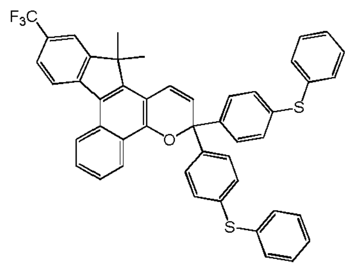
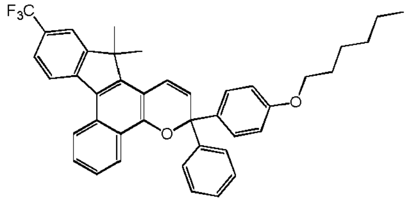
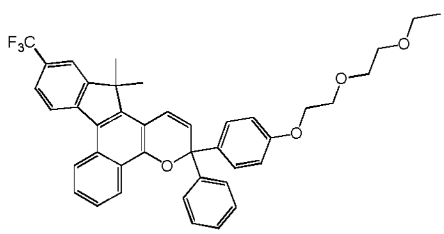
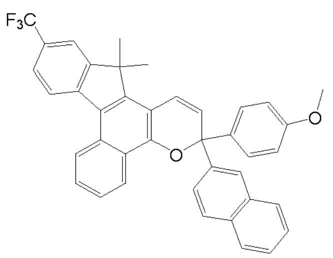
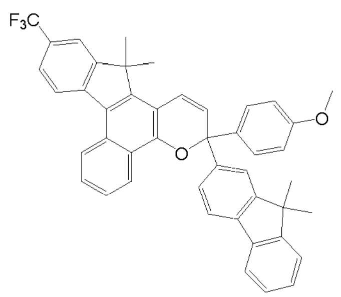
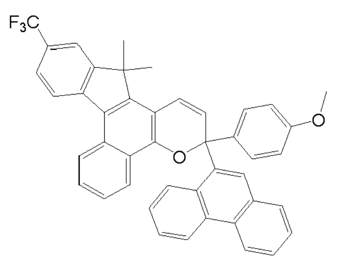

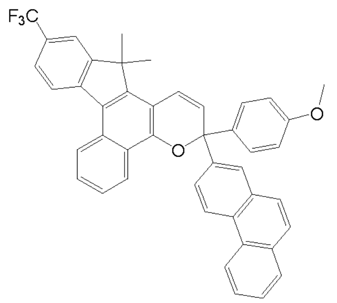
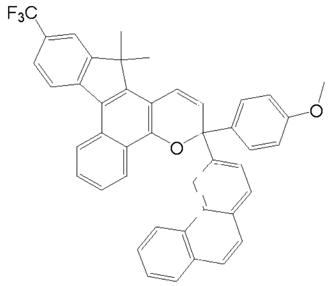
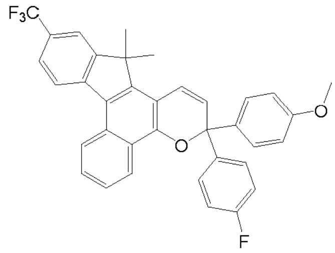
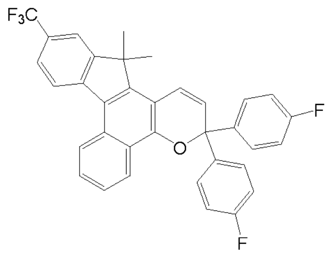
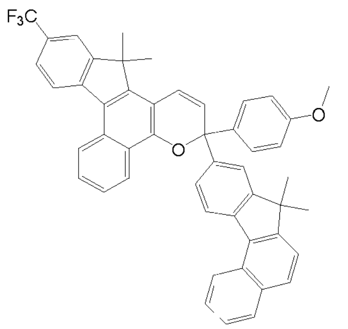
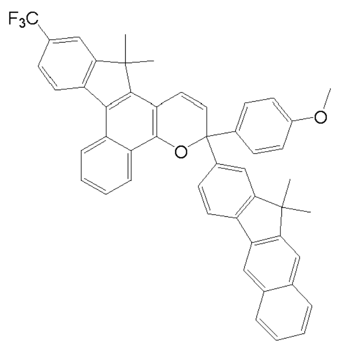
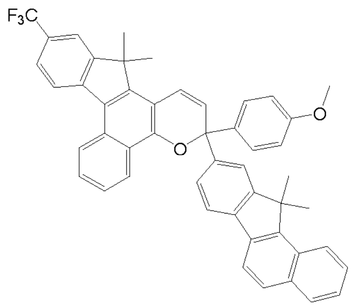
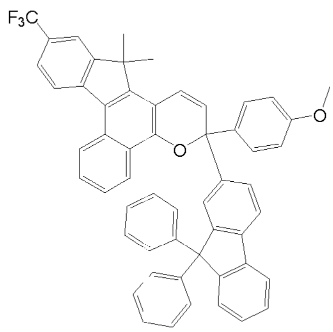
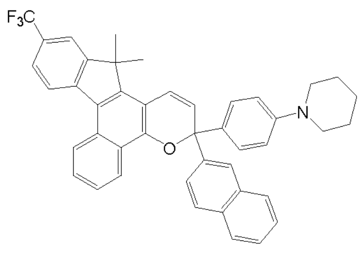
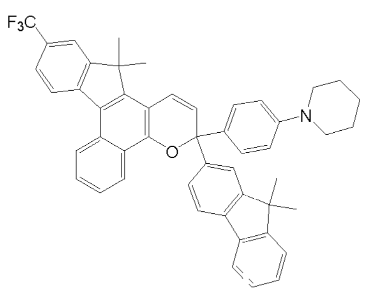
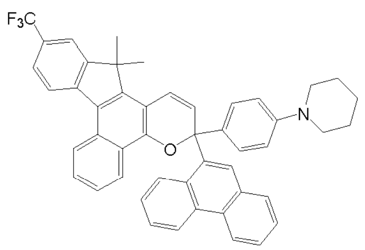
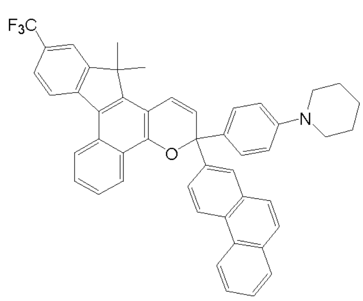
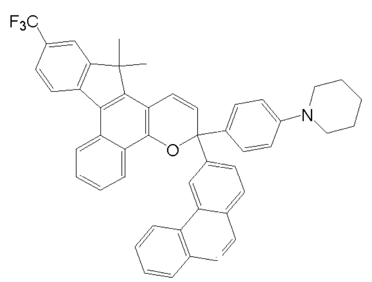
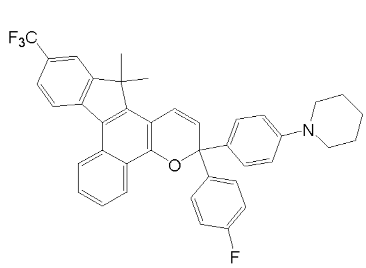

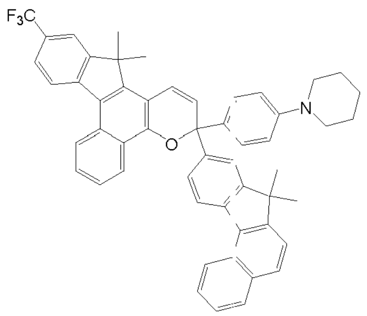

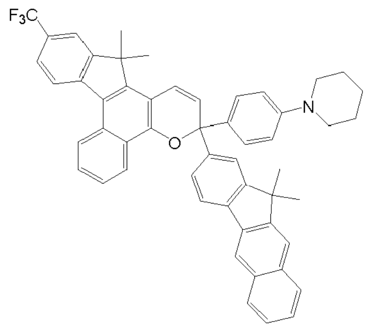

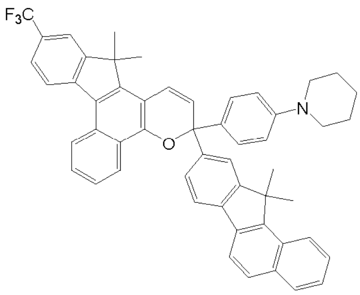

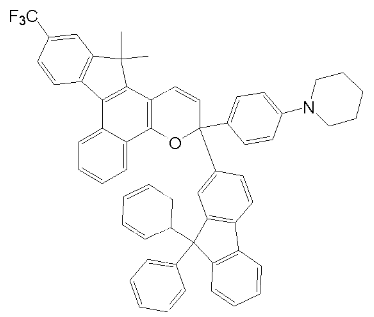

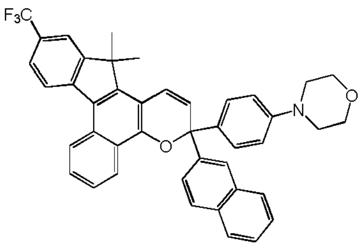

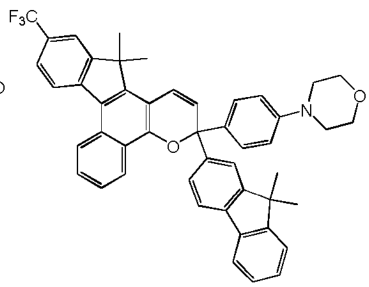

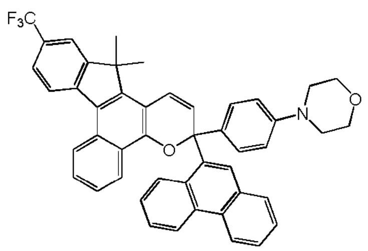

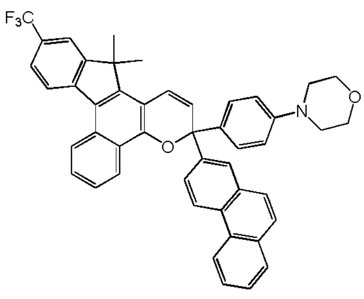

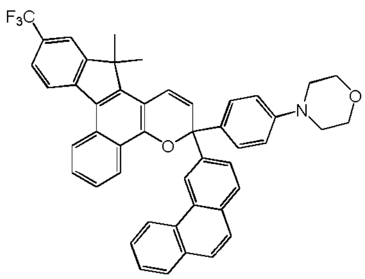

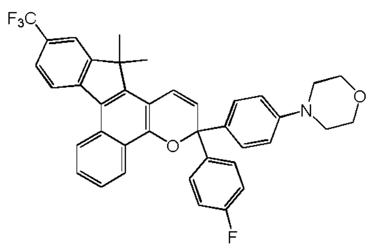

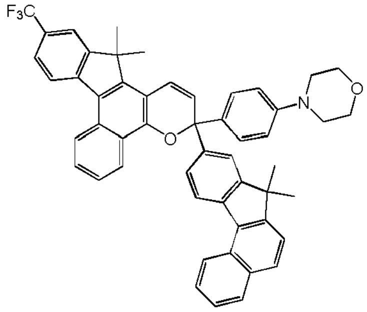

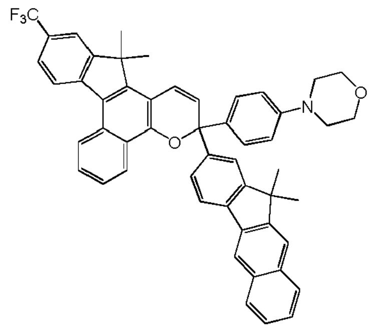

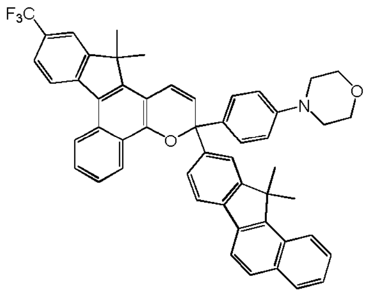

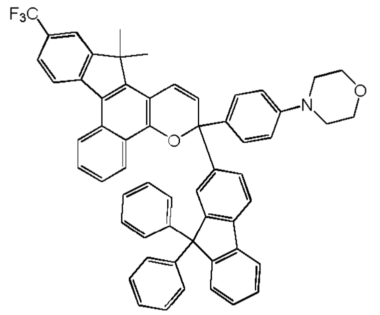

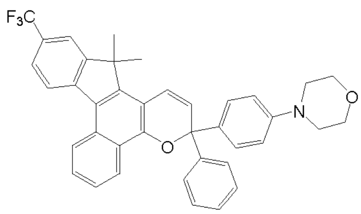

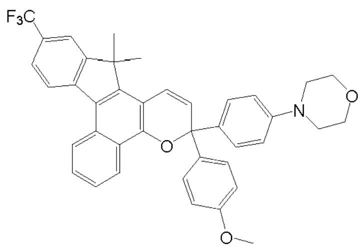

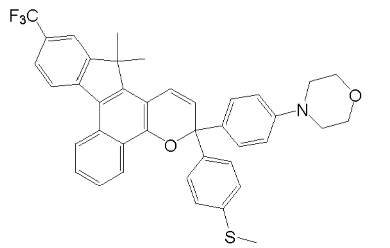

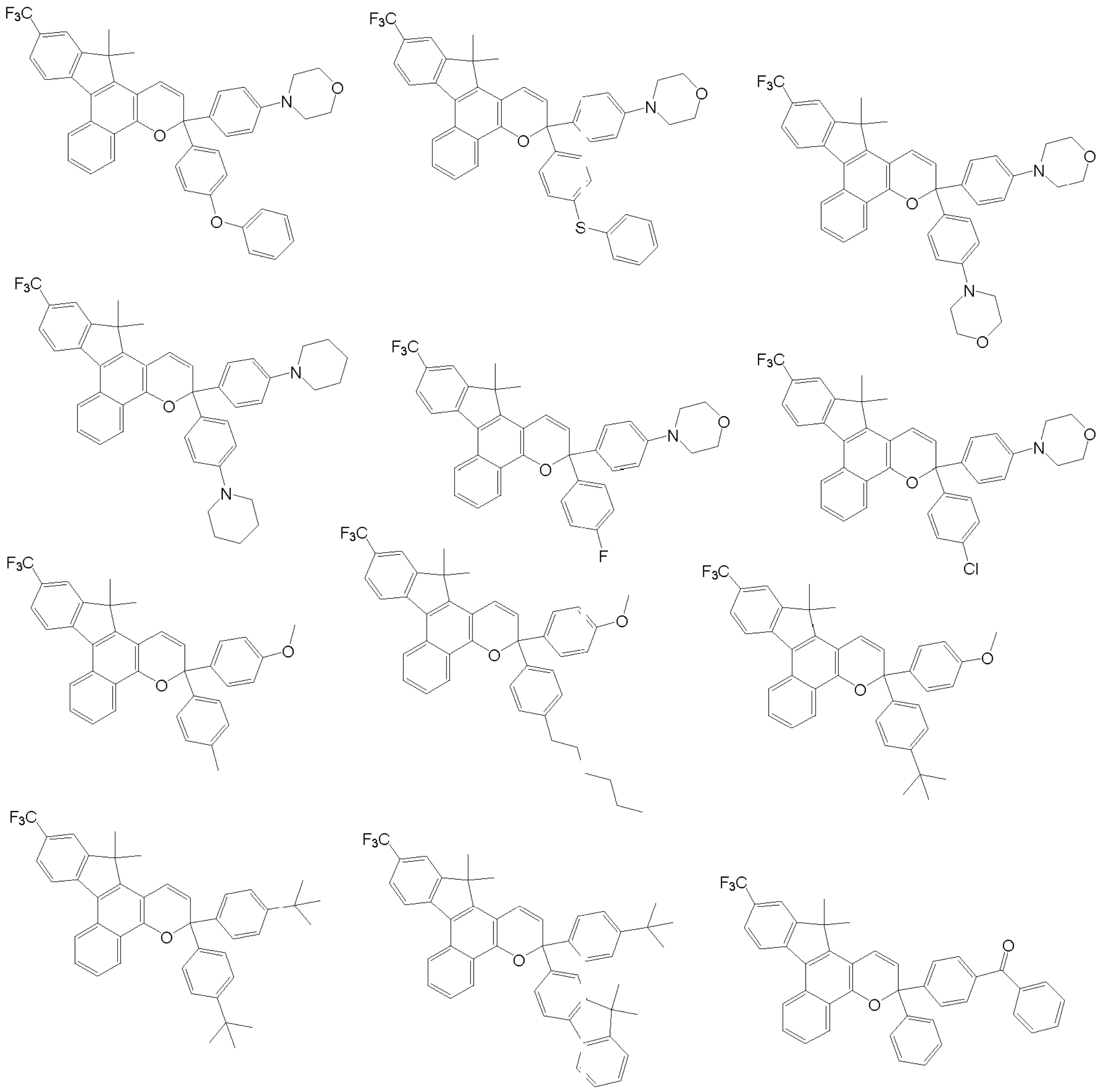

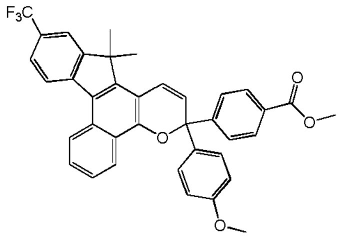
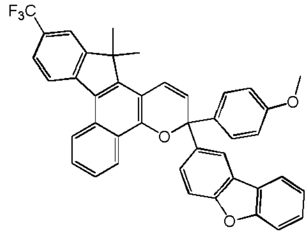
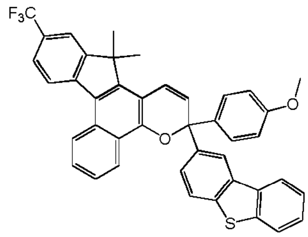
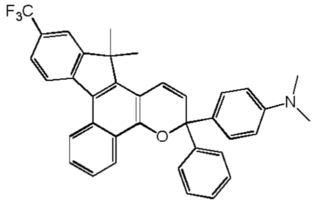
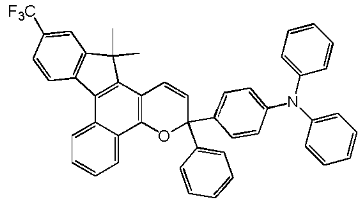
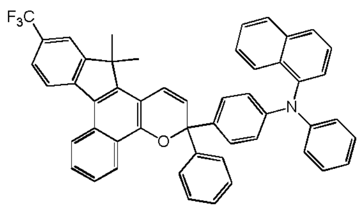
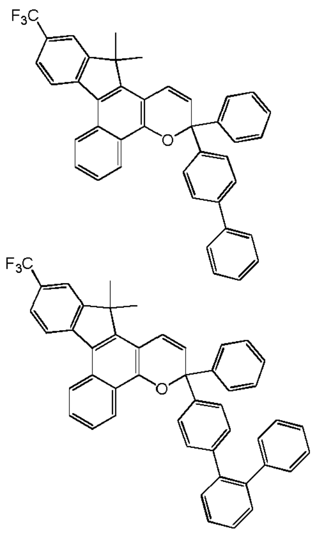
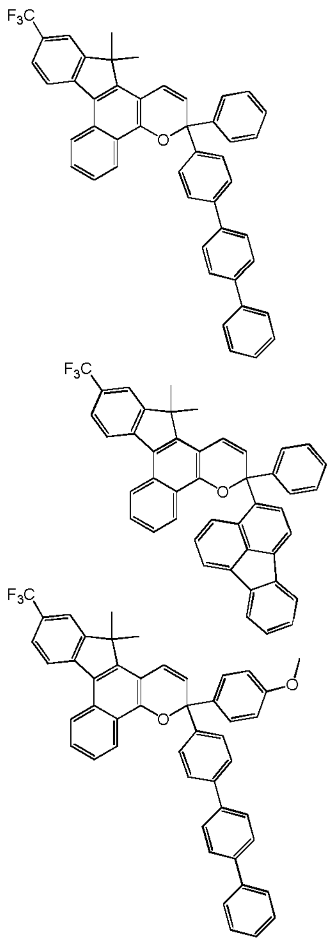
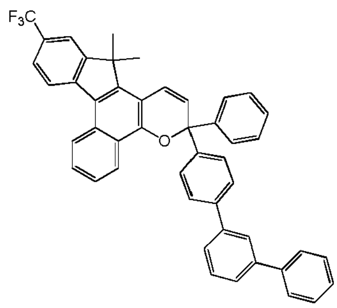
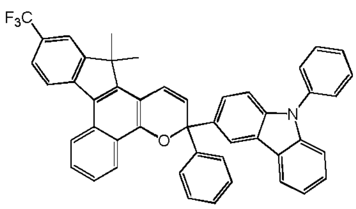
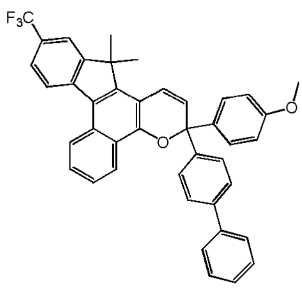
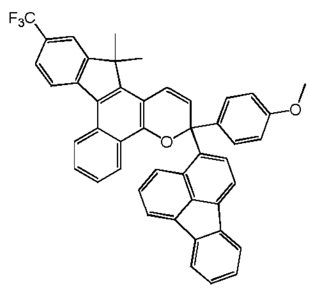

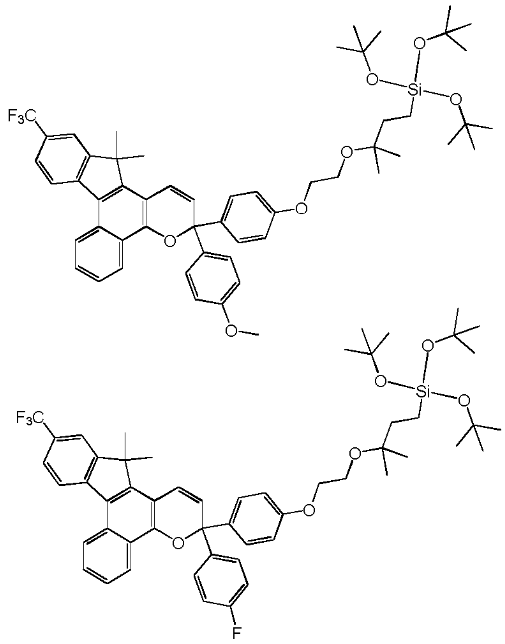

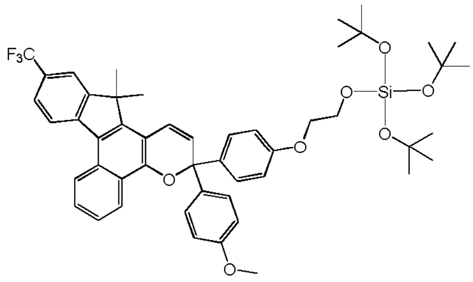

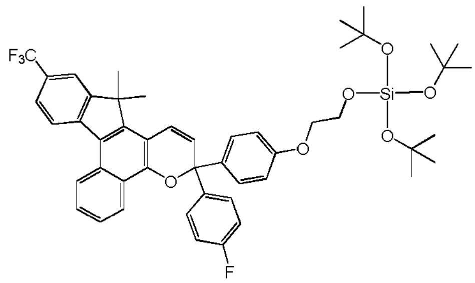

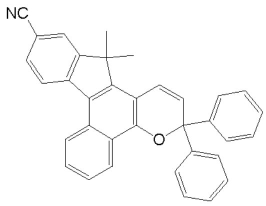
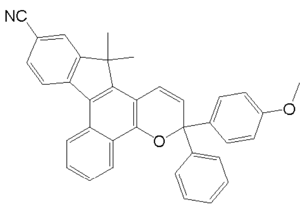
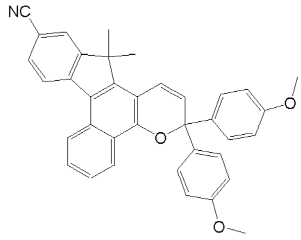
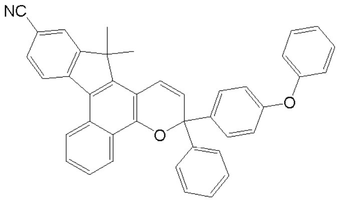
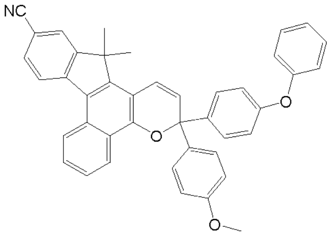
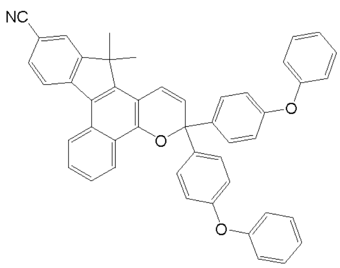
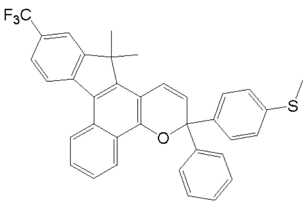
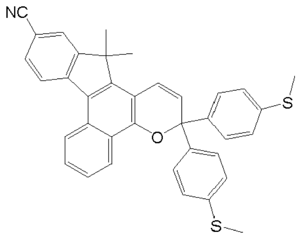
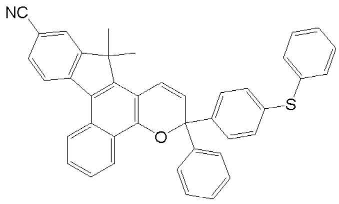
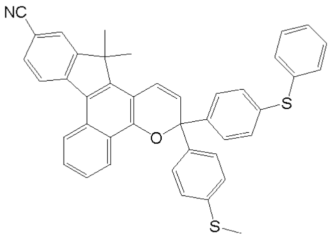
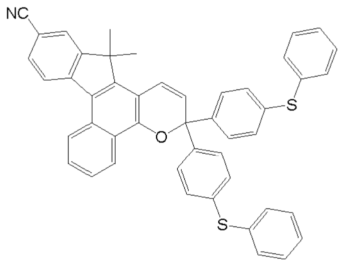
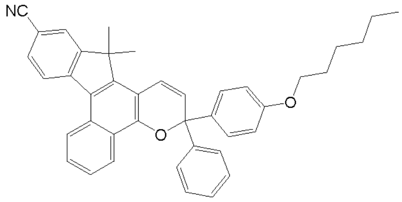
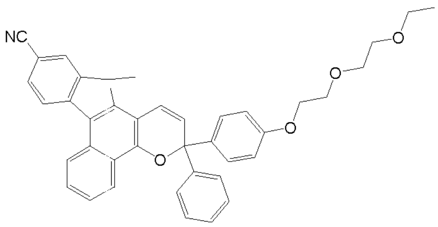
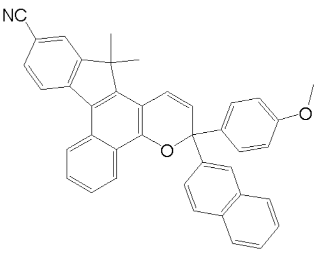
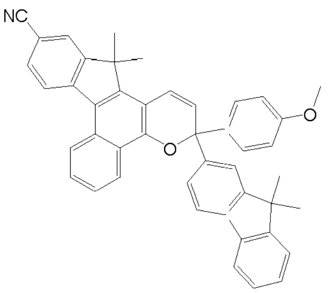
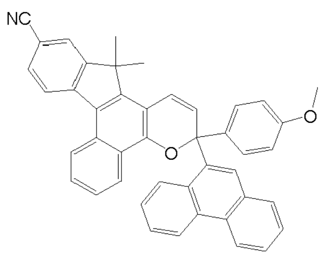

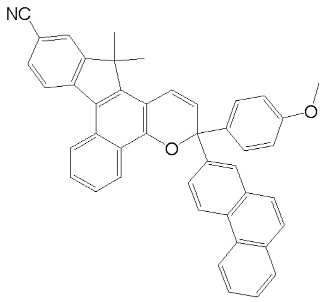
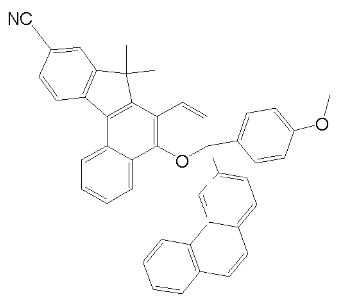
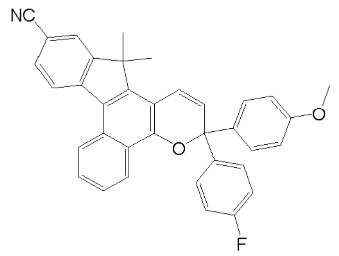
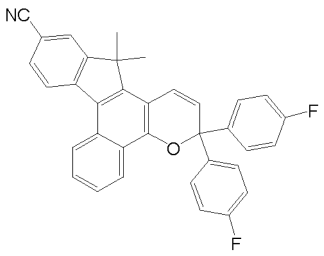
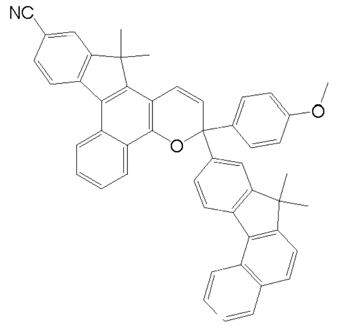
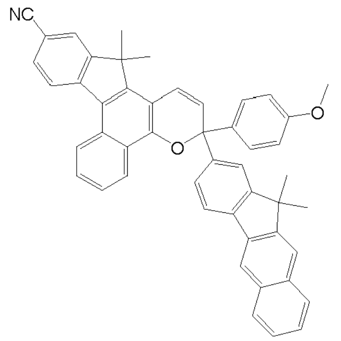
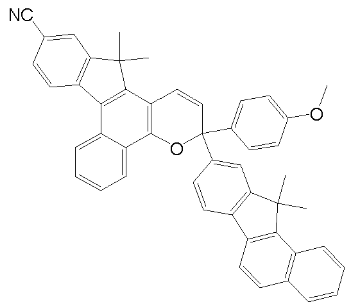
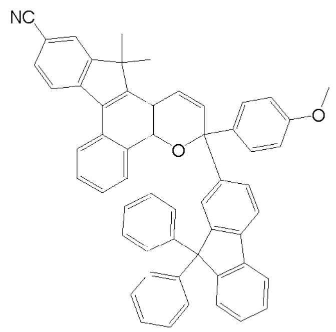
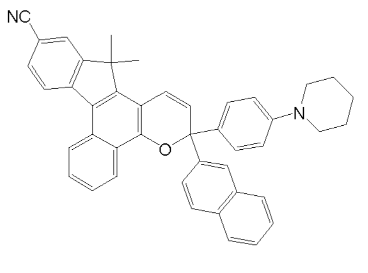
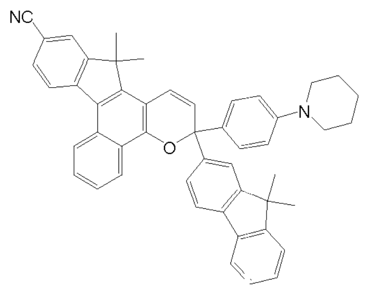
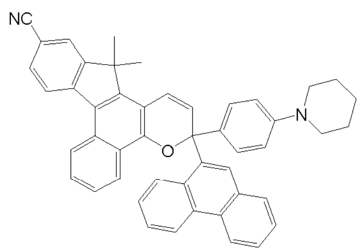
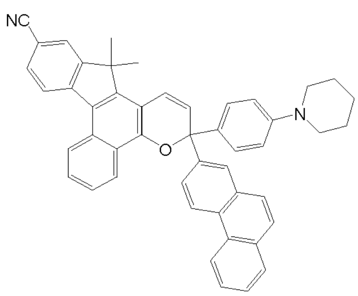
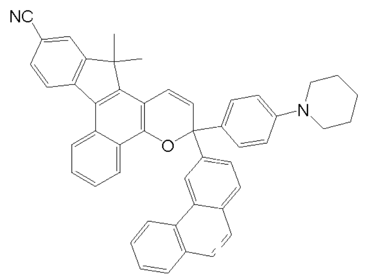
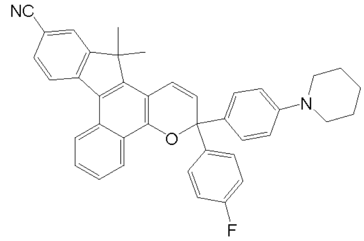

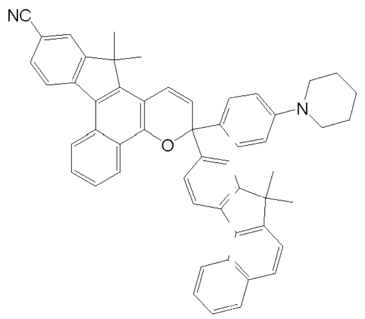

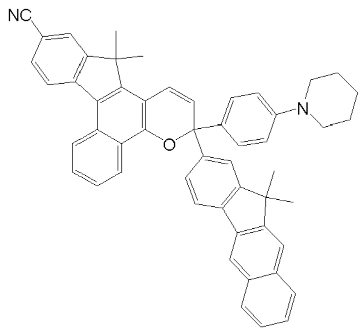

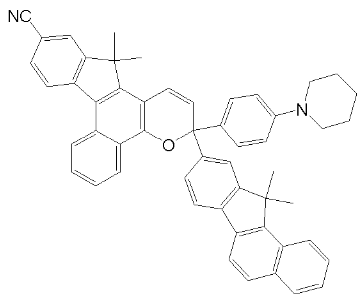

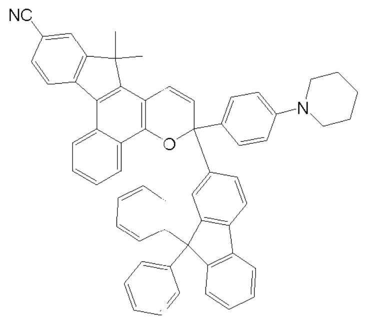

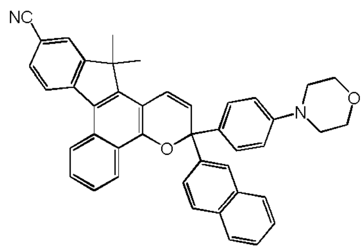

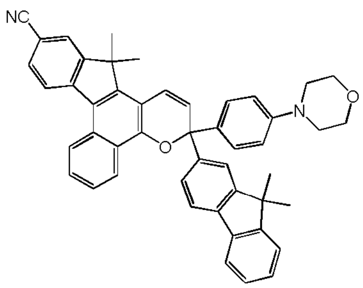

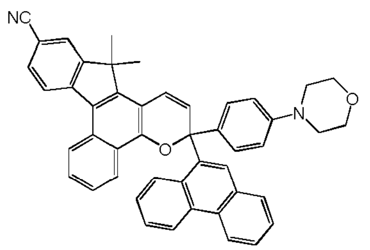

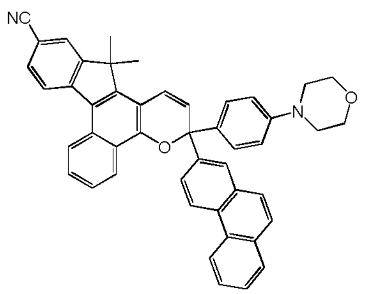

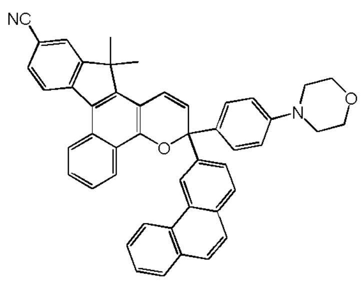

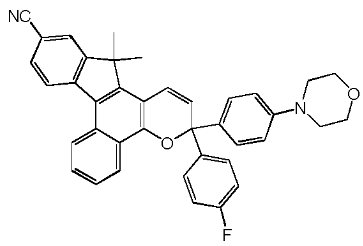

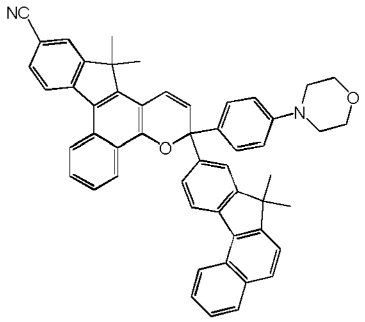

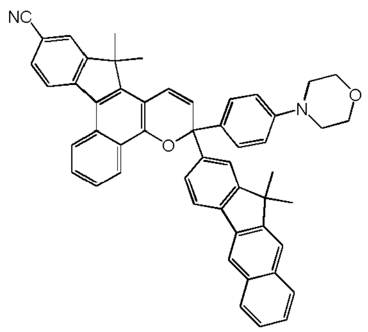

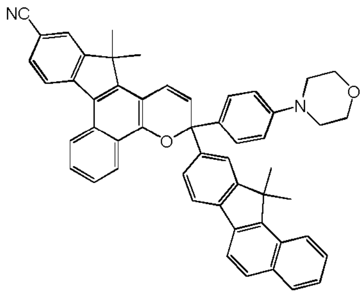

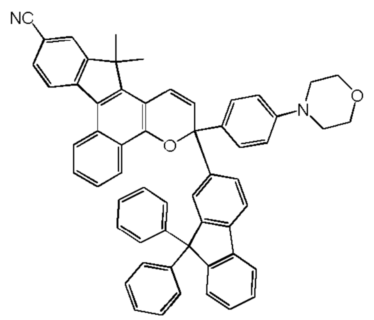

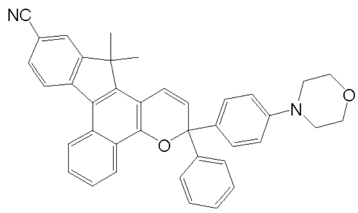

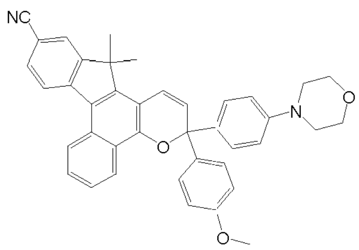

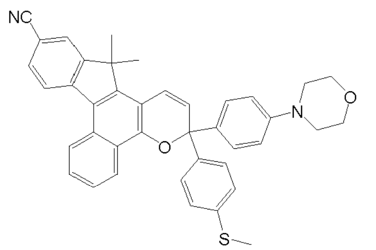

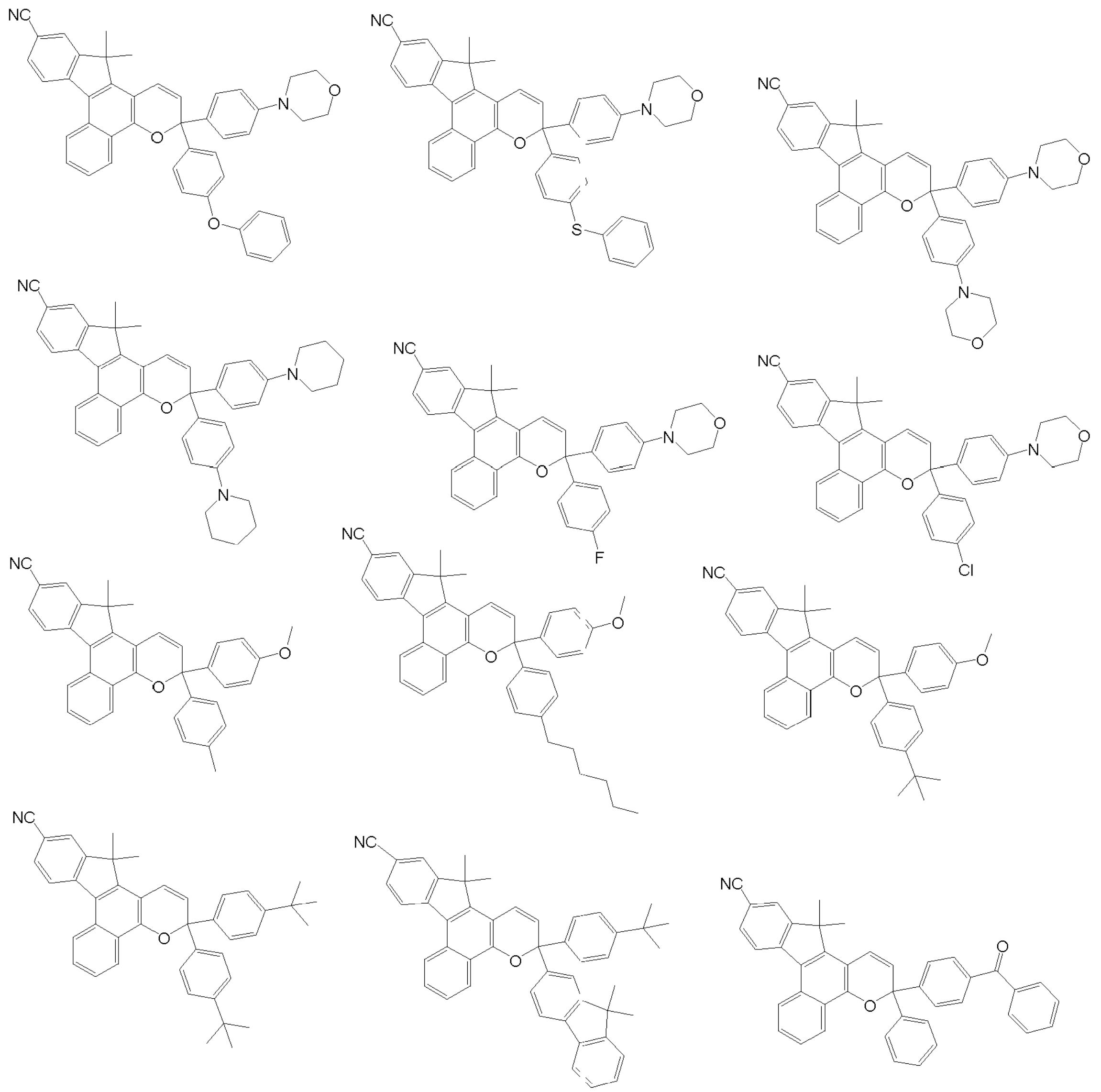

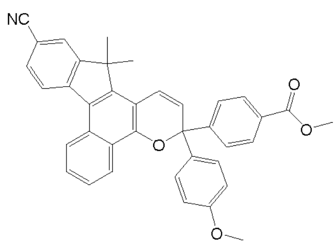

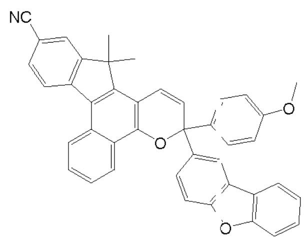

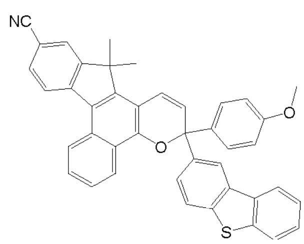

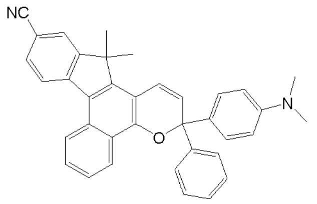

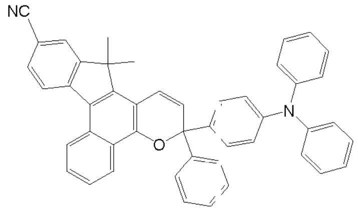

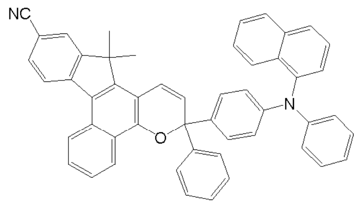

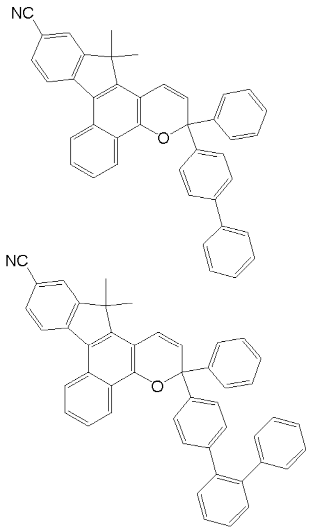

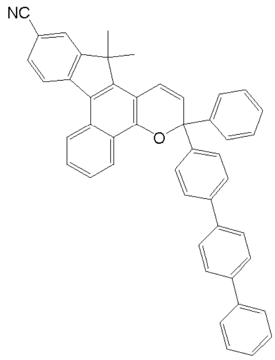

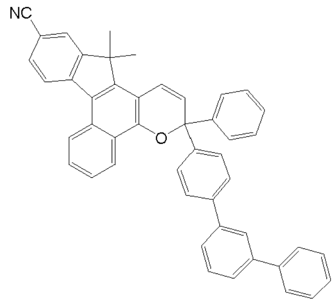

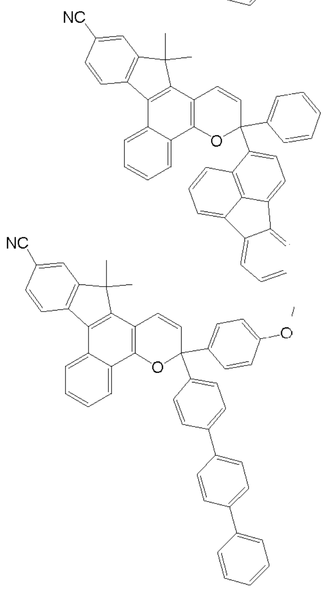

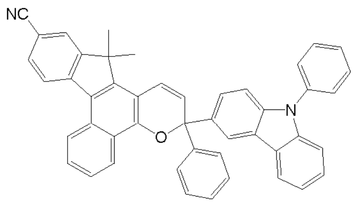

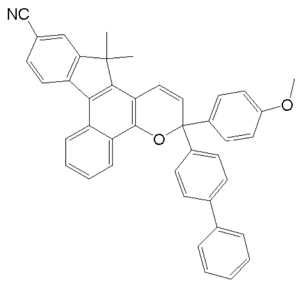

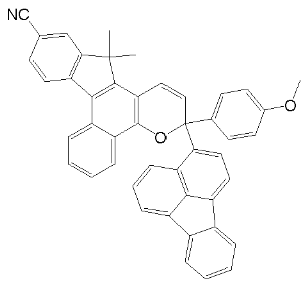

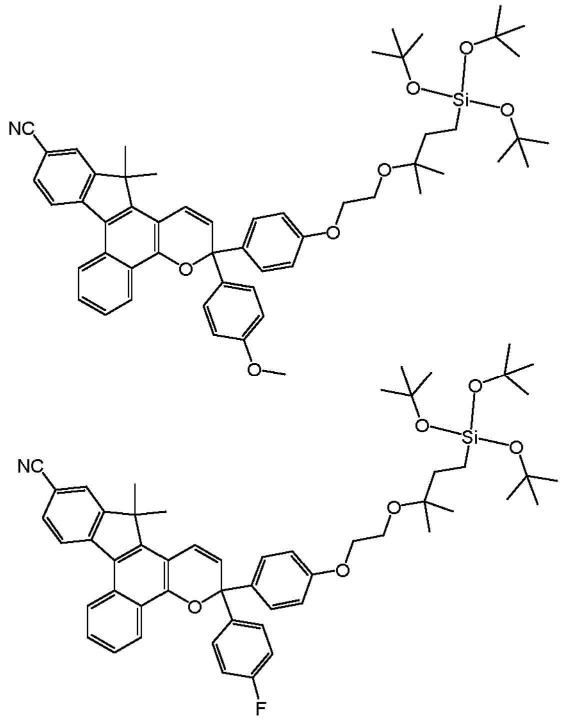

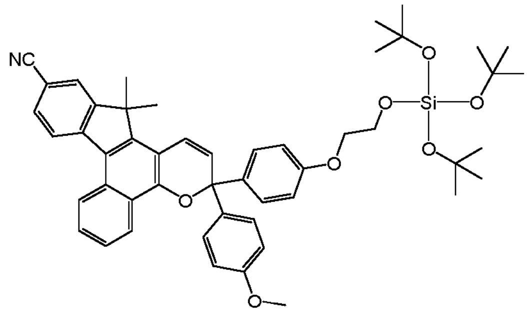

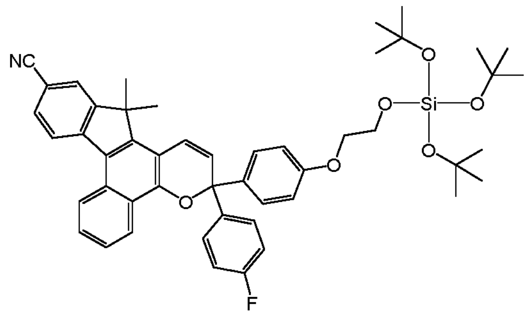

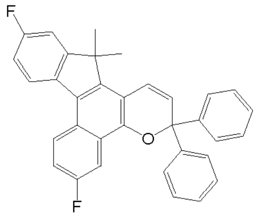

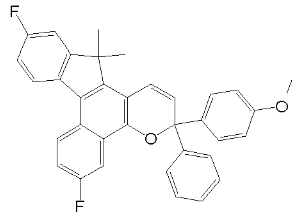

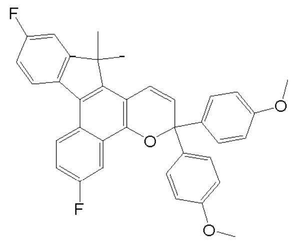

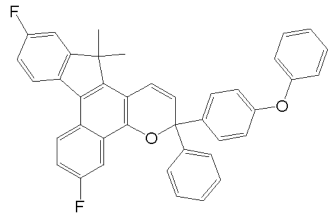

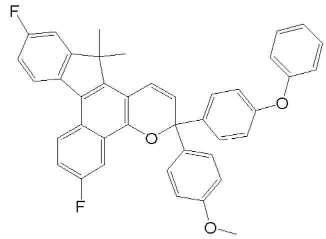

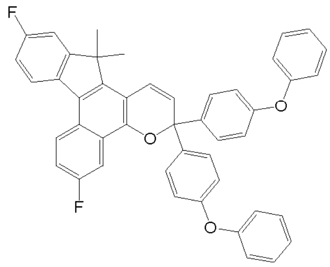

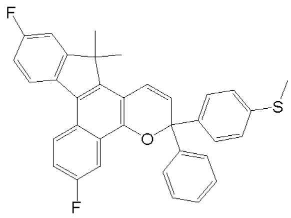

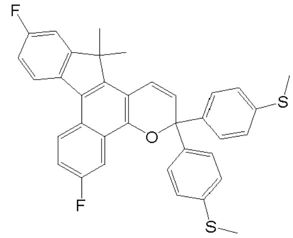

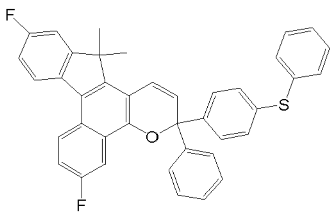

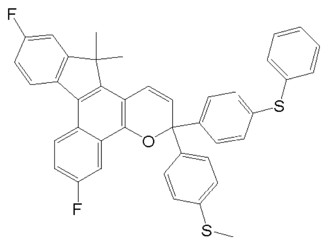

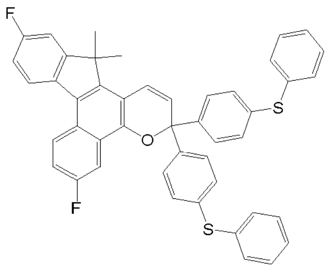

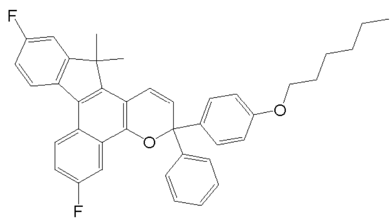

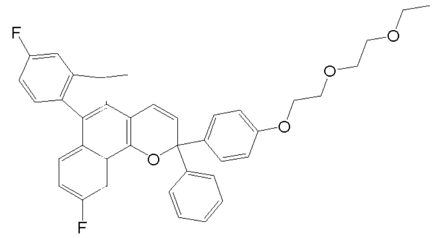

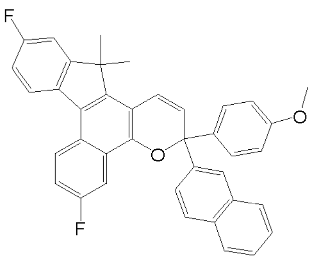

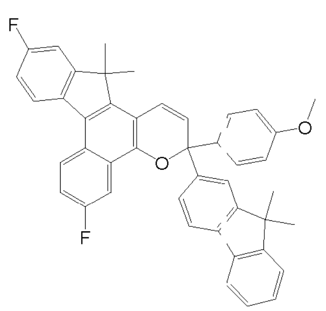

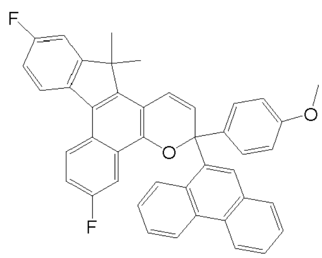

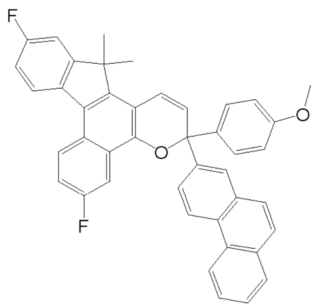
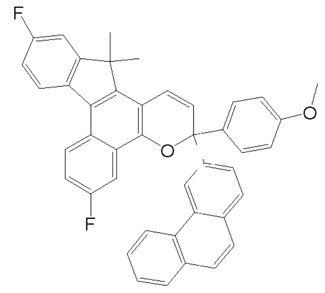
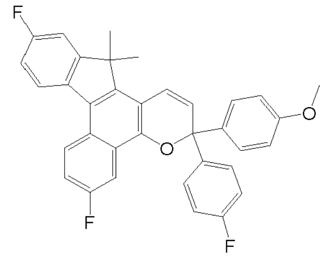
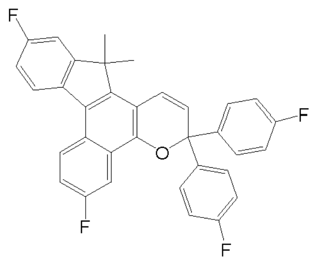
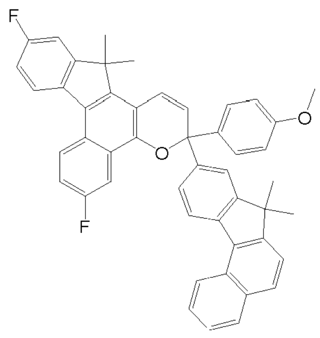
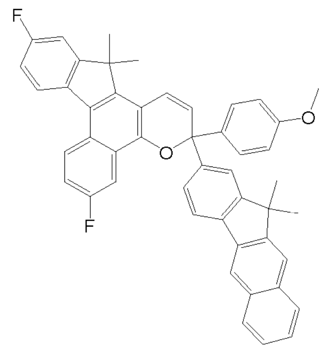
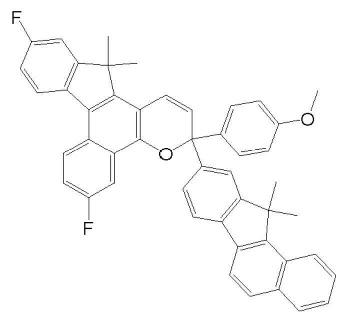
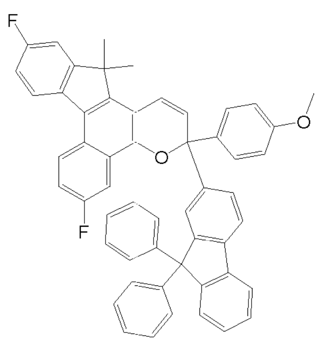
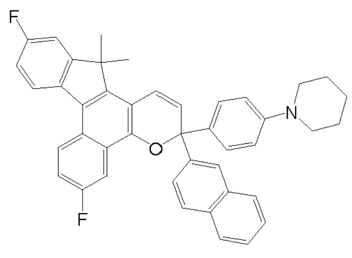
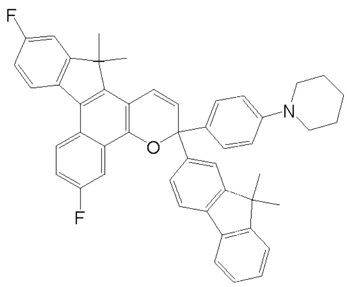
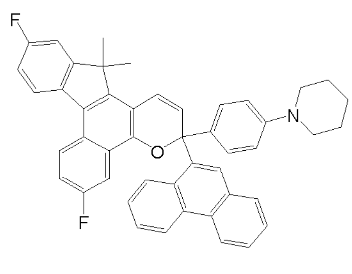
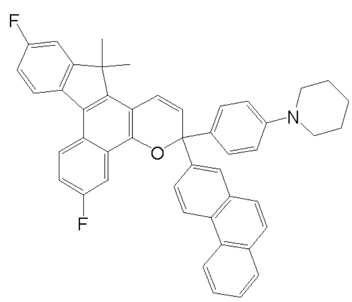
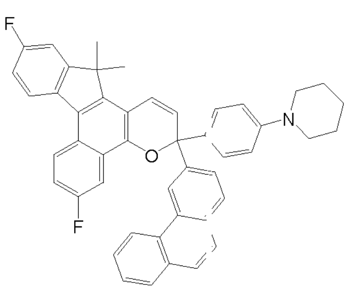
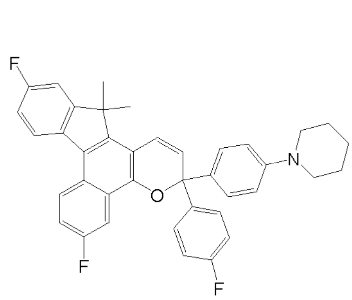

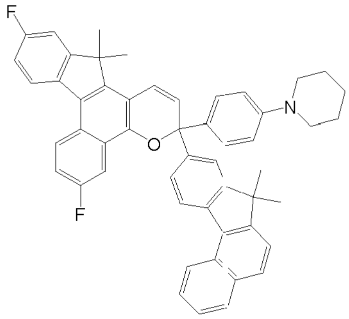

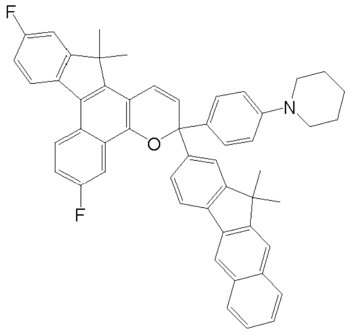

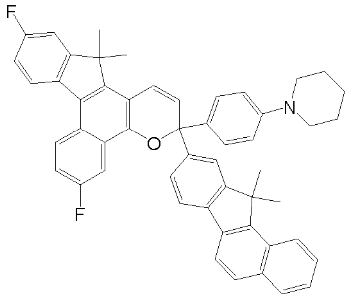

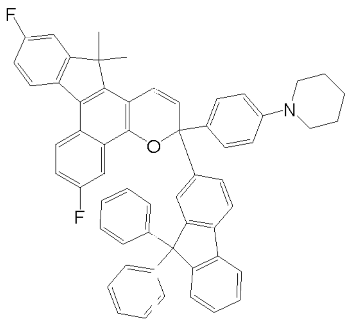

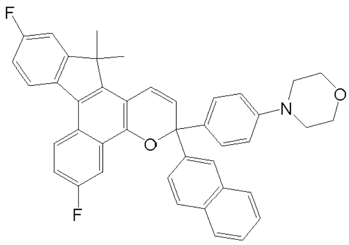

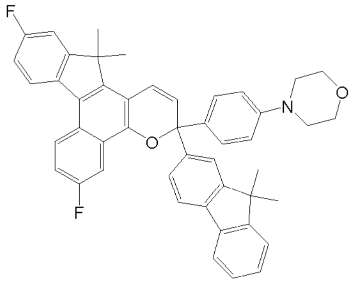

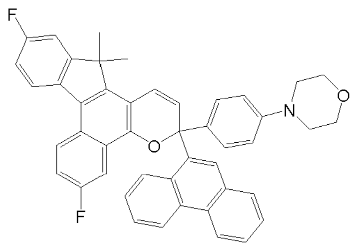

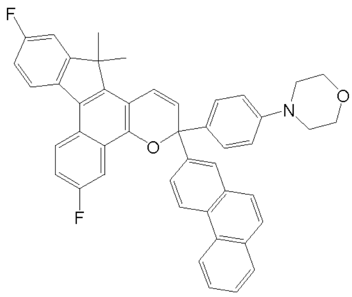

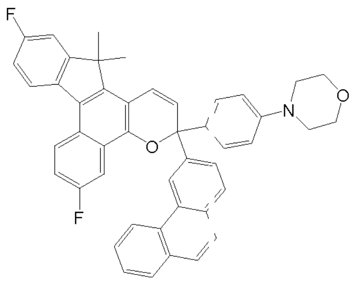

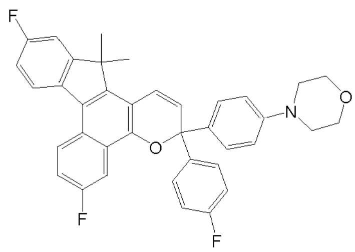

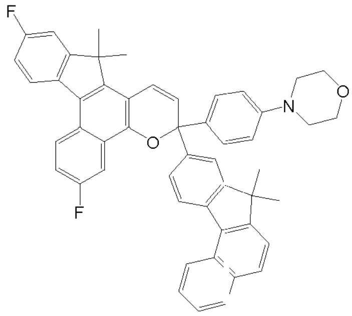

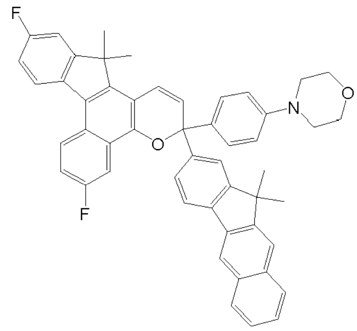

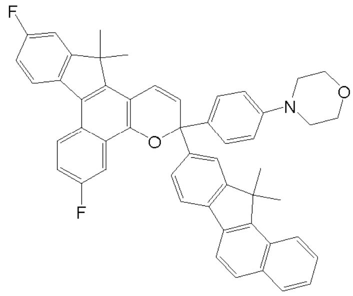

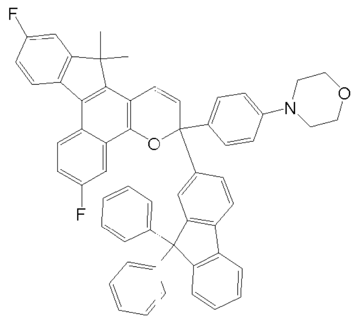

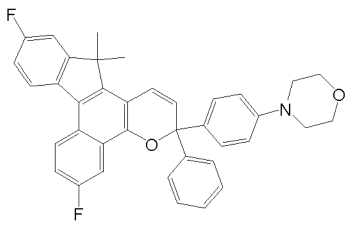

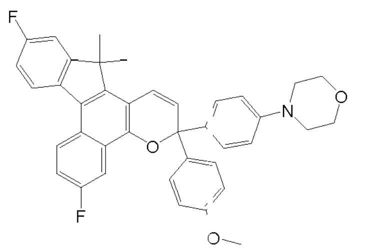

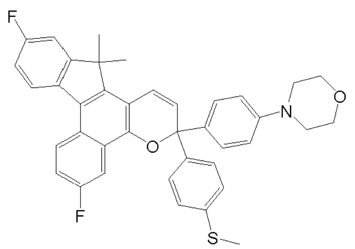

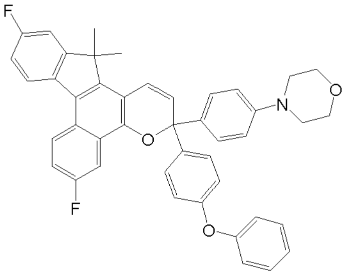
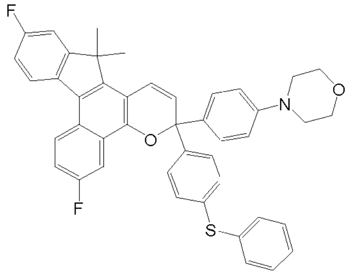
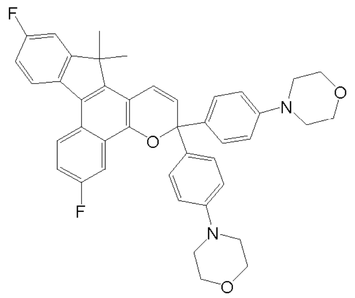
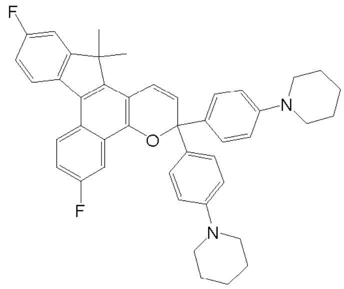
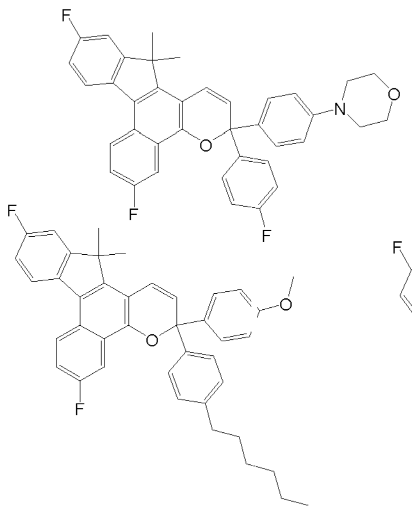
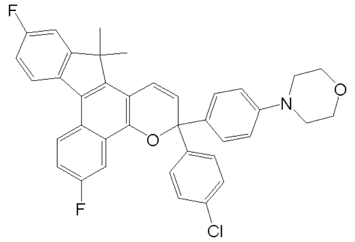
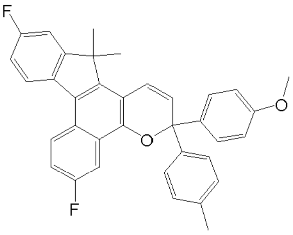
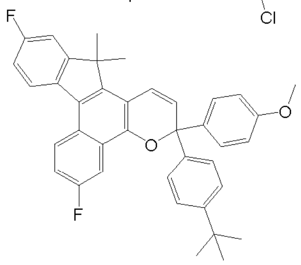
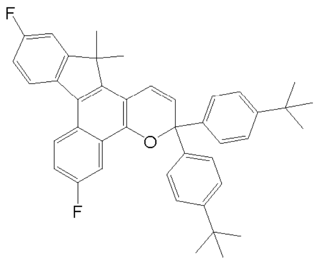
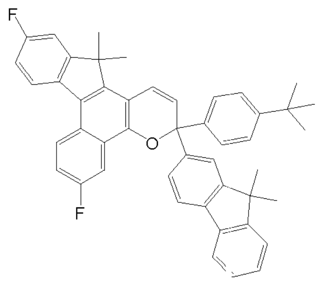
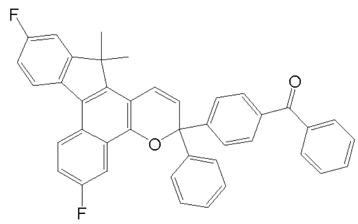
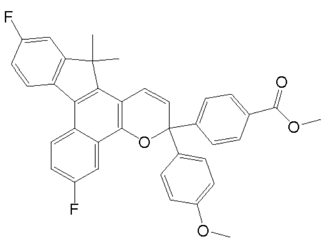
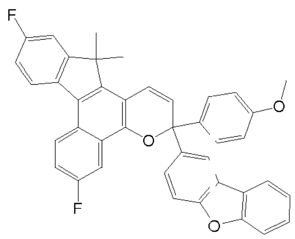
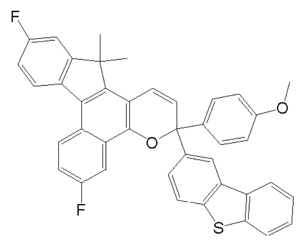

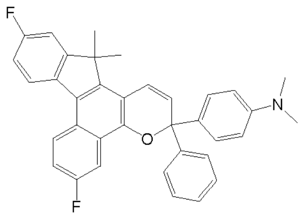

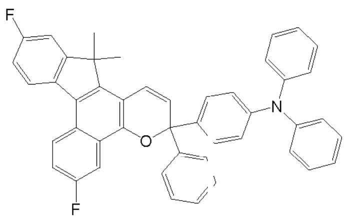

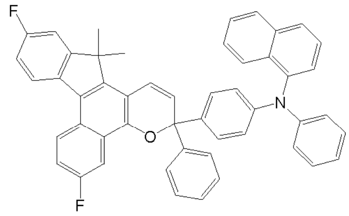

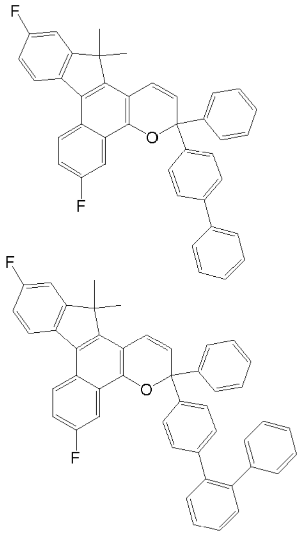

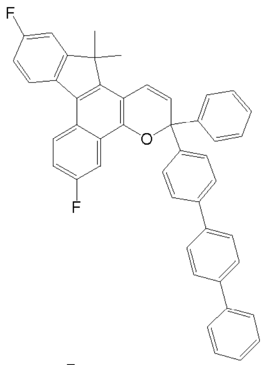

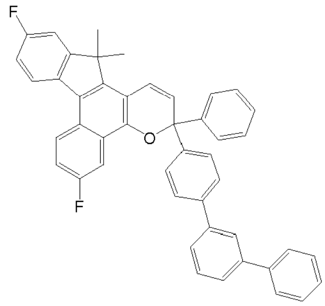

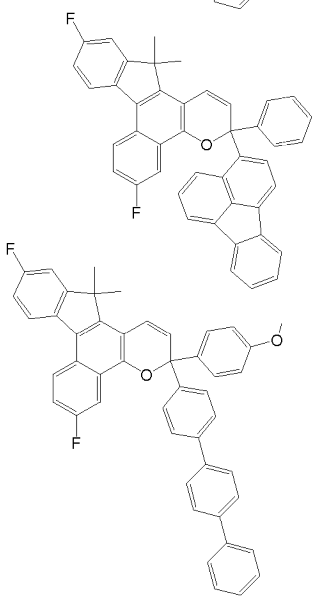

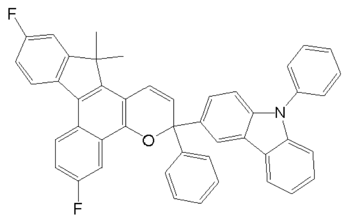

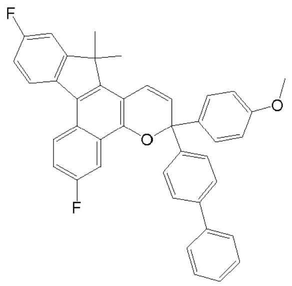

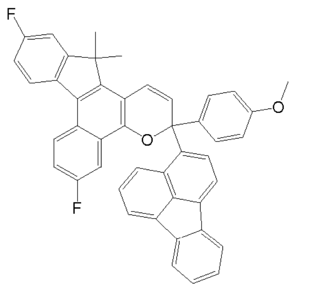

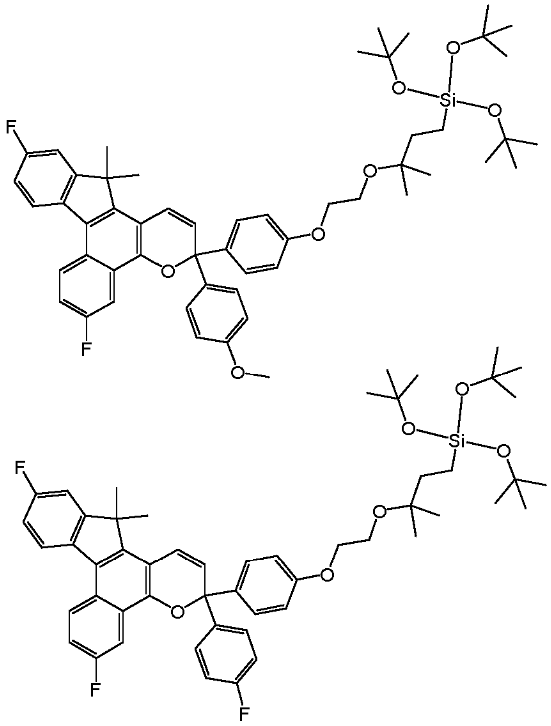

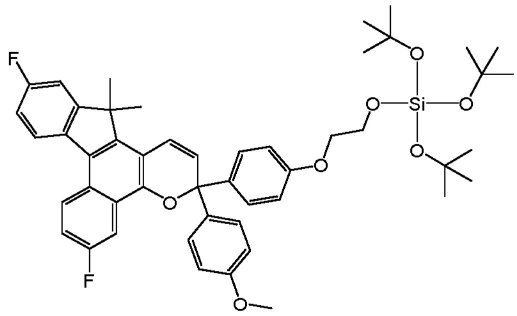

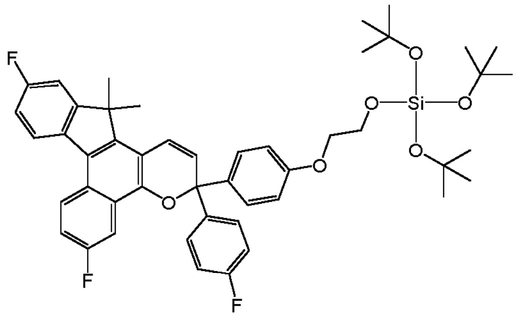

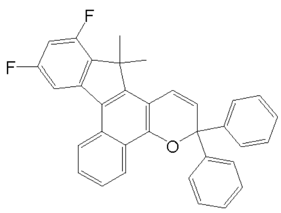

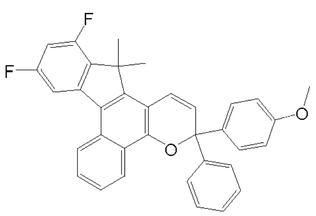

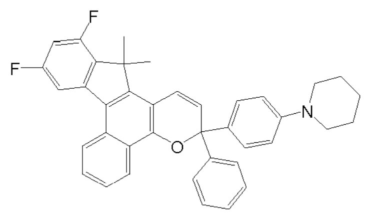

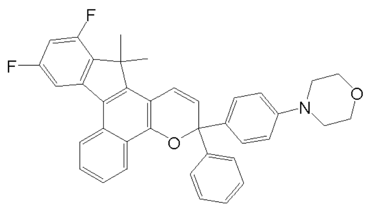

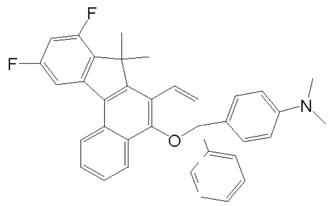

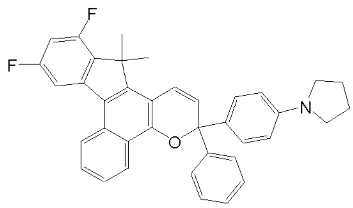

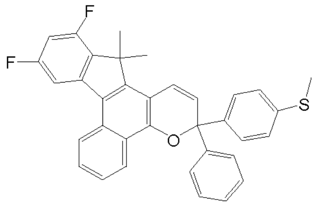
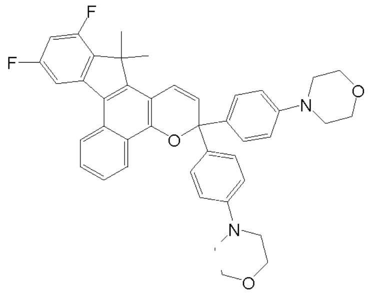
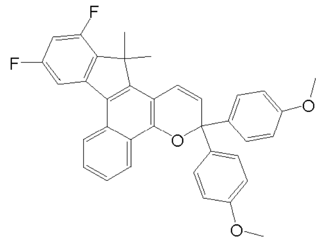
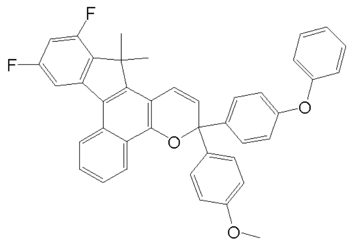
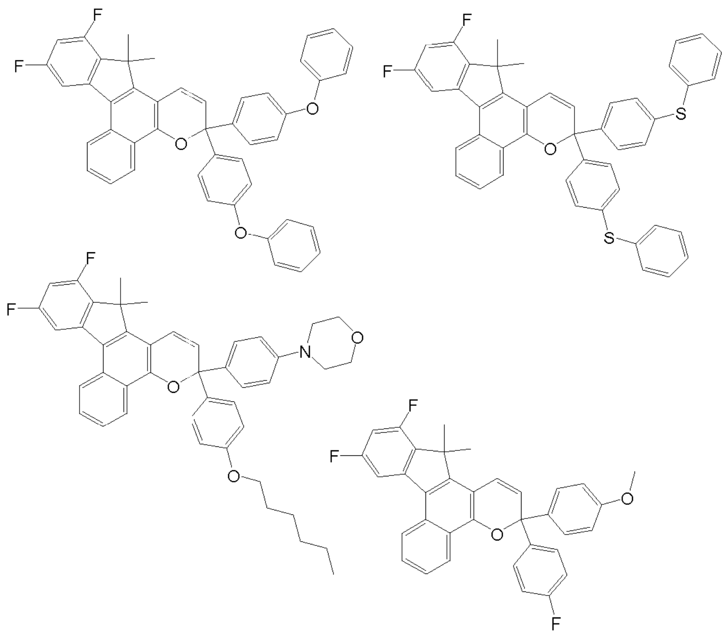
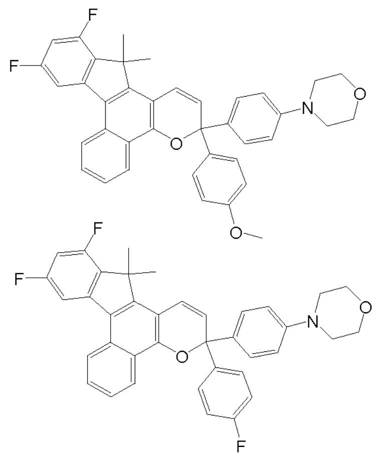
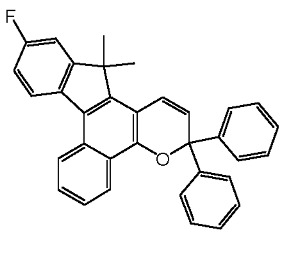
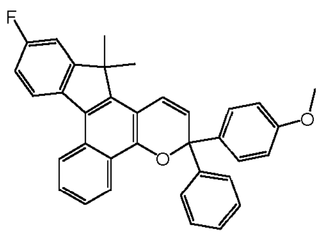
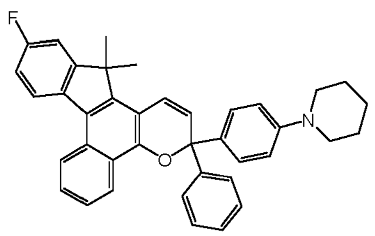

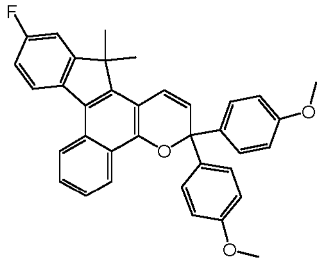

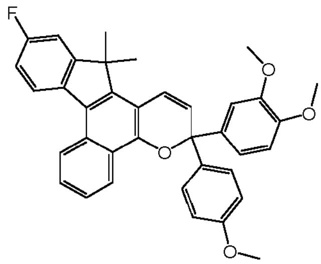

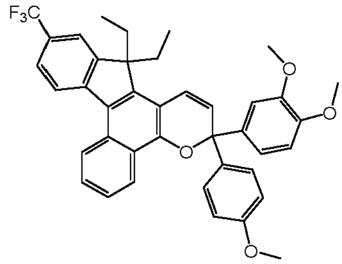

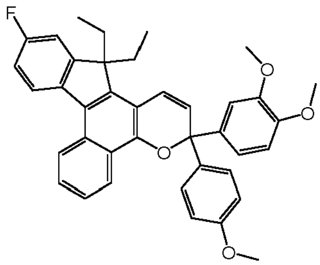

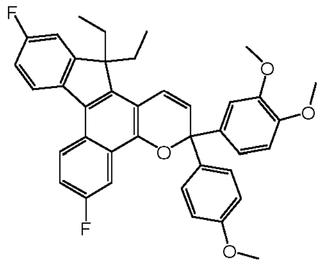

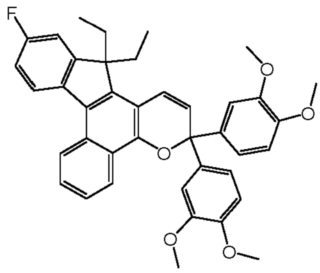

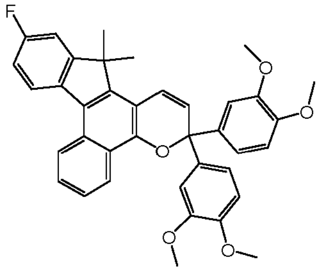

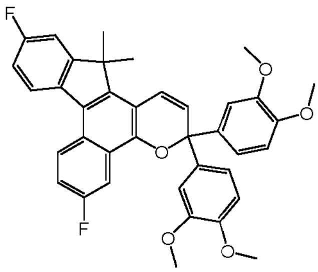

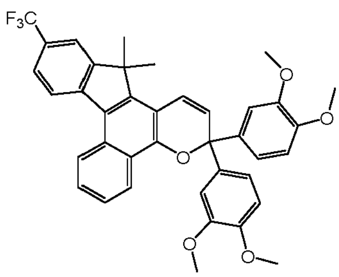

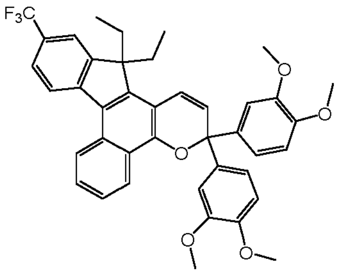

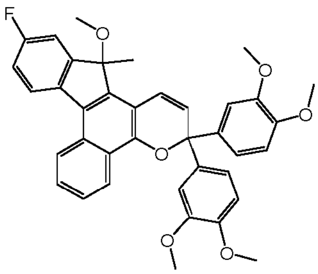

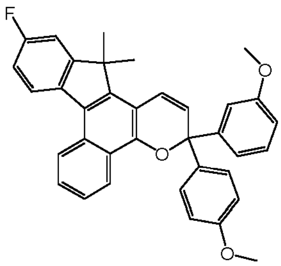

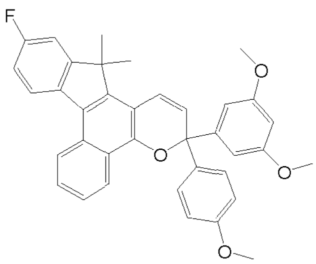

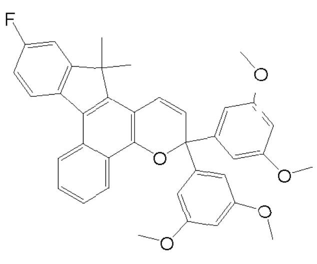

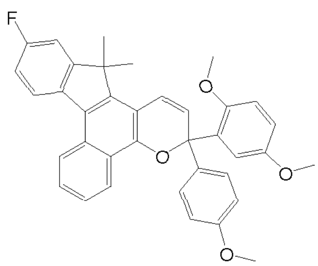

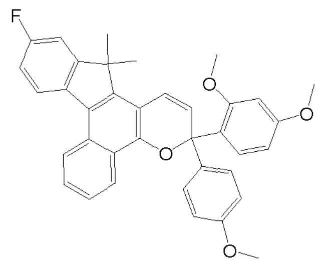
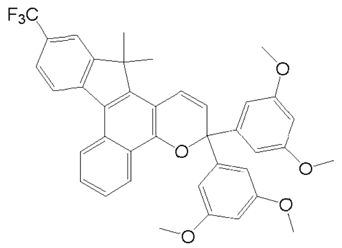
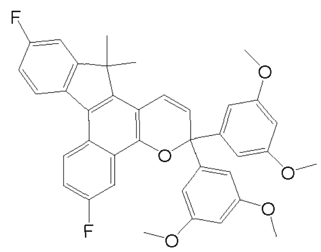

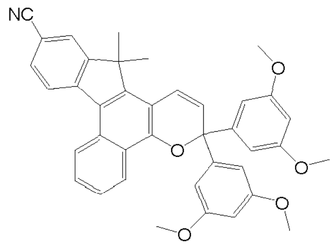
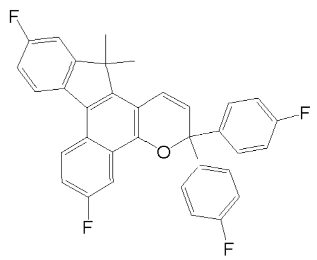
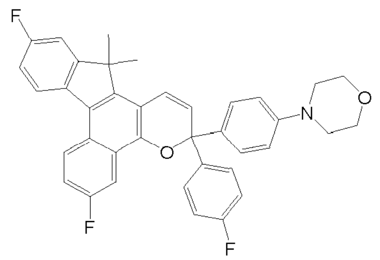

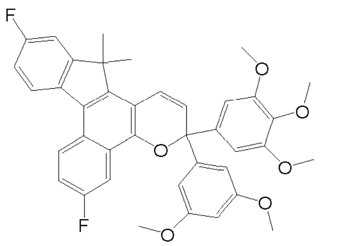
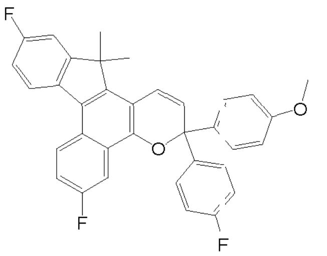
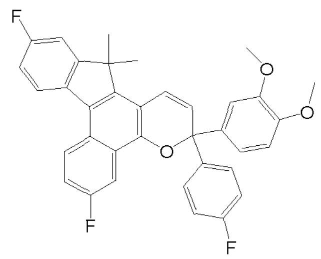

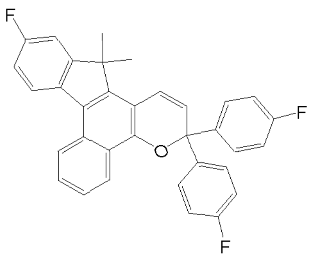
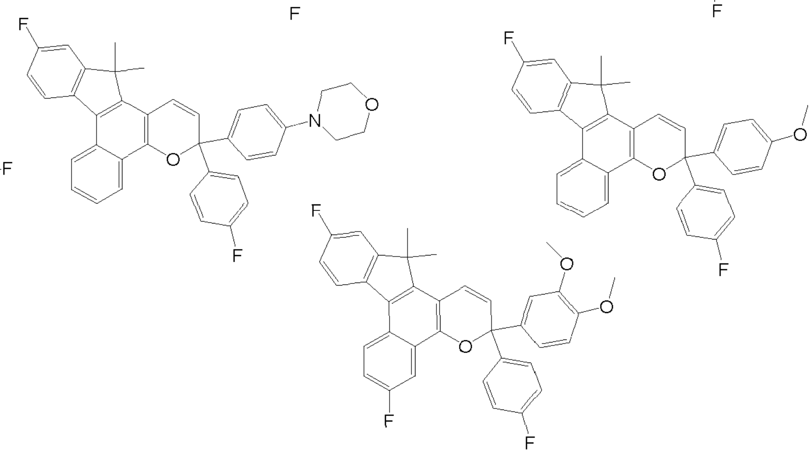

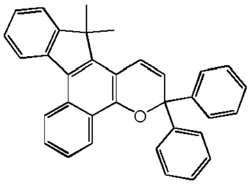
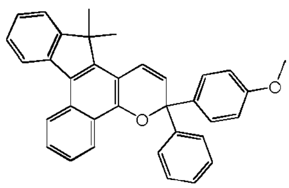
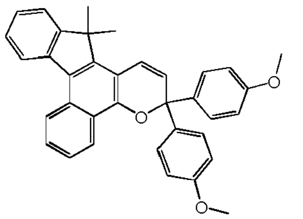
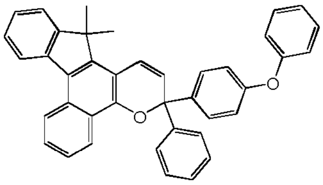
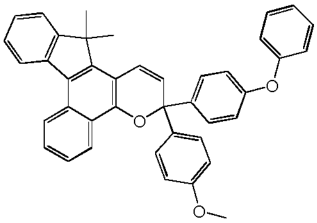
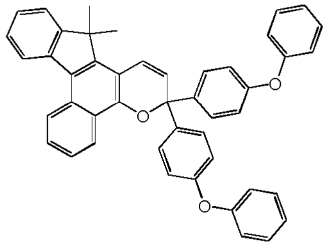
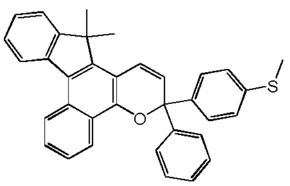
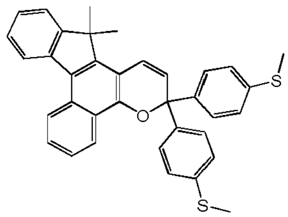
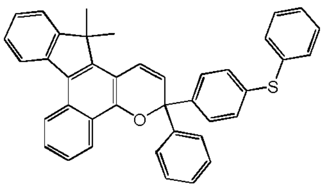
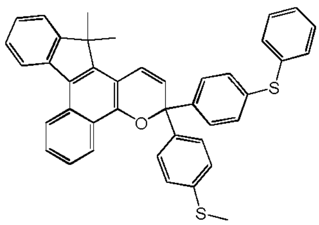
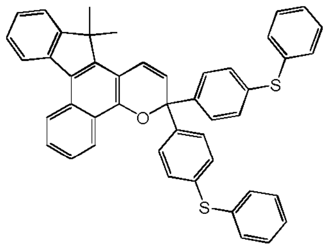

Q

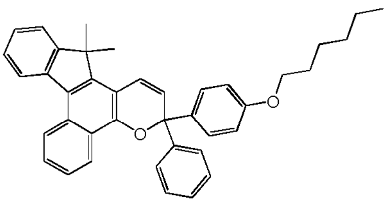
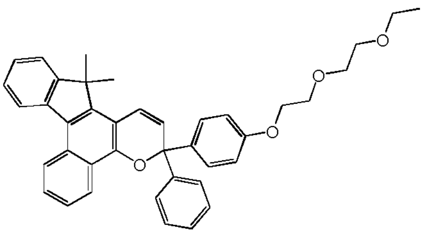
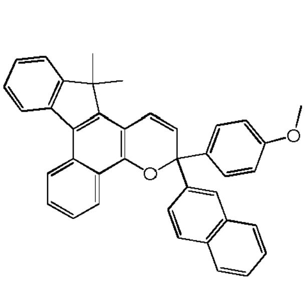
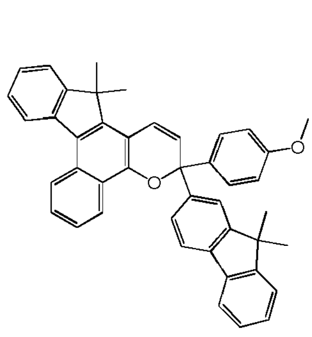
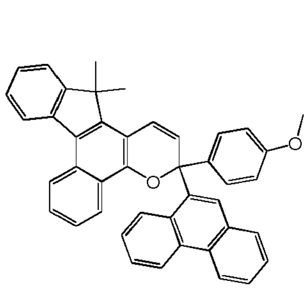

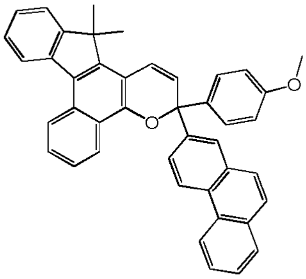
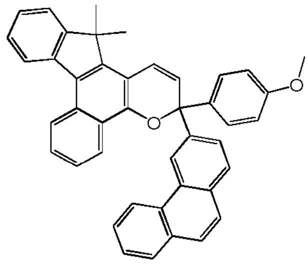
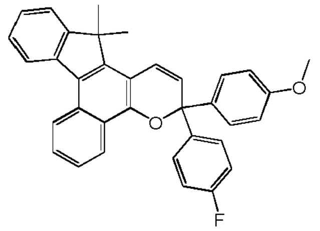

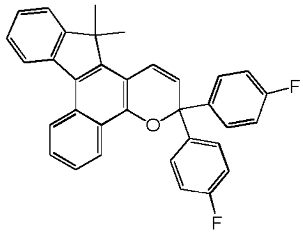
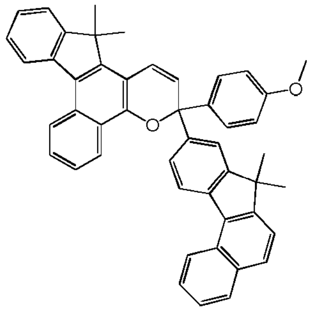
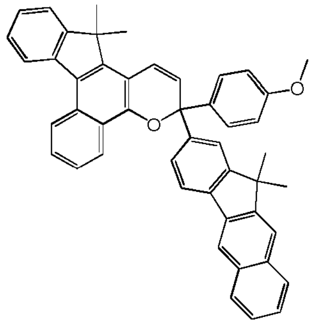

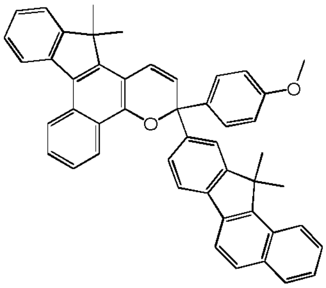
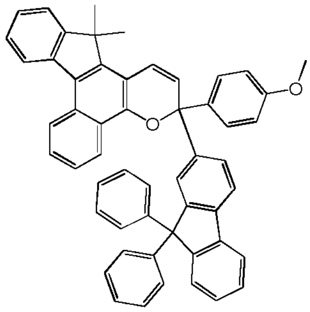

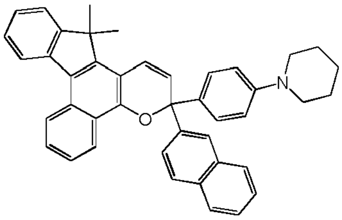
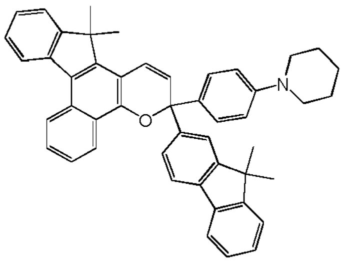
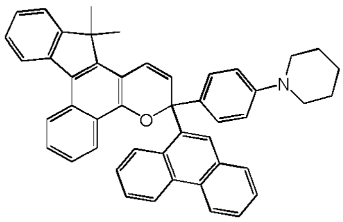

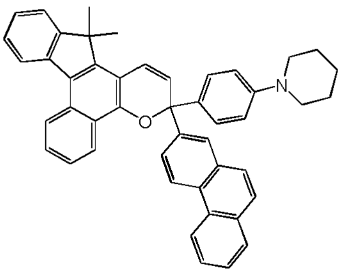
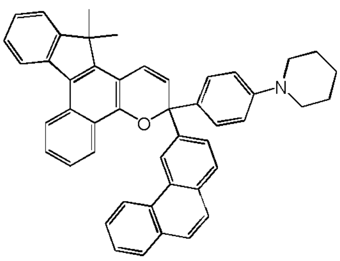
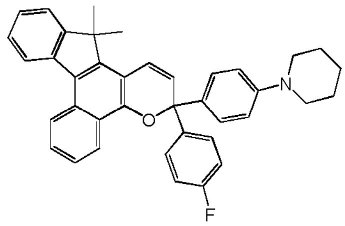

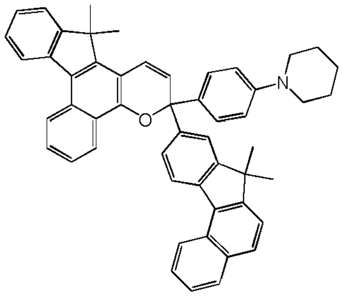

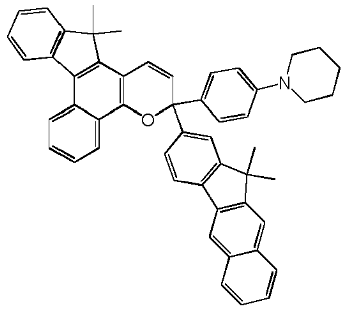

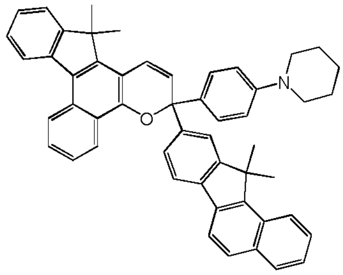

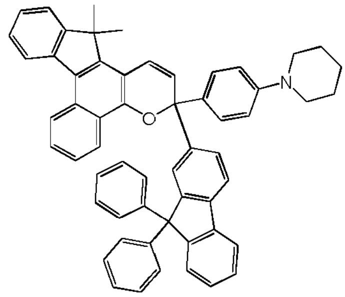

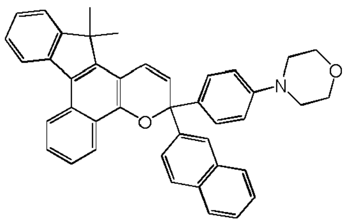

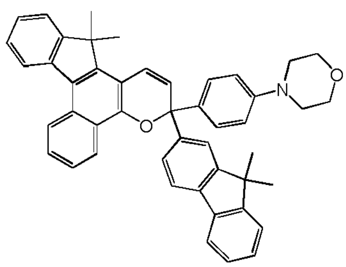

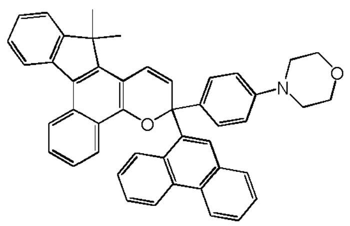

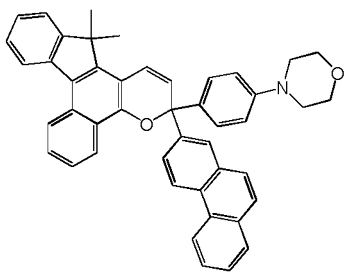

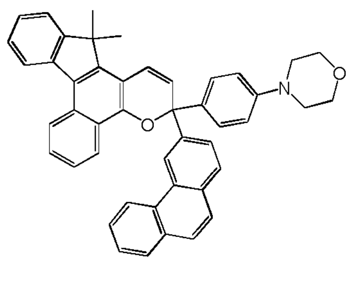

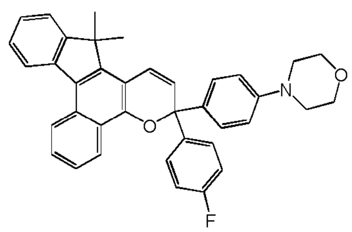

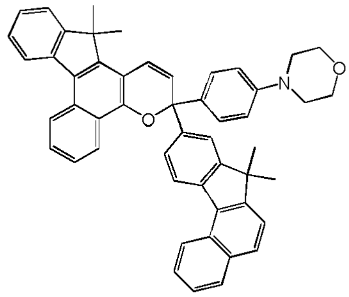

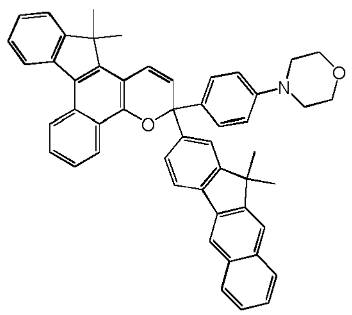

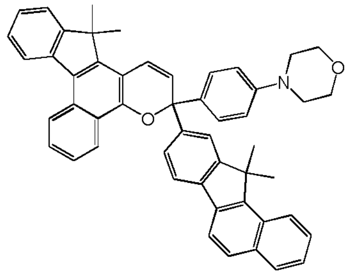

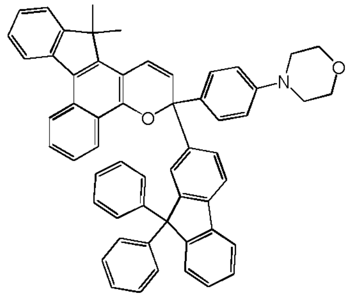

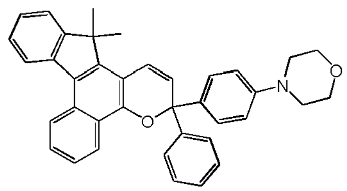

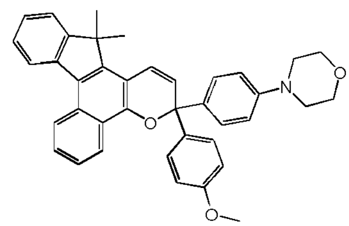

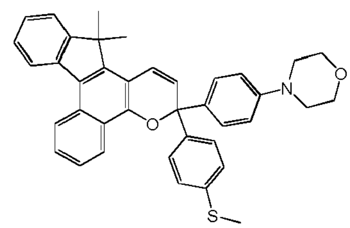

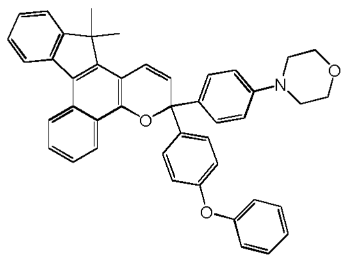

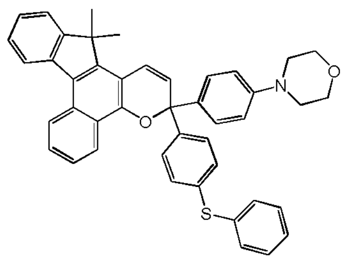

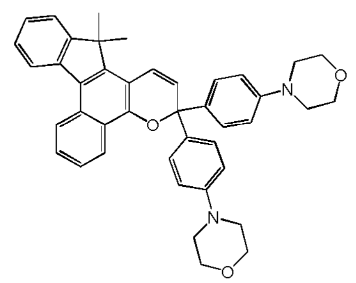

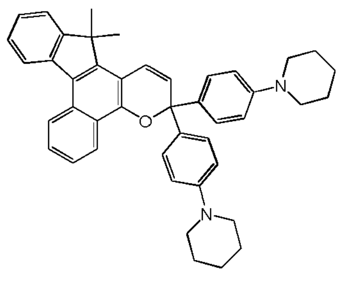

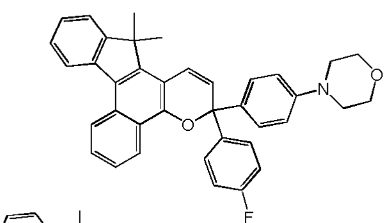

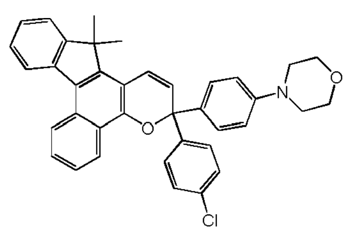

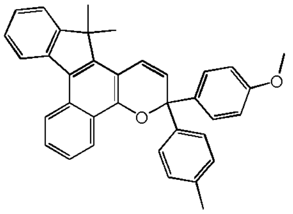

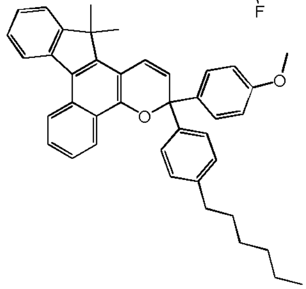

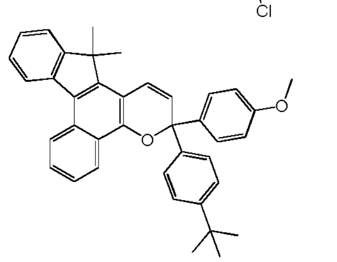

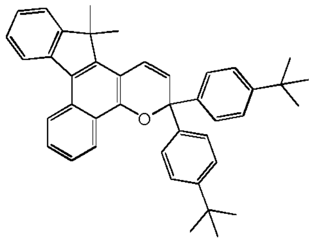
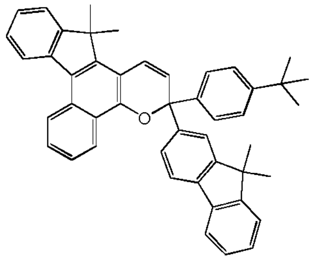
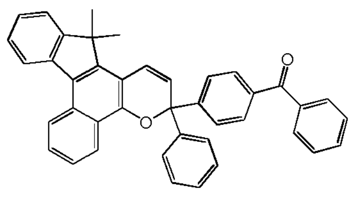
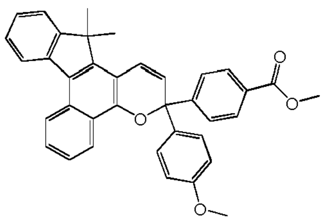
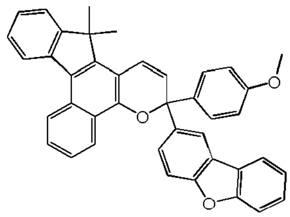
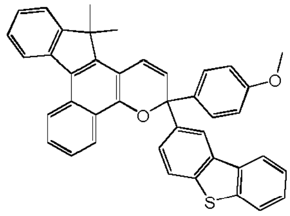
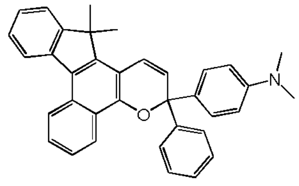
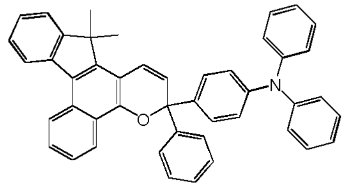
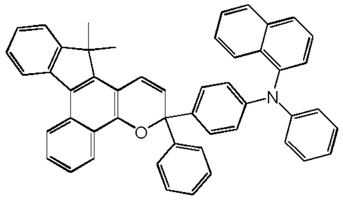
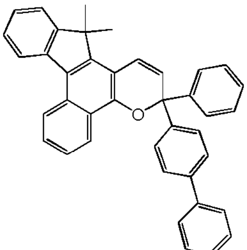
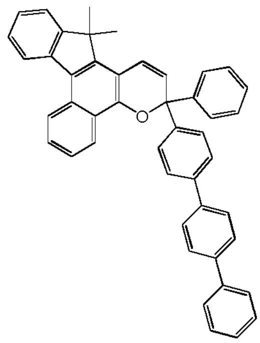
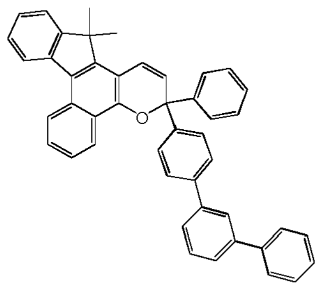
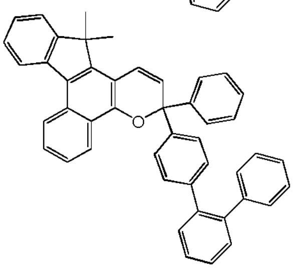
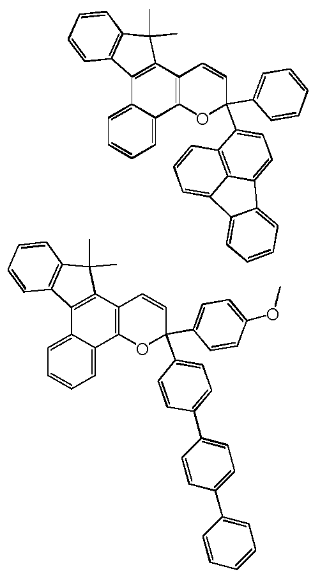
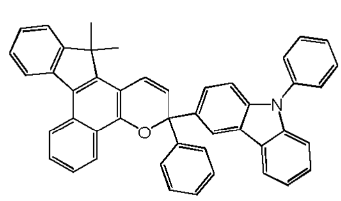
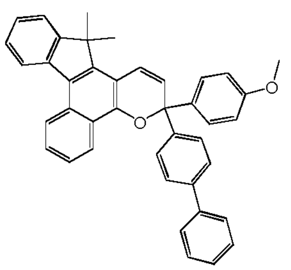
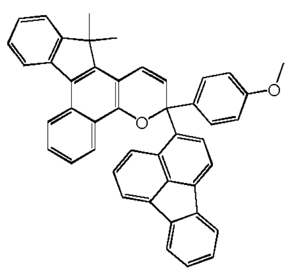

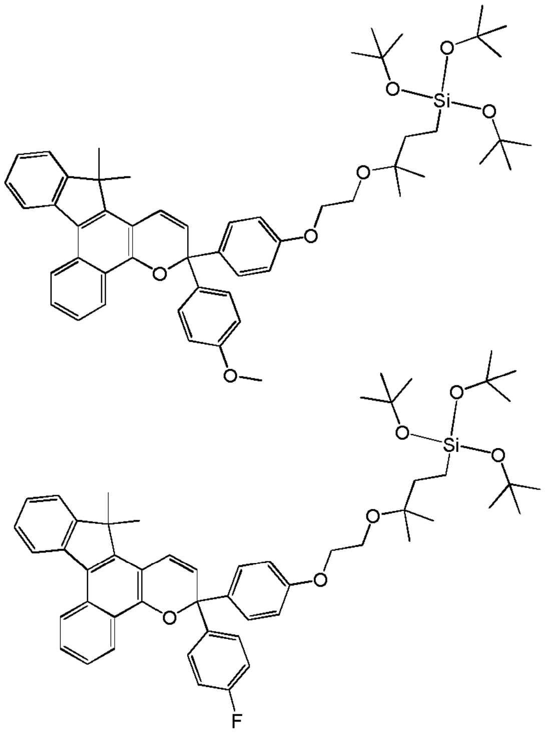

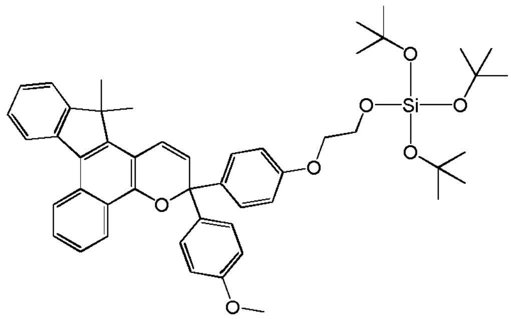

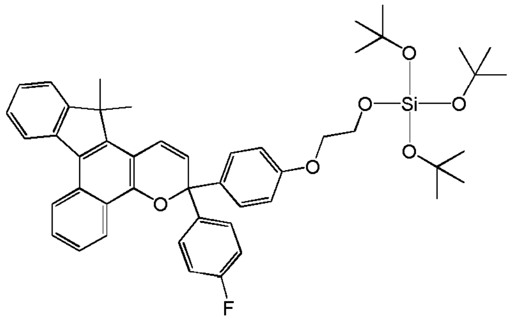

<Compound of Formula (B)>

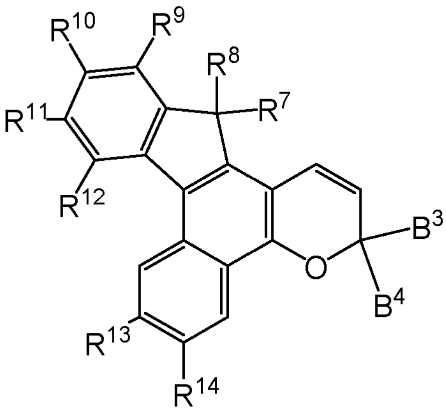

(B)

In Formula (B), $R^7$-$R^{12}$, $B^3$ and $B^4$ each independently are H or a substituent.

$R^7$ and $R^8$ each independently preferably are optionally substituted $C_{1-20}$-alkyl, and more preferably methyl, ethyl, propyl, butyl, pentyl or hexyl. $R^7$ and $R^8$ each independently are still more preferably methyl or ethyl, and more preferably, $R^7$ and $R^8$ both are methyl or both are ethyl.

$B^3$ and $B^4$ each independently represent preferably optionally substituted phenyl. When the phenyl group has a plurality of substituents, two or more of these substituents may be bonded to form a ring. Specific examples of the formed rings include rings included in exemplary compounds listed below. The substitution position of the substituent in the substituted phenyl group is preferably a position that is a para position with respect to a carbon atom to which $B^3$ and $B^4$ are bonded. Specific examples of substituents of substituted phenyl groups include morpholino, piperidino, halogen, alkoxy, and substituents included in exemplary compounds listed below such as the following substituents.

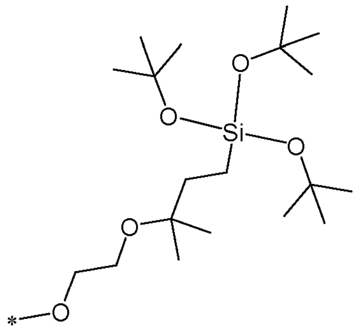

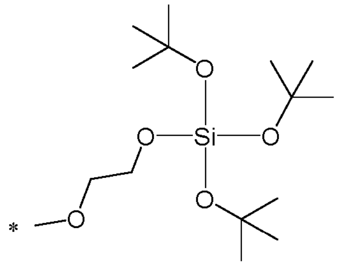

$R^9$-$R^{12}$ each independently are H or a substituent. In one aspect, $R^9$-$R^{12}$ all may be H. In another aspect, $R^{10}$ may be an electron-attracting group, and $R^9$, $R^{11}$ and $R^{12}$ all may be H. In addition, in another aspect, $R^9$ and $R^{11}$ may each independently represent an electron-attracting group, and $R^{10}$ and $R^{12}$ may be H. The electron-attracting group is preferably halogen, $C_{1-6}$-perfluoroalkyl, perfluorophenyl, perfluoroalkylphenyl or cyano. The halogen atom is preferably F. The $C_{1-6}$-perfluoroalkyl group is preferably trifluoromethyl.

In one aspect, $R^{10}$ may be optionally substituted phenyl, and preferably, $R^{10}$ may be a optionally substituted phenyl group and $R^9$, $R^{11}$ and $R^{12}$ may be H. Specific examples of such substituted phenyl groups include phenyl groups substituted with one or more halogen atoms and/or one or more cyano groups are, for example, phenyl groups substituted with halogen atoms (preferably, fluorine atoms) at all five substitution positions of the phenyl group, and monosubstituted phenyl groups substituted with a cyano group at a position that is a para position with respect to a carbon atom to which $R^{10}$ is bonded.

$R^{13}$ and $R^{14}$ each independently represent an electron-donating group. That is, $R^{13}$ and $R^{14}$ represent the same or different electron-donating groups. $R^{13}$ and $R^{14}$ each independently preferably represent an electron-donating group selected from methoxy, ethoxy, phenoxy, methylsulfide, phenylsulfide, dimethylamino, pyrrolidino, piperidino, morpholino and thiomorpholino. Among these, for $R^{13}$ and $R^{14}$, preferably, $R^{13}$ is morpholino and $R^{14}$ is alkoxy (preferably, methoxy), $R^{13}$ is morpholino and $R^{14}$ is methylsulfide ($-S-CH_3$), $R^{13}$ and $R^{14}$ both are alkoxy (preferably, methoxy), and $R^{13}$ and $R^{14}$ both are methylsulfide.

Examples of compounds of Formula (B) include the following compounds..

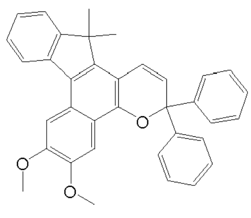

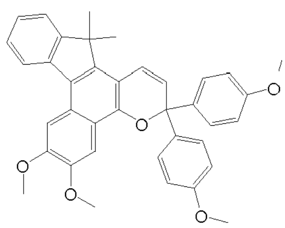

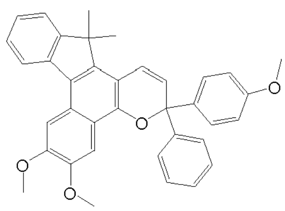

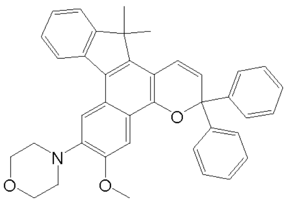

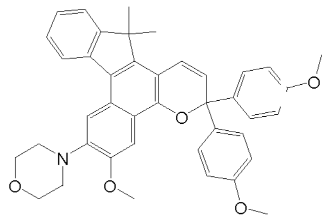

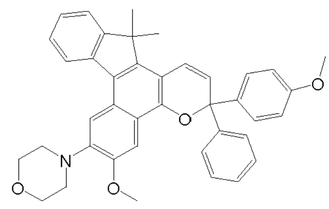

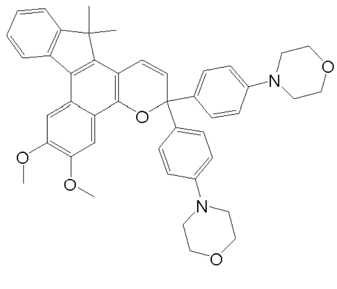

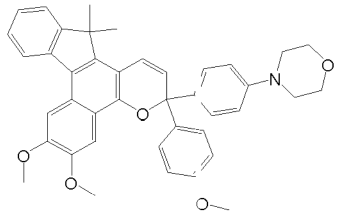

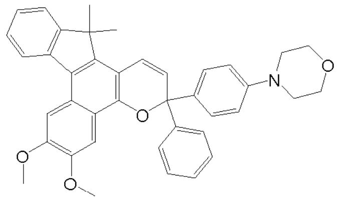

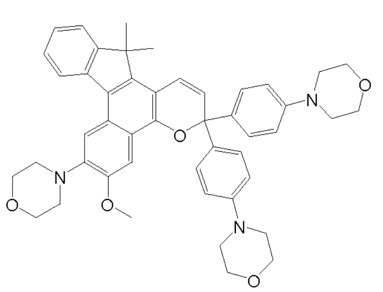
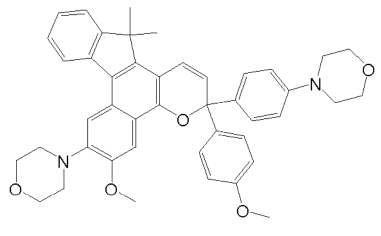
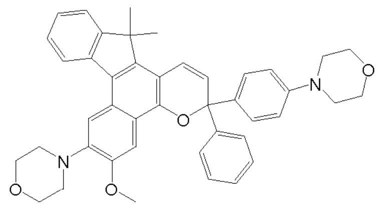

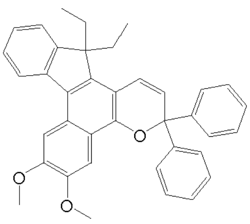
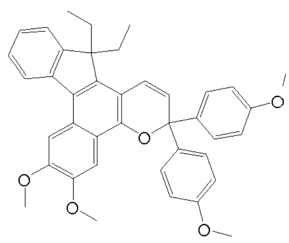
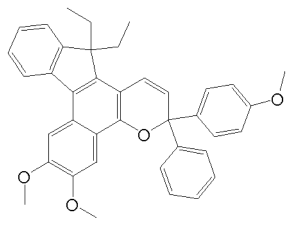

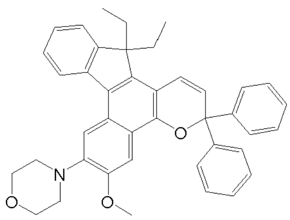
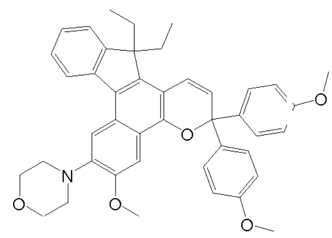
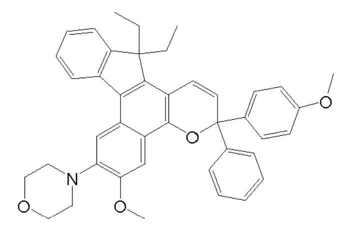

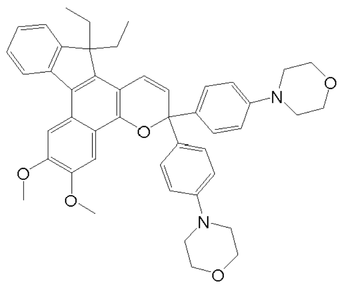
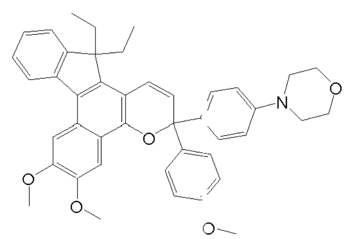
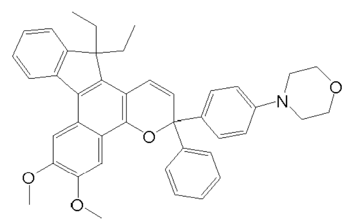

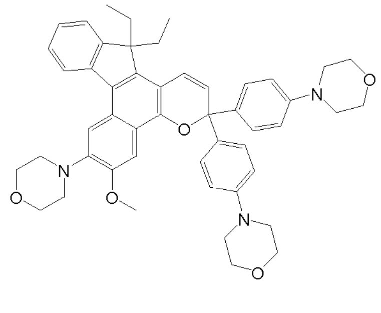
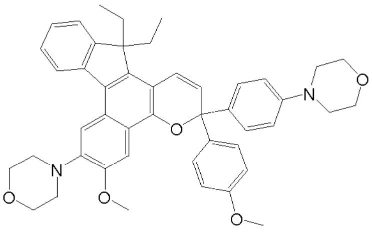
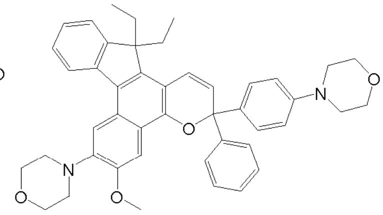

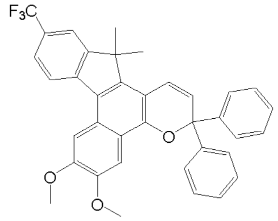
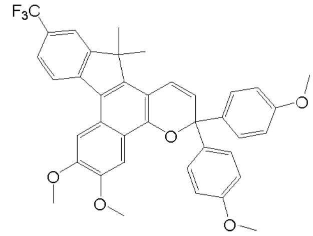
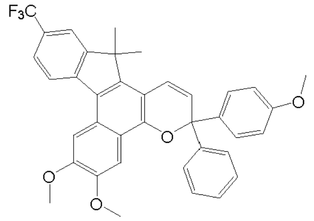

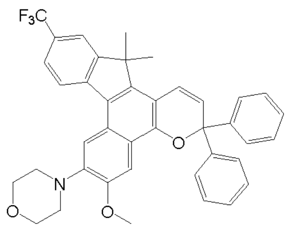
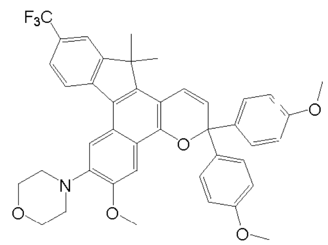
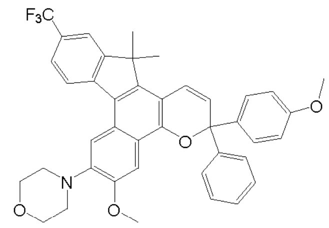
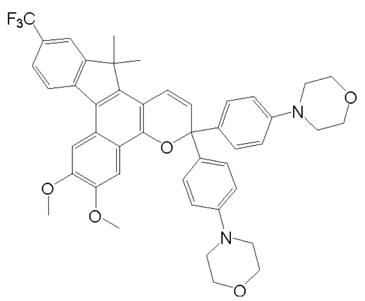
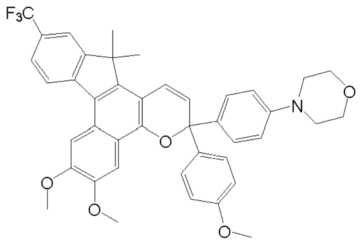
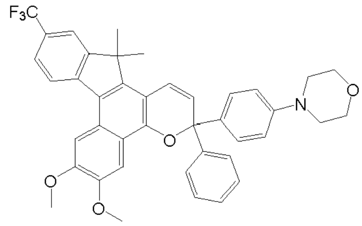
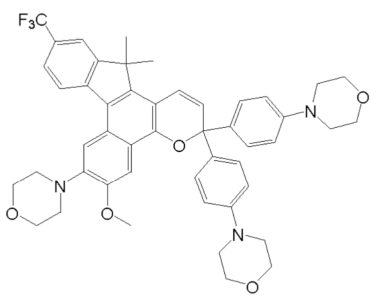
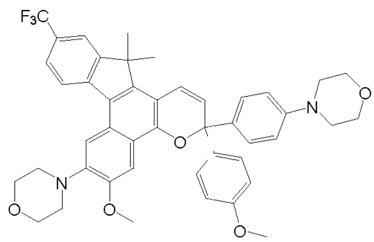
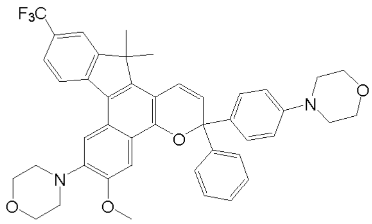
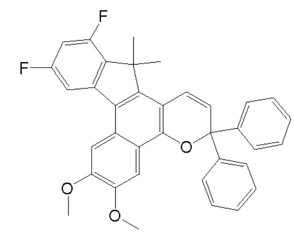
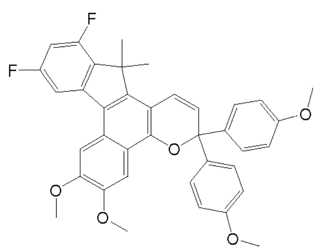
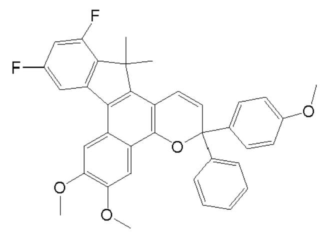
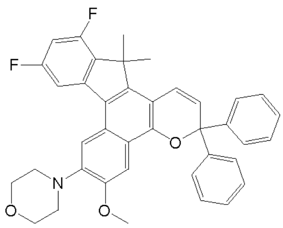
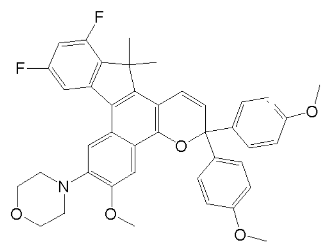
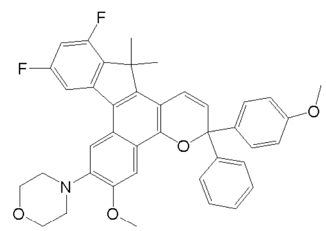

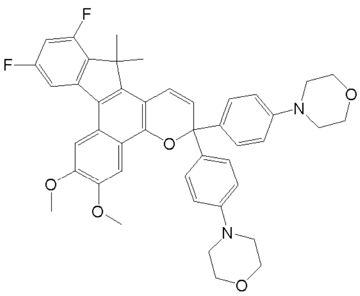
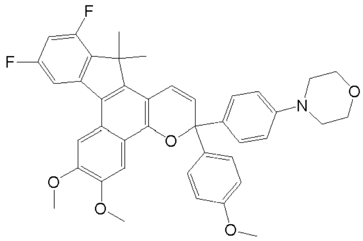
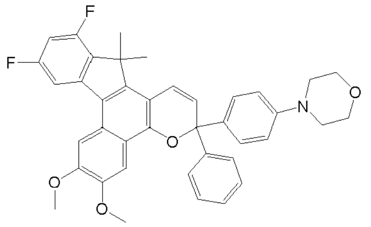

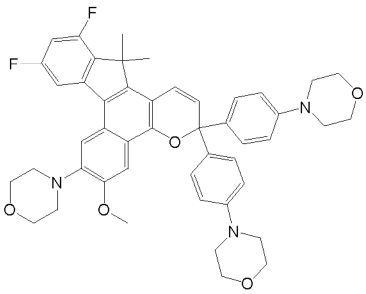
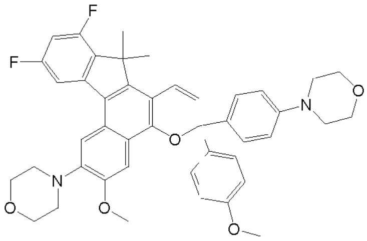
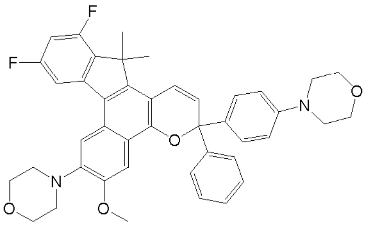

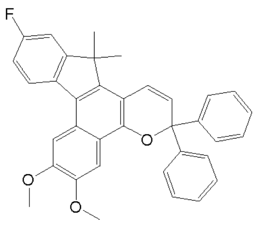
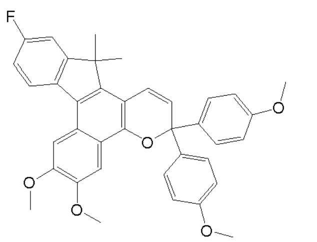
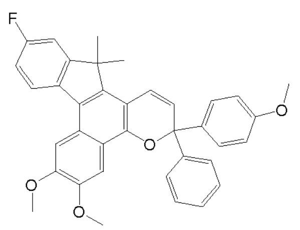

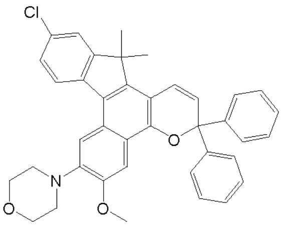
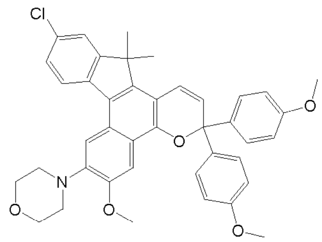
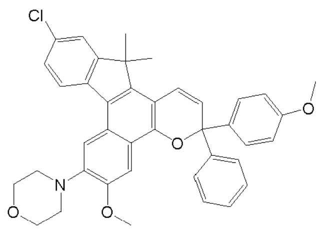

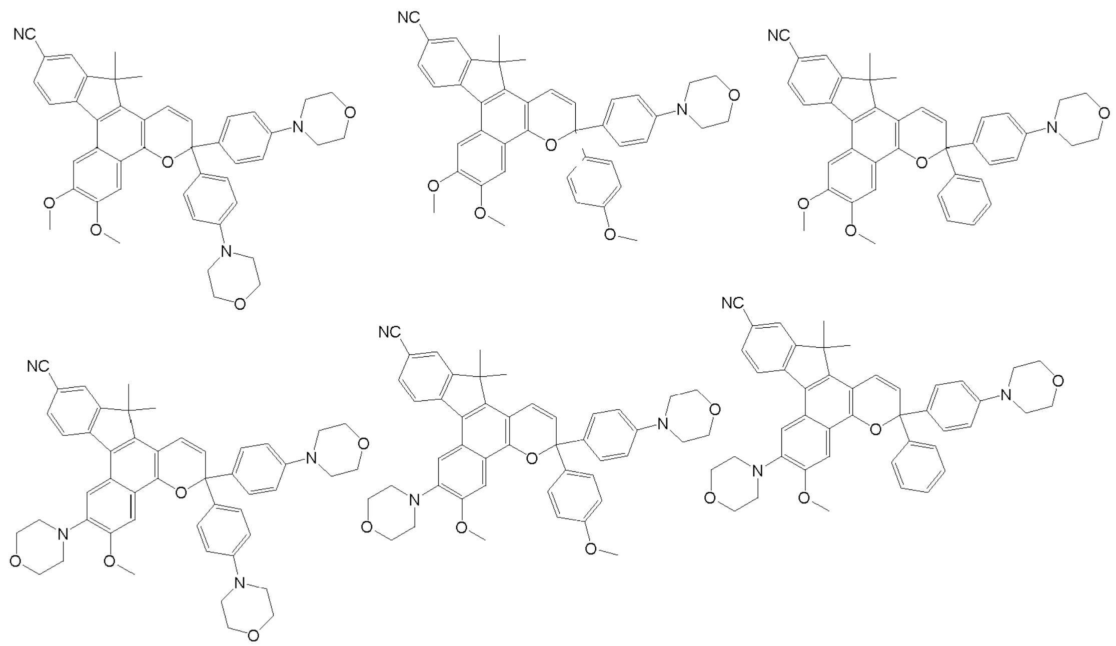

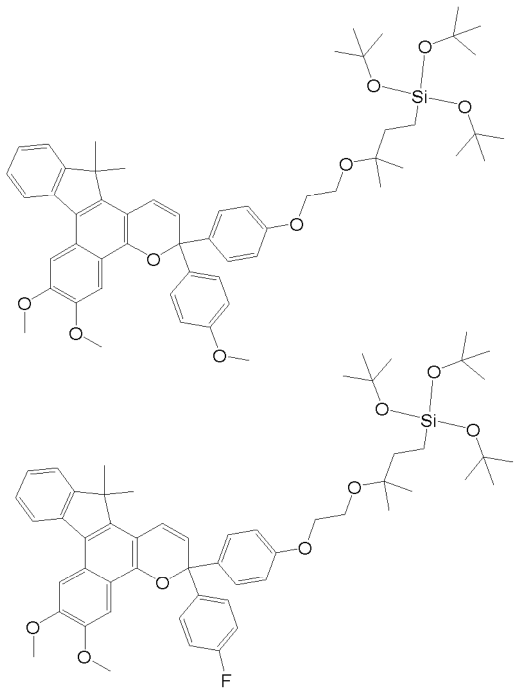

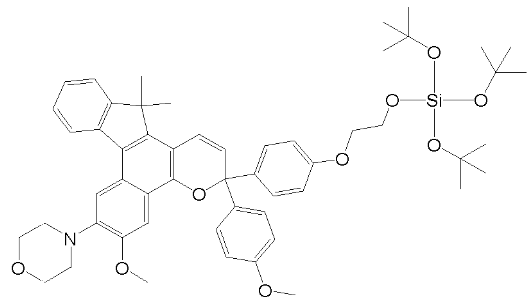

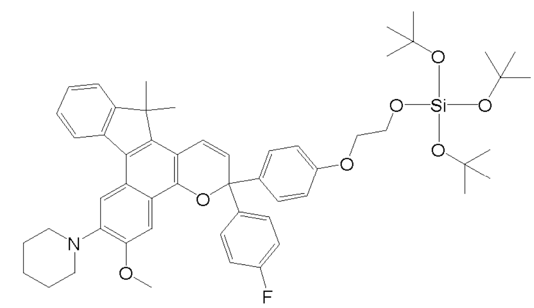

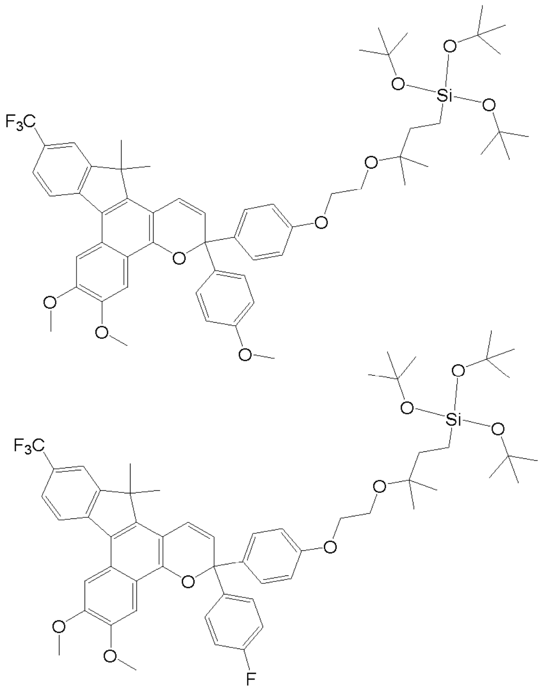

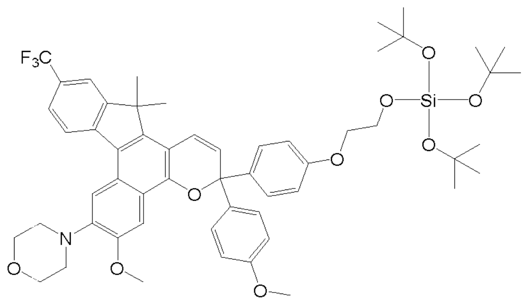

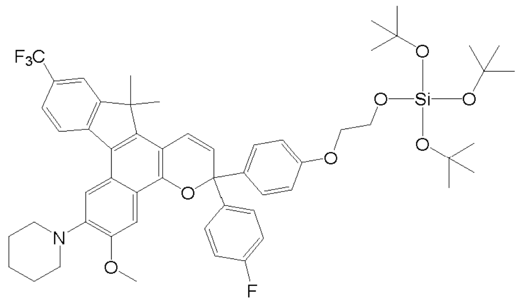

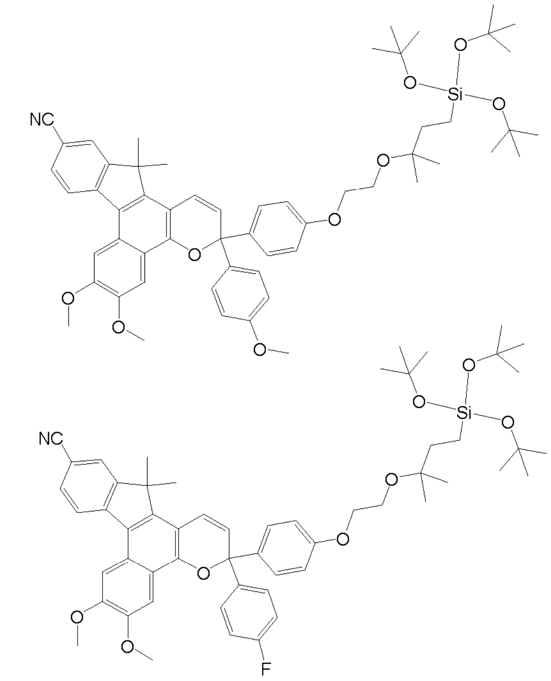

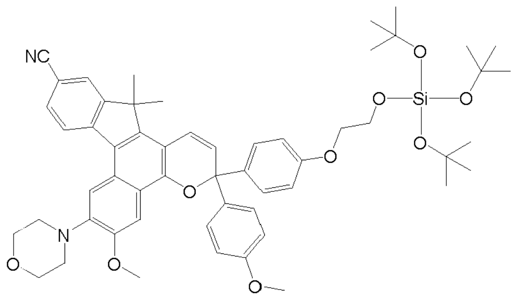

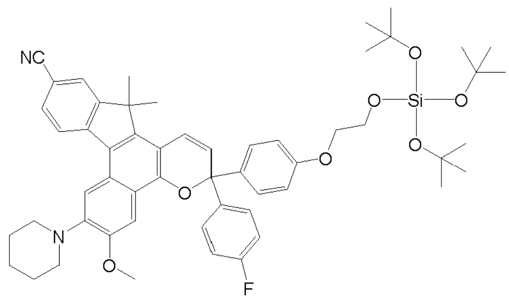

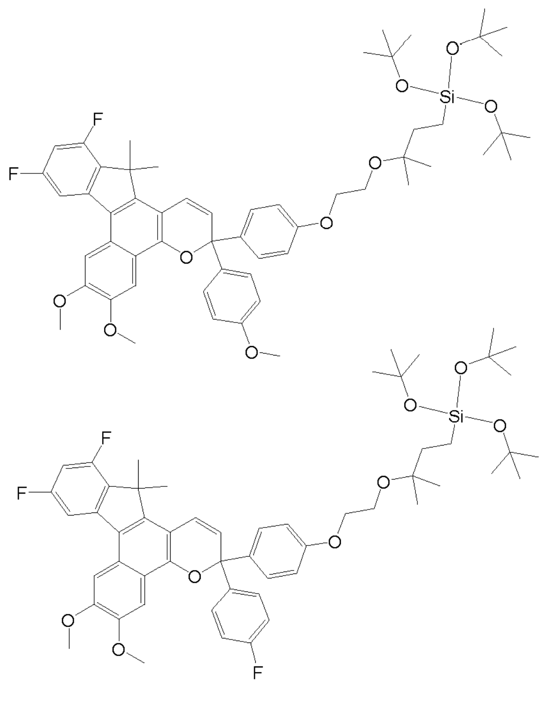

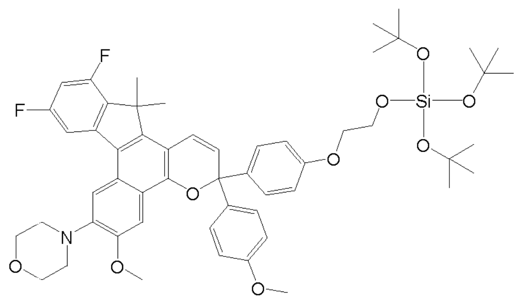

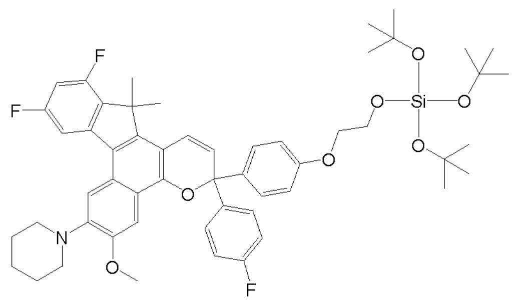

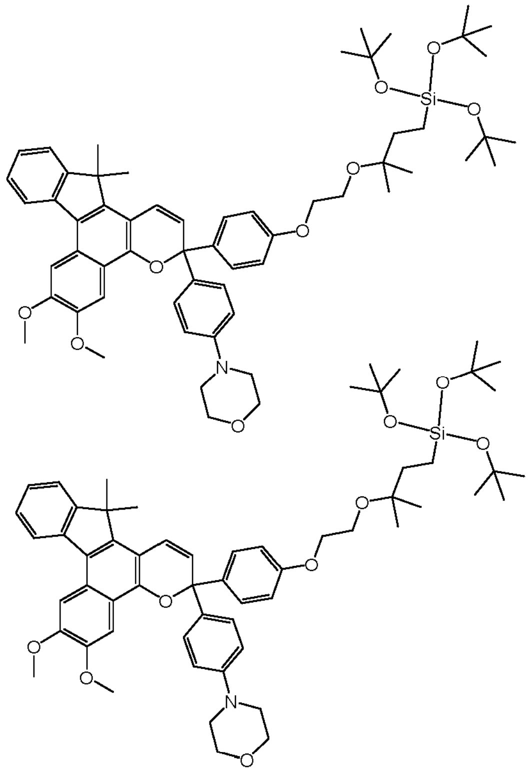

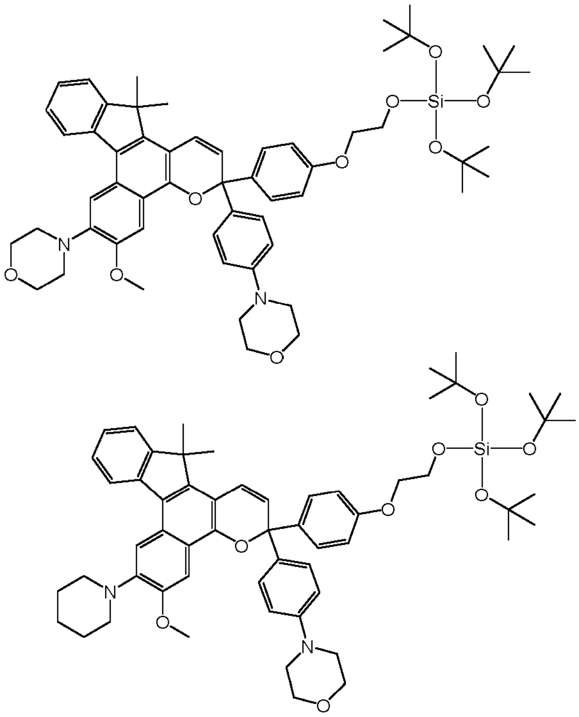

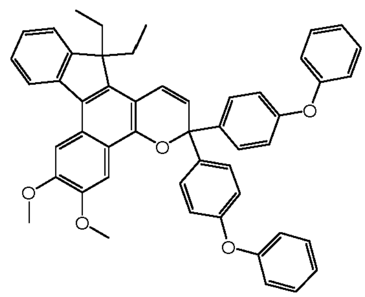

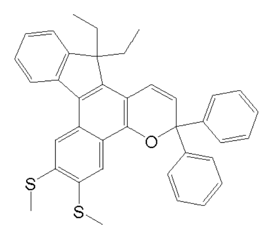

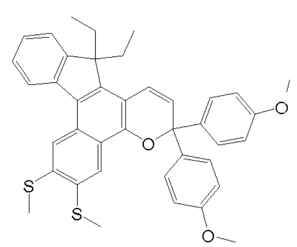

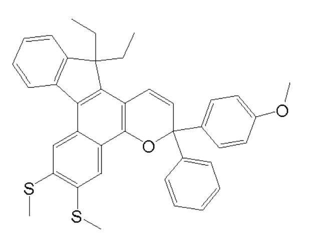

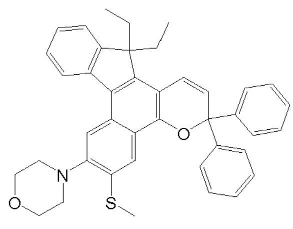

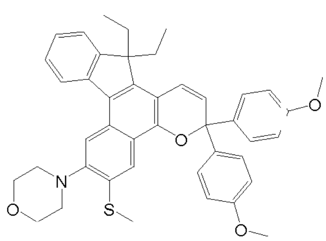

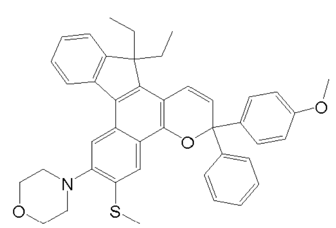

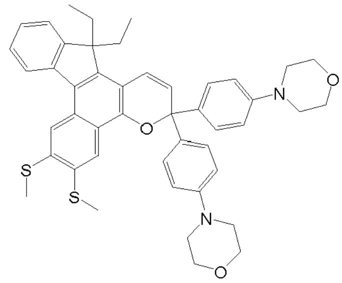
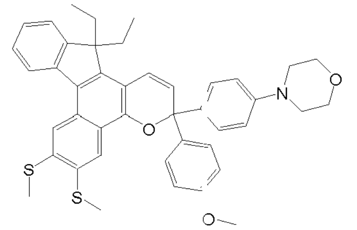
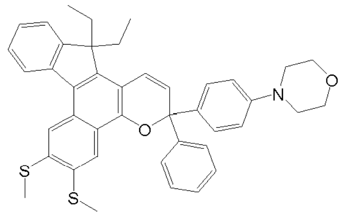
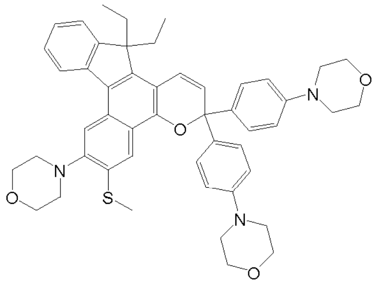
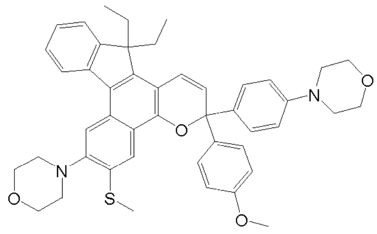
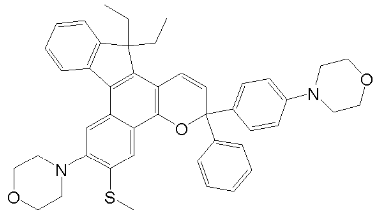
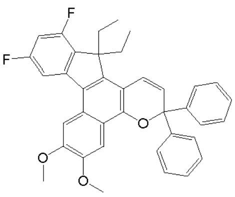
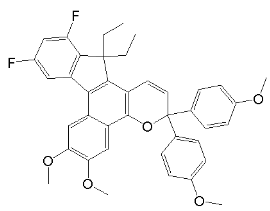
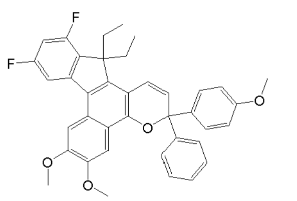
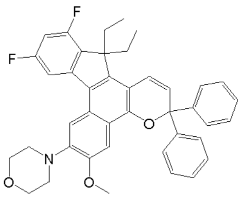
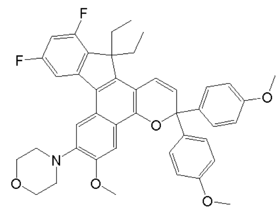
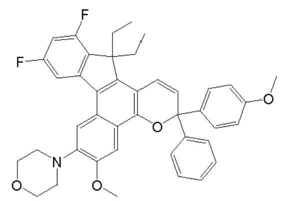
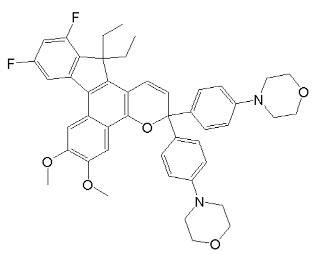
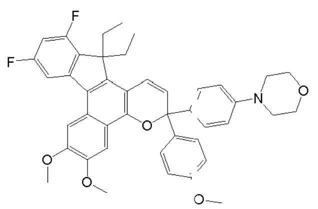
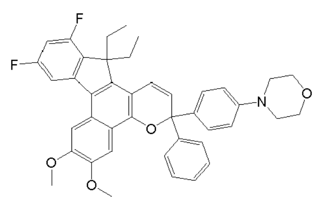
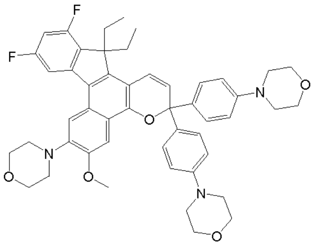
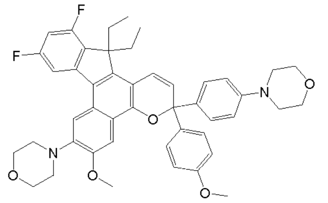
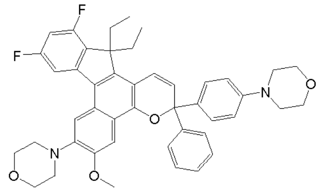

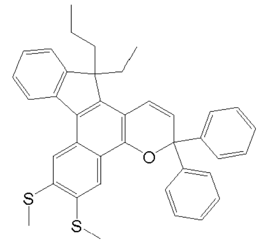
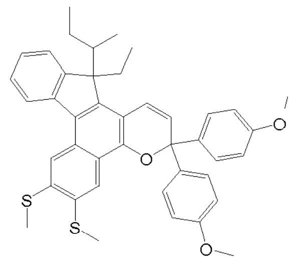
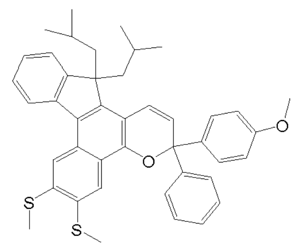

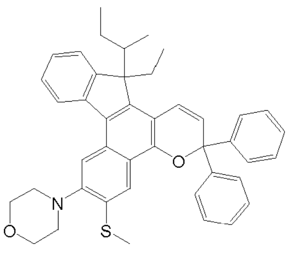
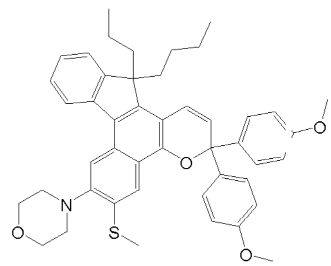
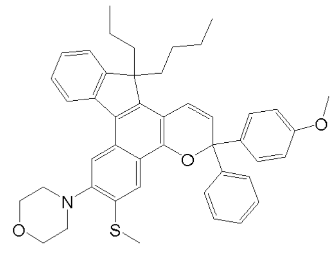

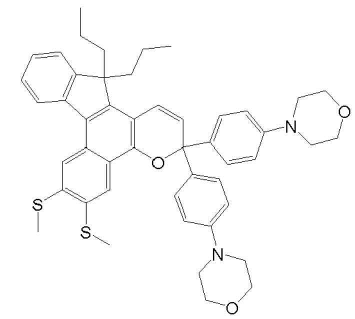
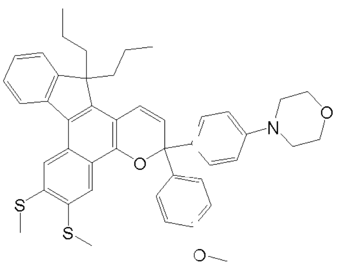
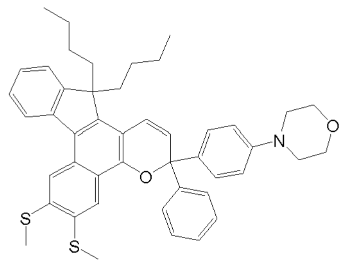

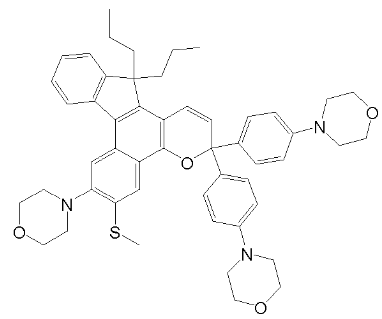
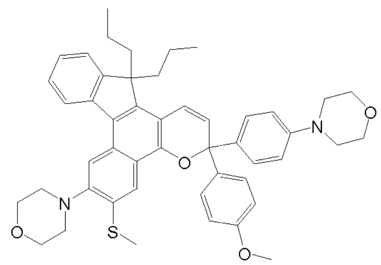
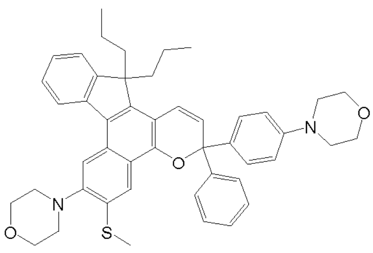

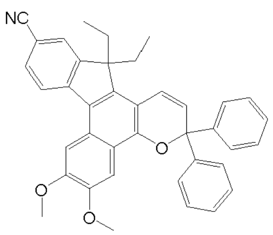
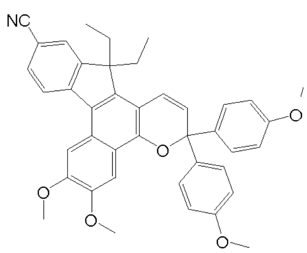
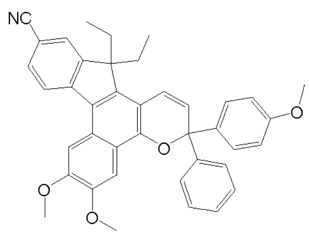

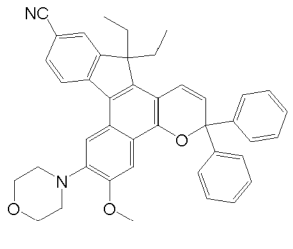

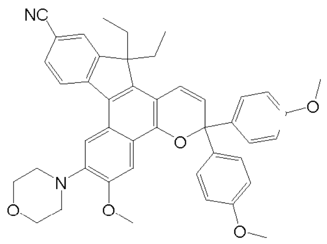

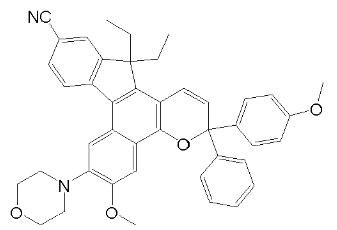

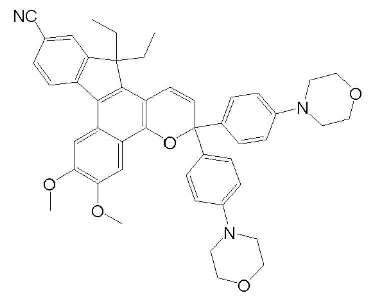

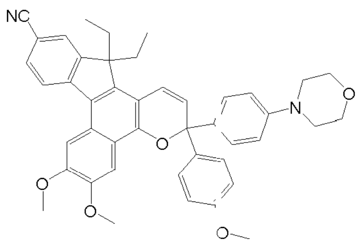

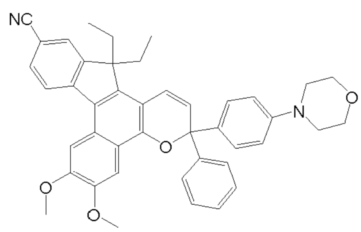

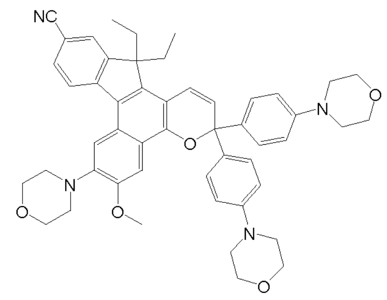

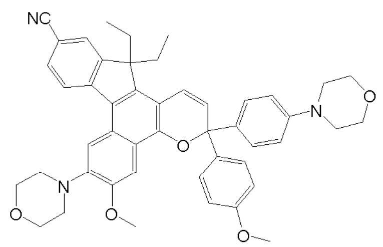

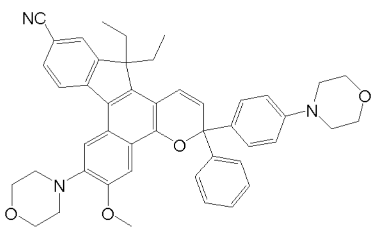

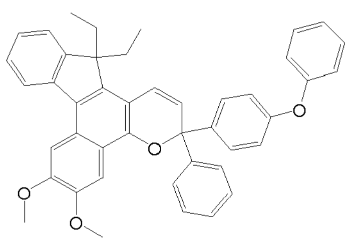

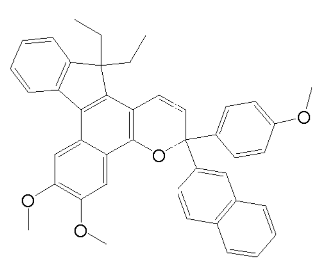

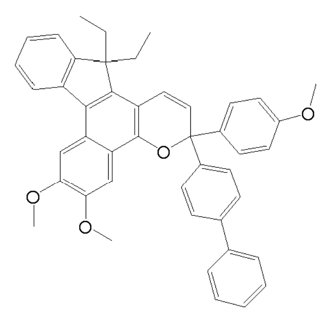

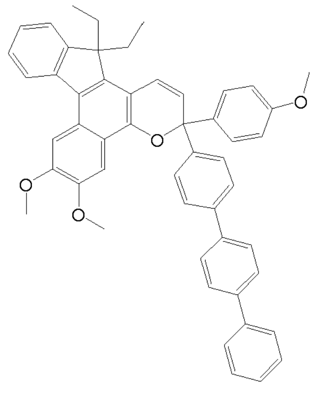

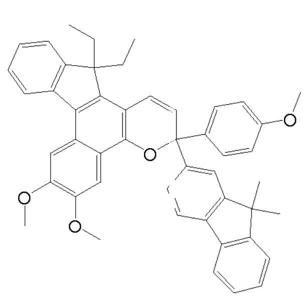

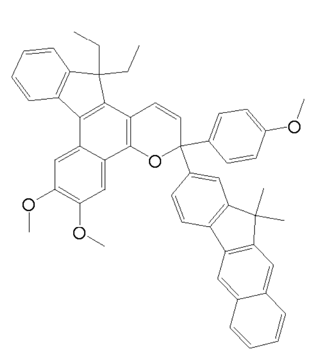

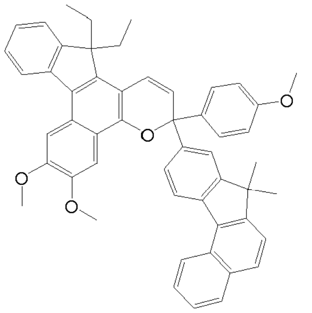
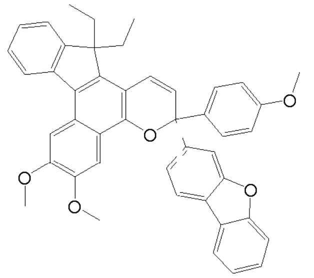
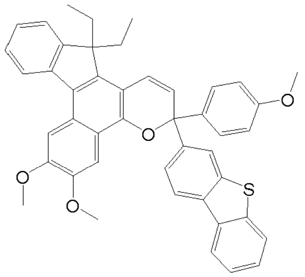

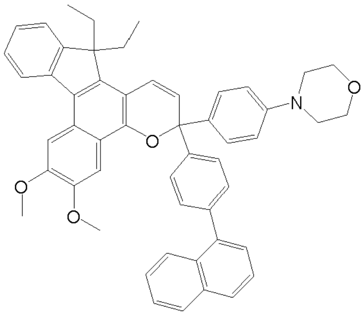
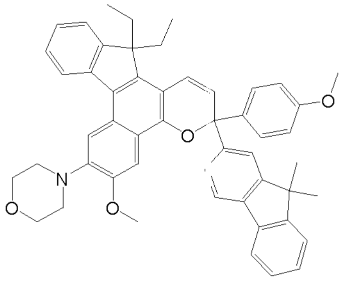
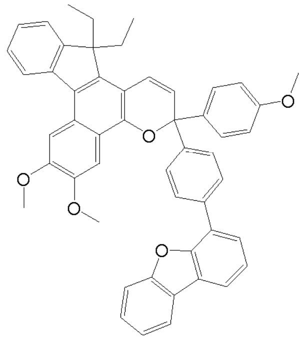

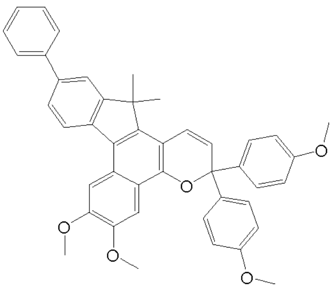
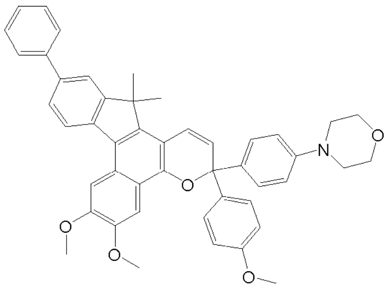
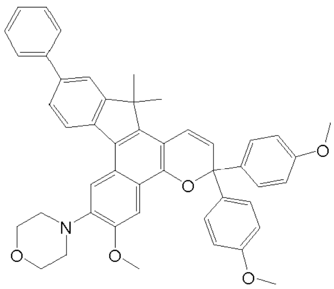

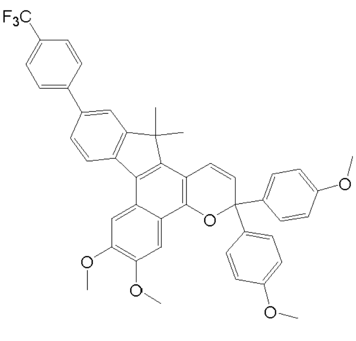
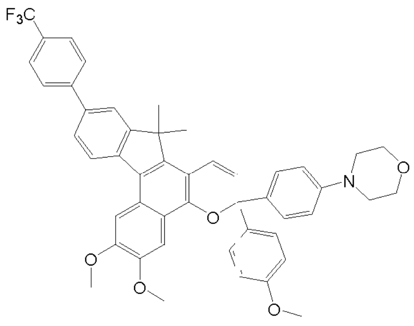
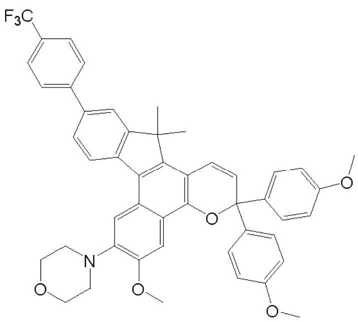

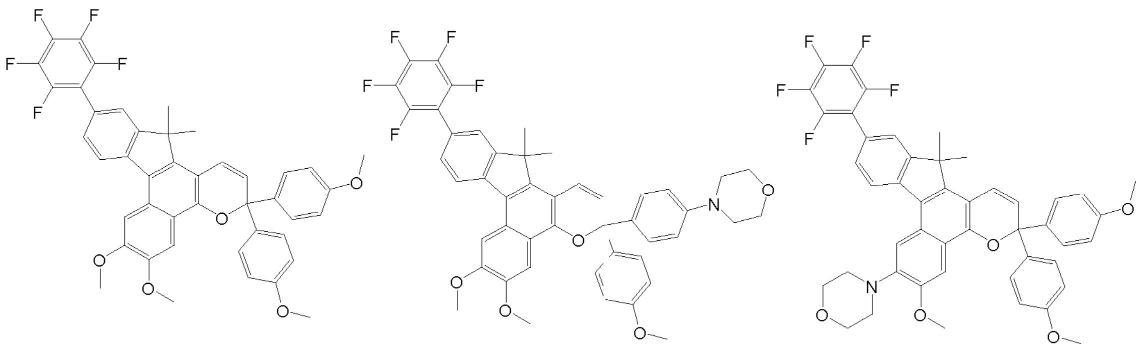

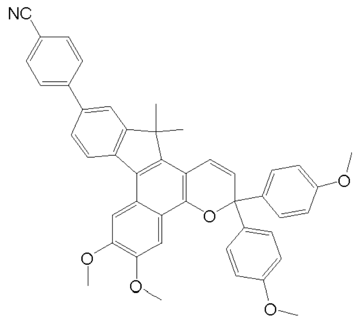

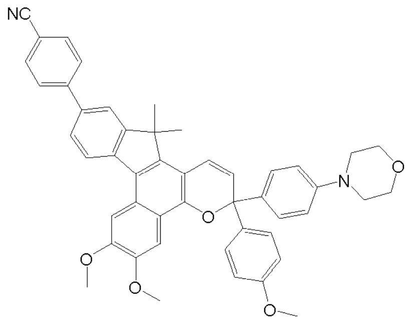

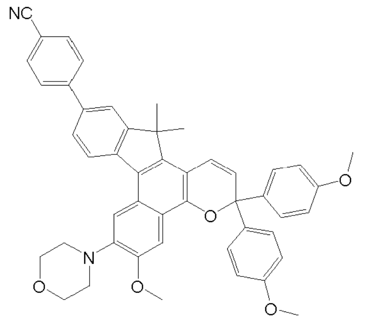

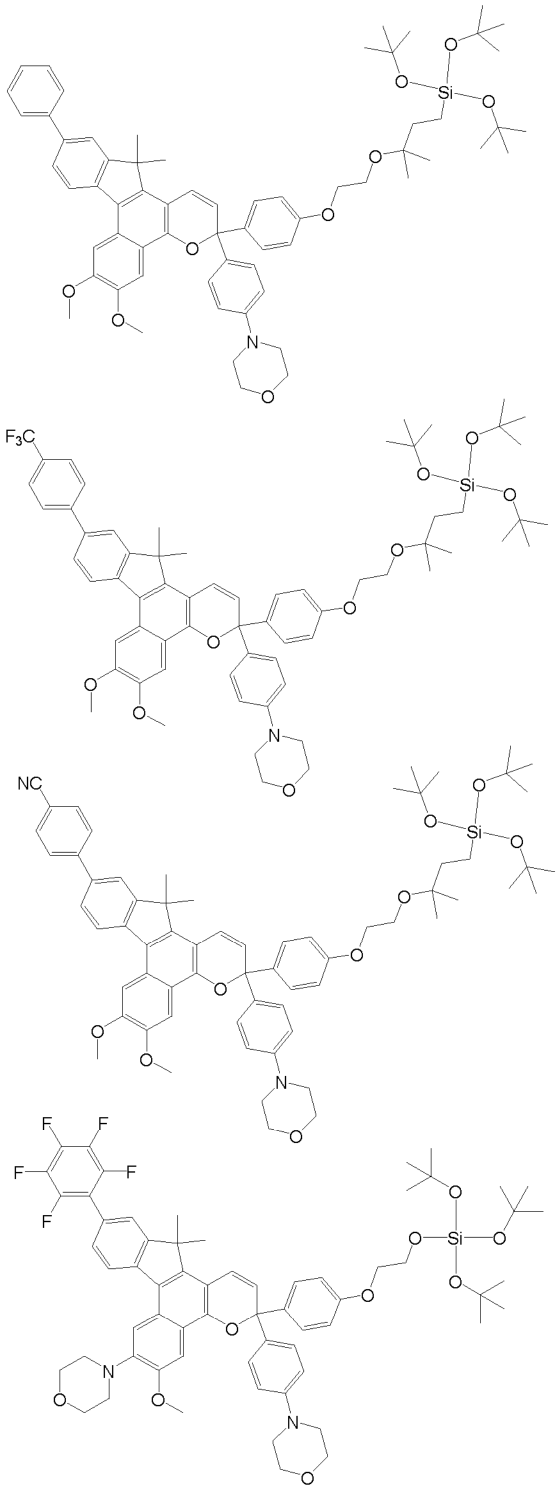

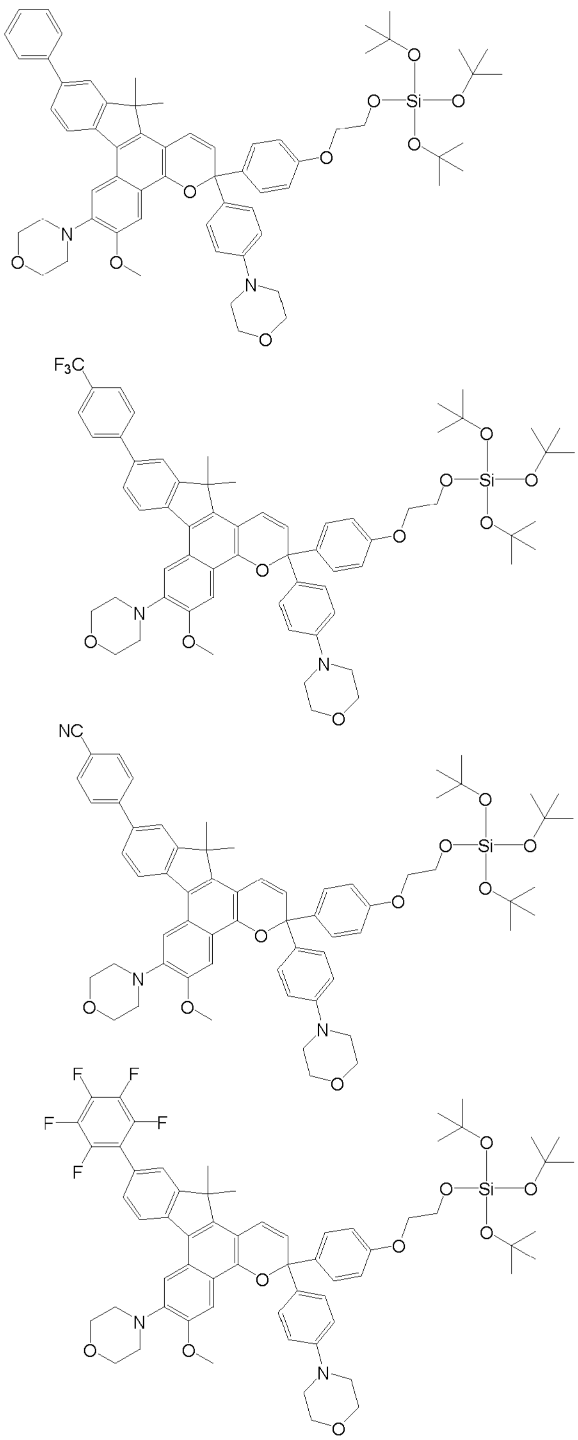

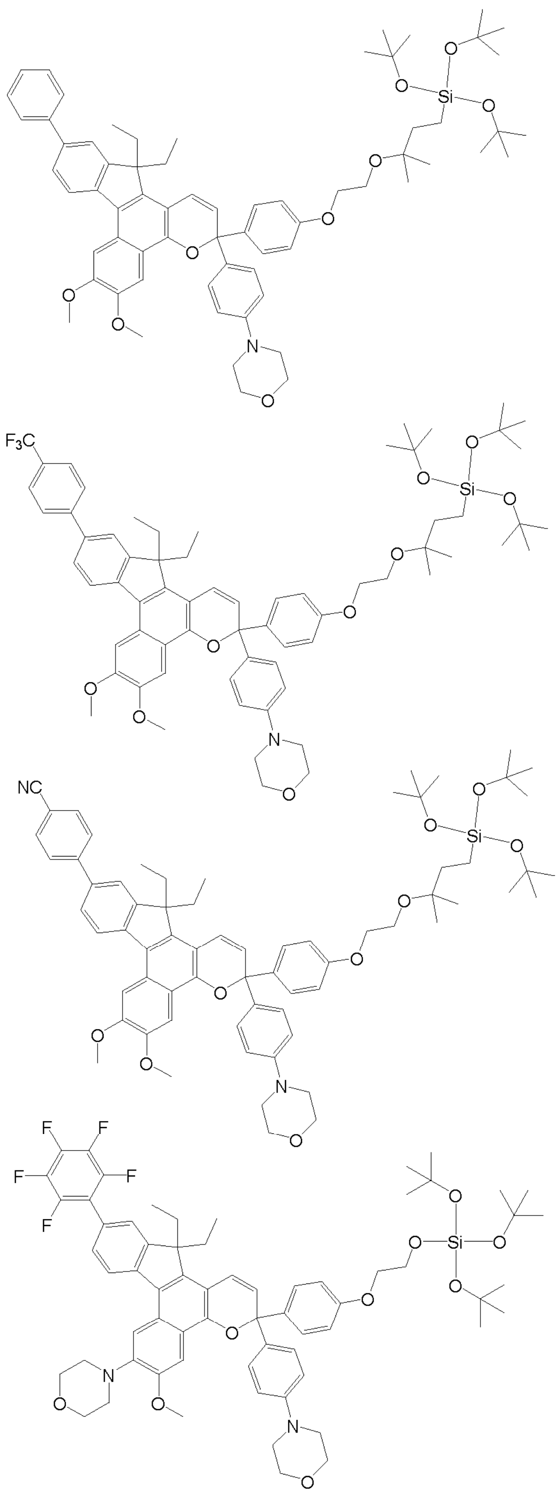

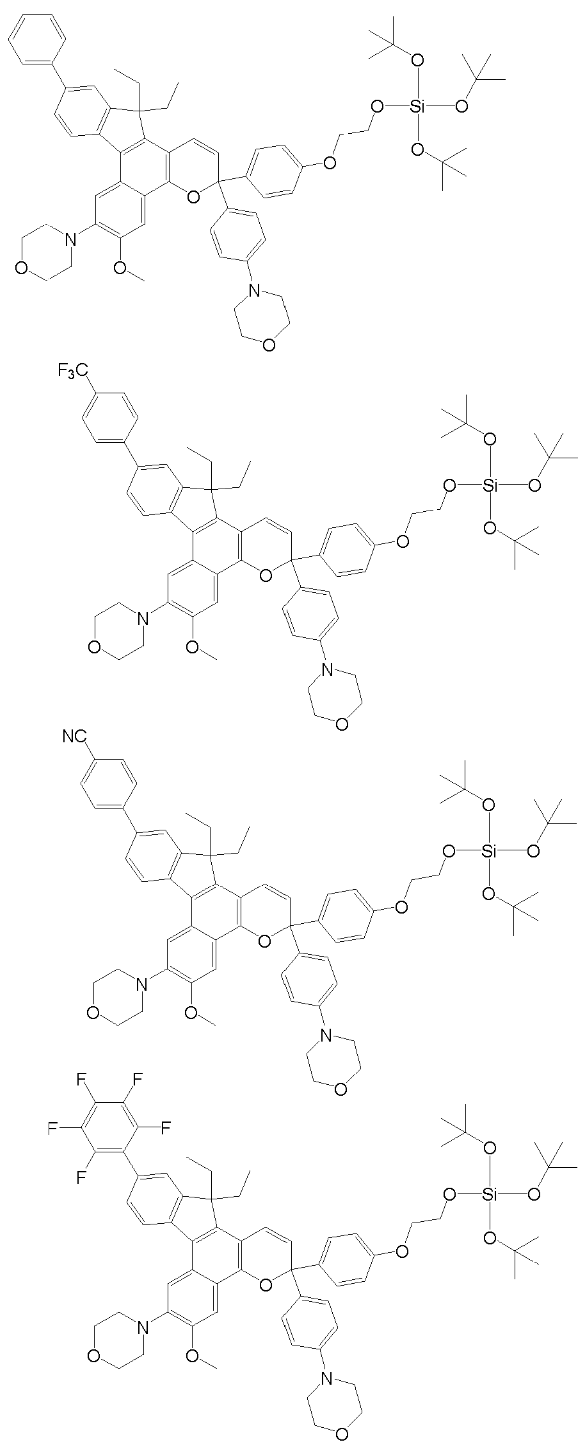

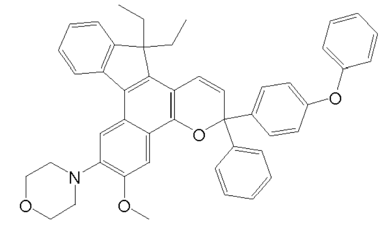

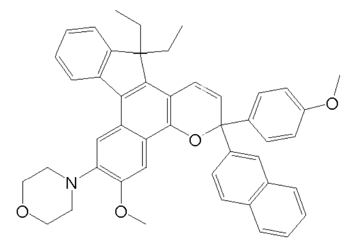

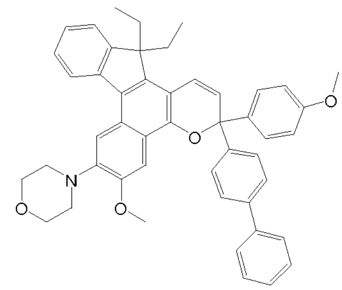

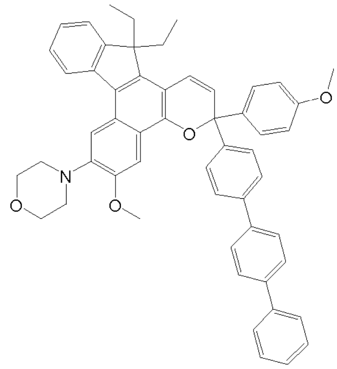

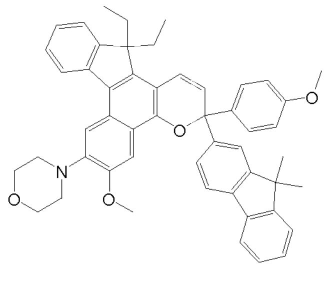

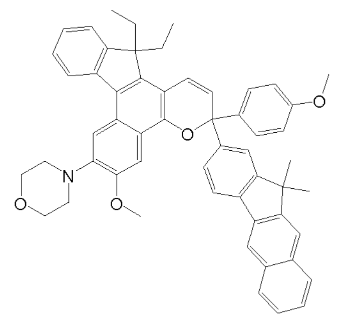

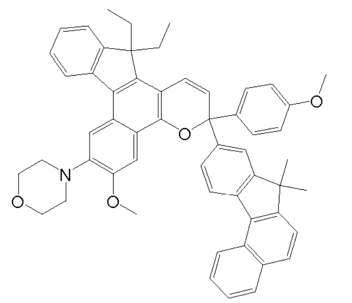

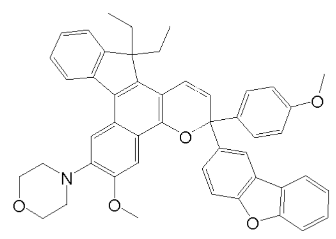

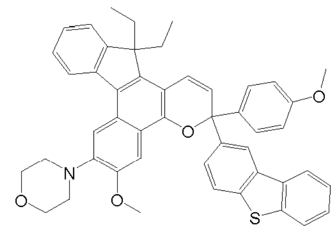

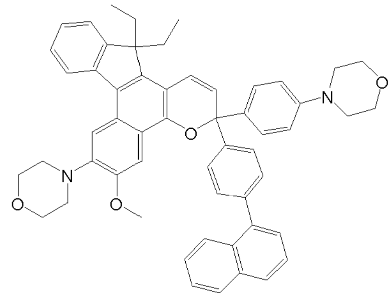

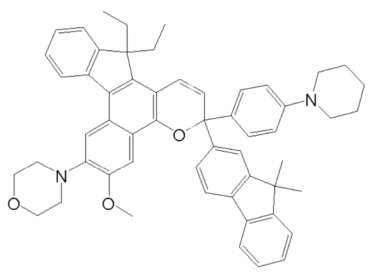

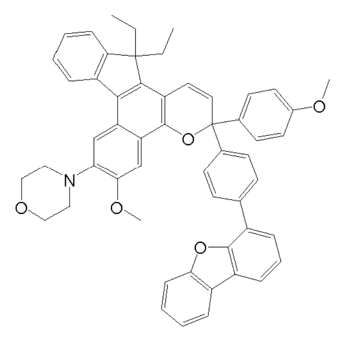

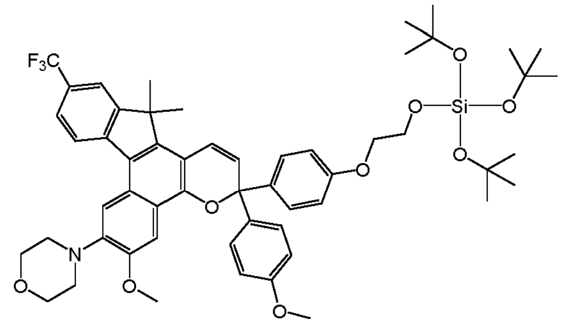

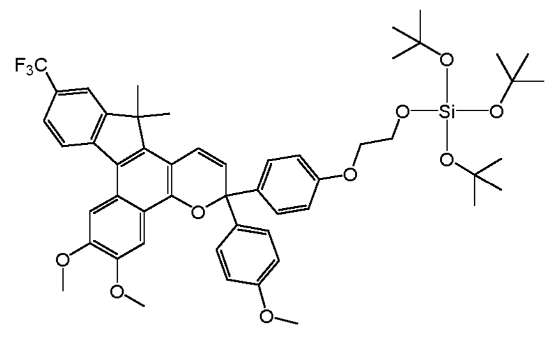

<Compound of Formula (C)>

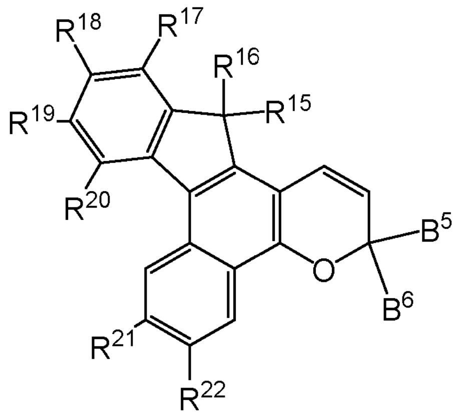

(C)

In Formula (C), $R^{15}$-$R^{20}$, $B^5$ and $B^6$ each independently are H or a substituent, one of $R^{21}$ and $R^{22}$ is H, and the other is an electron-donating group.

In Formula (C), $R^{15}$ and $R^{16}$ each independently preferably are optionally substituted $C_{1-20}$-alkyl, and more preferably methyl, ethyl, propyl, butyl, pentyl or hexyl. $R^{15}$ and $R^{16}$ each independently are still more preferably methyl or ethyl, and more preferably, $R^{15}$ and $R^{16}$ both are methyl or both are ethyl.

$B^5$ and $B^6$ each independently preferably are optionally substituted phenyl. When the phenyl group has a plurality of substituents, two or more of these substituents may be bonded to form a ring. Specific examples of the formed rings include rings included in exemplary compounds listed below. The substitution position of the substituent in the substituted phenyl group is preferably a position that is a para position with respect to a carbon atom to which $B^5$ and $B^6$ are bonded. Specific examples of substituents of substituted phenyl groups are morpholino, piperidino, halogen, group, and the following substituents.

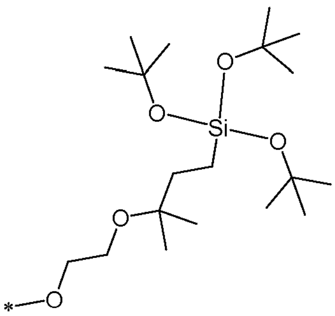
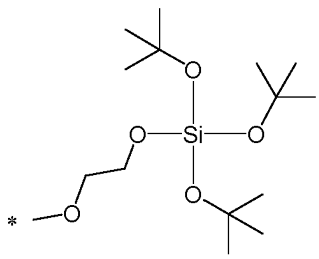

$R^{17}$-$R^{20}$ each independently are H or a substituent. In one aspect, $R^{17}$-$R^{20}$ all may be H. In another aspect, $R^{18}$ may be an electron-attracting group and $R^{17}$, $R^{19}$ and $R^{20}$ all may be H. In addition, in another aspect, $R^{17}$ and $R^{19}$ may each independently represent an electron-attracting group, and $R^{18}$ and $R^{20}$ may be H. The electron-attracting group is preferably halogen, $C_{1-6}$-perfluoroalkyl, , perfluoroalkylphenyl or cyano group. The halogen atom is preferably F. The $C_{1-6}$-perfluoroalkyl is preferably trifluoromethyl.

In one aspect, $R^{18}$ may be optionally substituted phenyl, and preferably, $R^{18}$ is optionally substituted phenyl, and $R^{17}$, $R^{19}$ and $R^{20}$ may be H. Specific examples of such substituted phenyl groups include phenyl groups substituted with one or more halogen atoms and/or one or more cyano groups are, for example, phenyl groups substituted with halogen atoms (preferably, fluorine atoms) at all five substitution positions of the phenyl group, and monosubstituted phenyl groups substituted with a cyano group at a position that is a para position with respect to a carbon atom to which $R^{10}$ is bonded.

One of $R^{21}$ and $R^{22}$ is H, and the other is an electron-donating group. Preferably, $R^{21}$ is is H, and $R^{22}$ is an electron-donating group. More preferably, the electron-donating group represented by $R^{21}$ or $R^{22}$ (preferably, $R^{22}$) is an electron-donating group selected from methoxy, ethoxy, phenoxy, methylsulfide, phenylsulfide, dimethylamino, pyrrolidino, piperidino, morpholino and thiomorpholino.

Examples of compounds of Formula (C) include the following compounds.

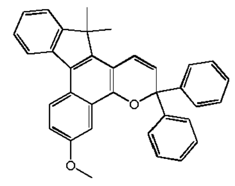
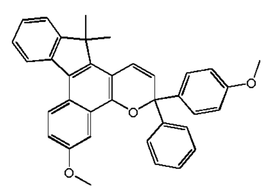
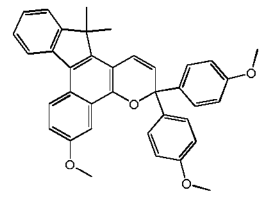

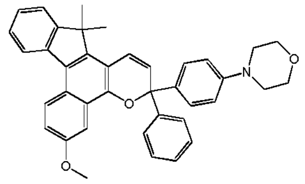
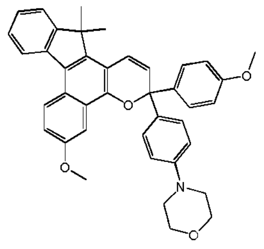
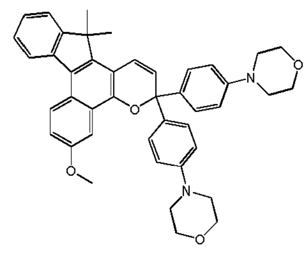

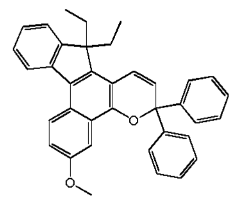
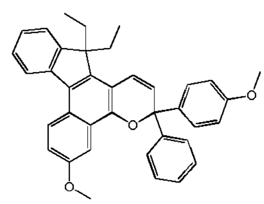
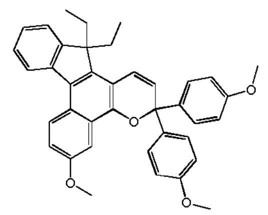

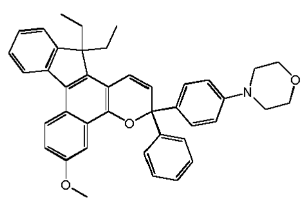
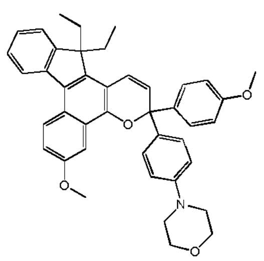
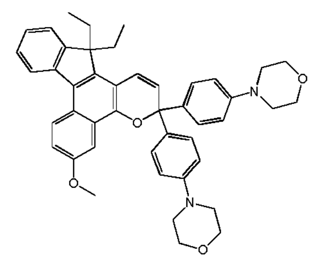

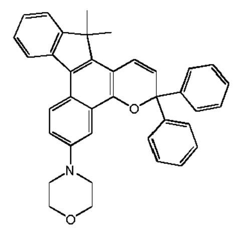
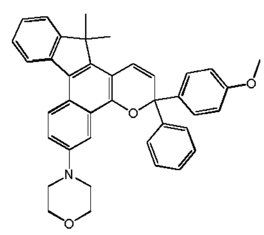
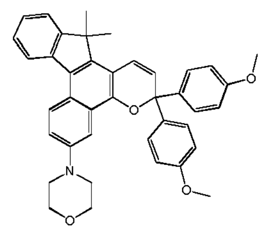

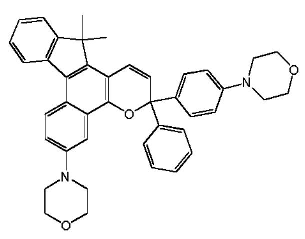
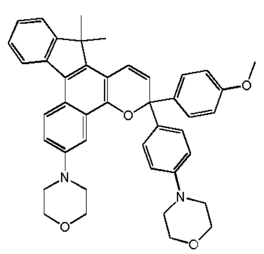
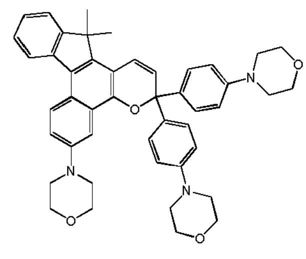

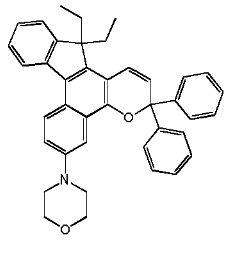
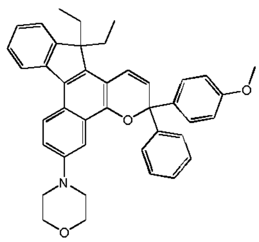
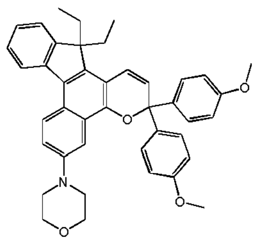

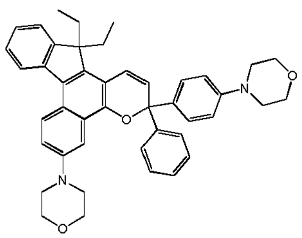
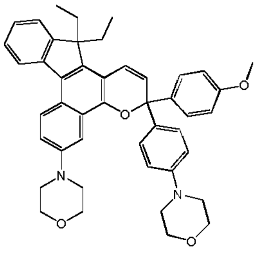
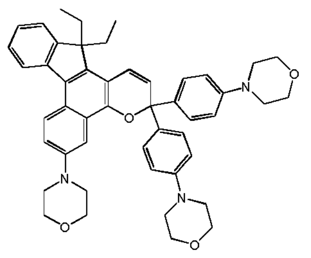

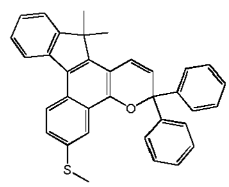
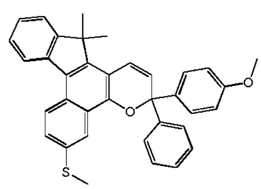
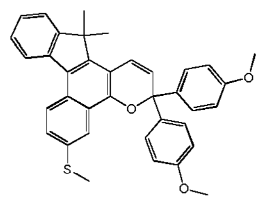

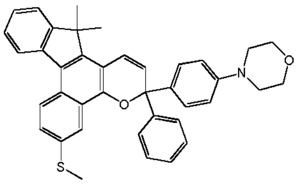
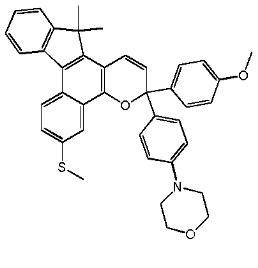
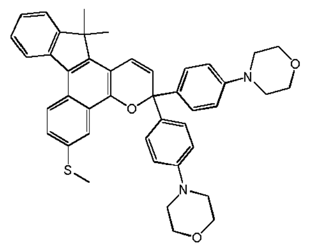

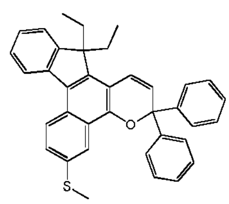
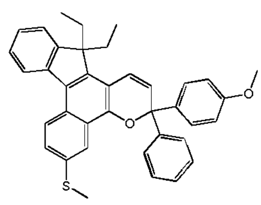
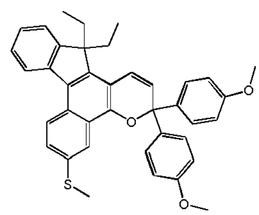

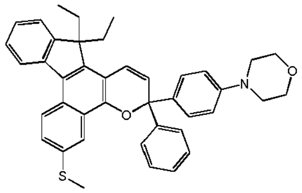
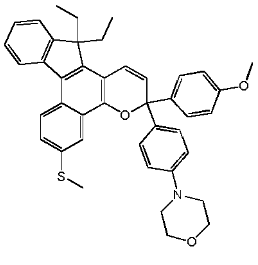
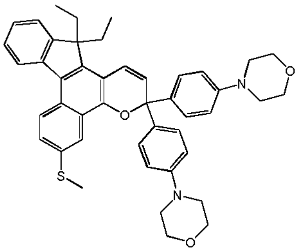

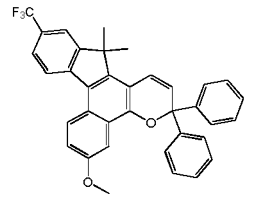
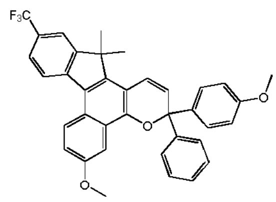
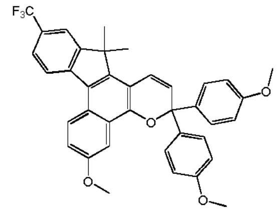

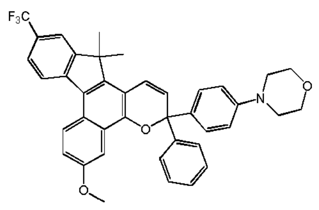
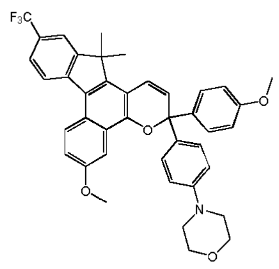
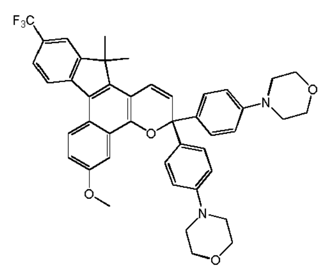

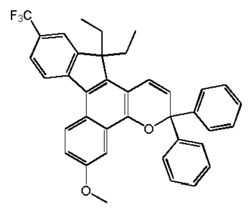
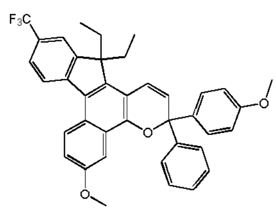
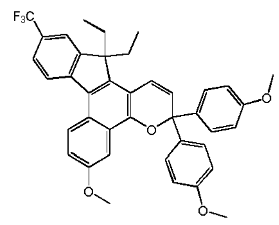

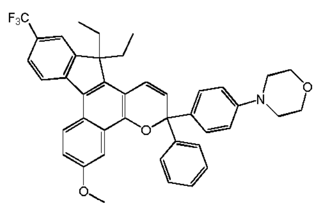
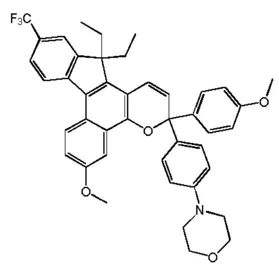
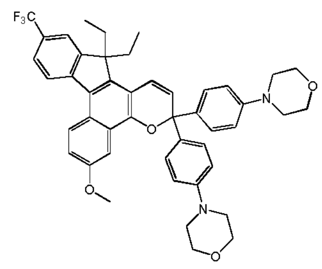

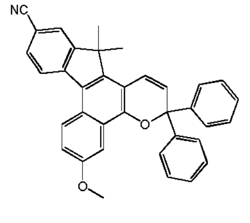
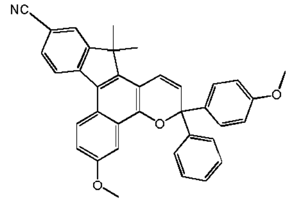
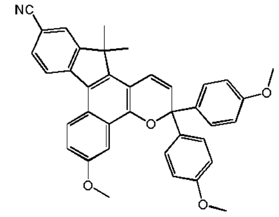

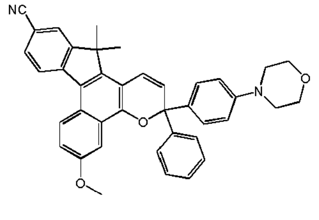
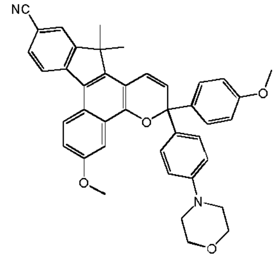
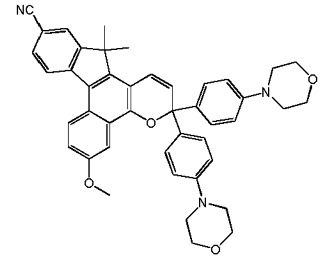

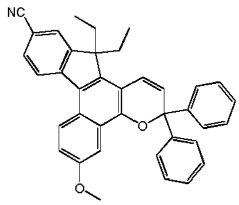

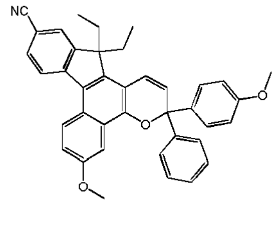

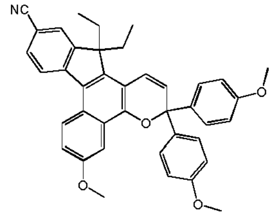

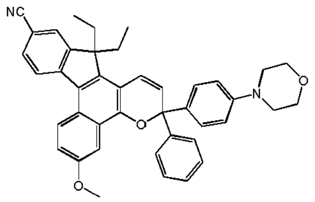

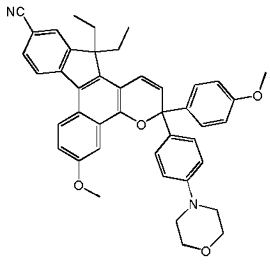

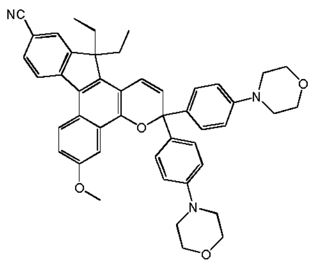

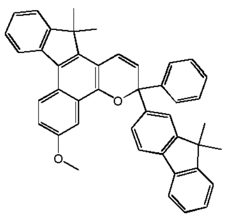

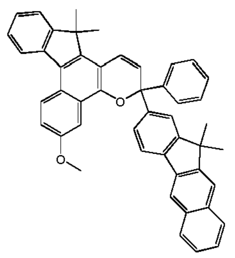

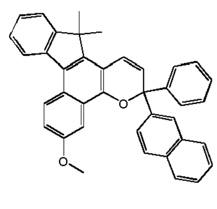

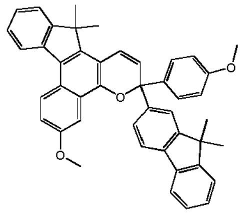

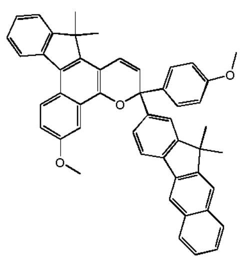

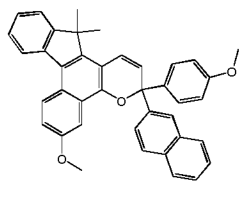

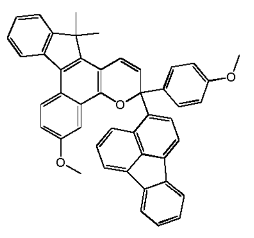

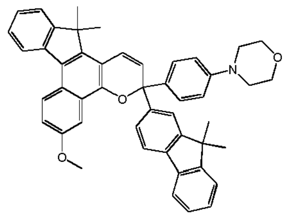

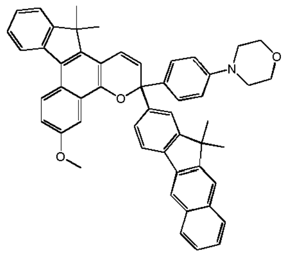

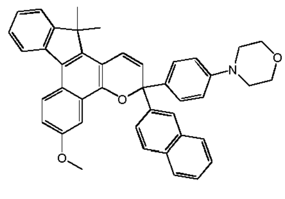

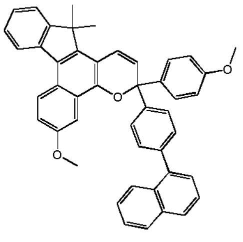

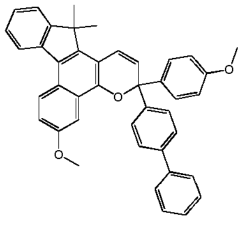

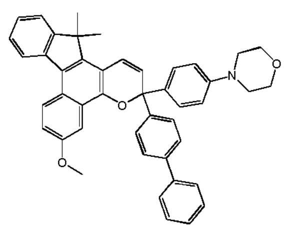

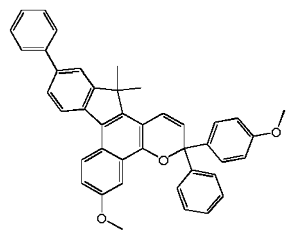
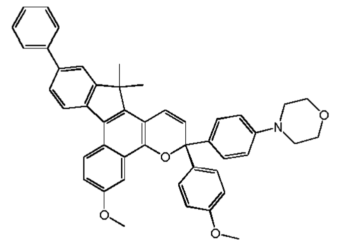
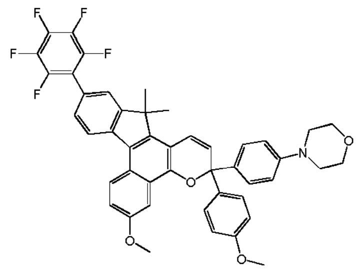
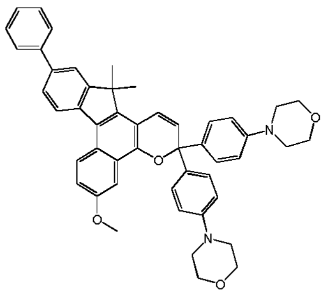
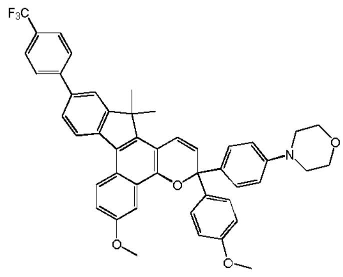
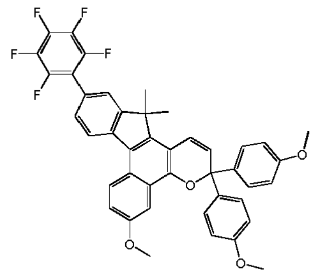
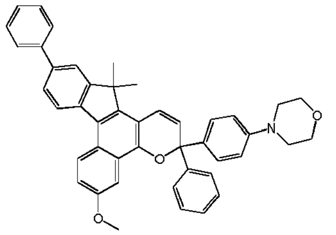
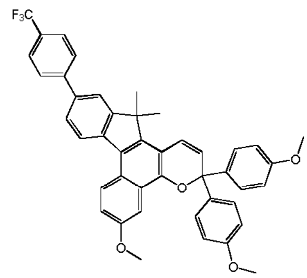
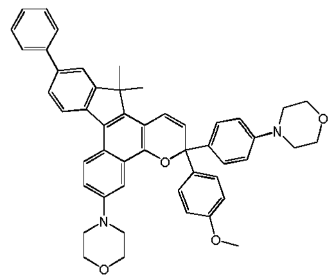
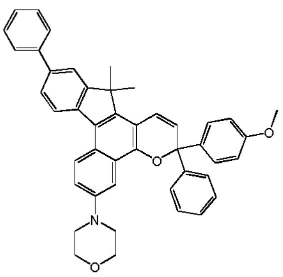
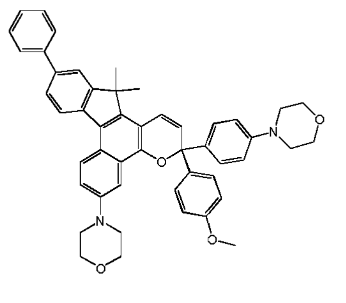
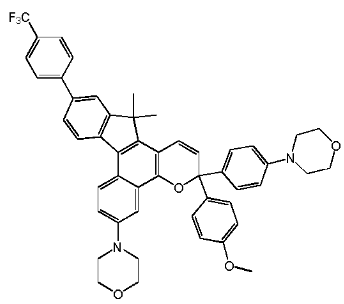
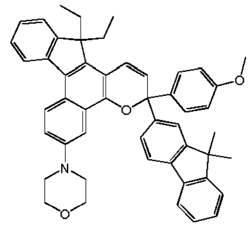
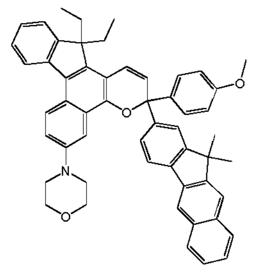
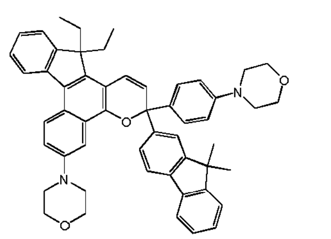

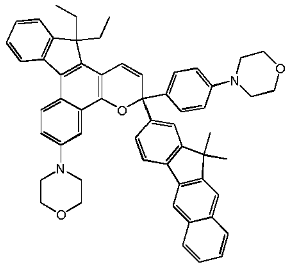
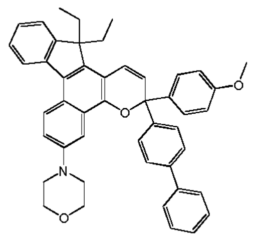
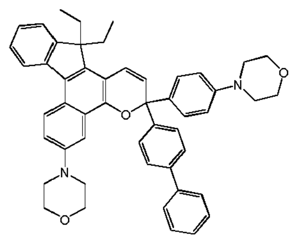

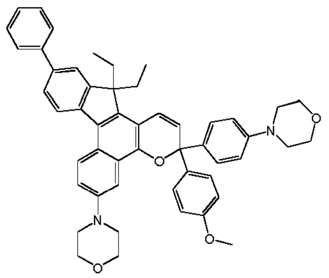
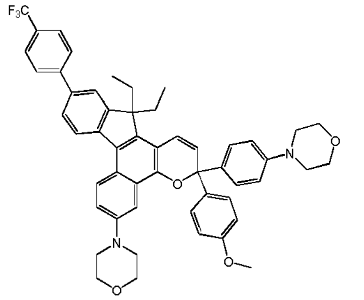
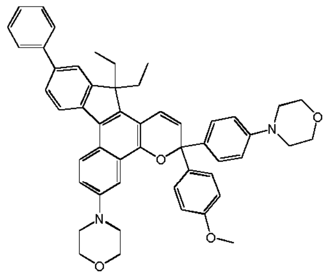

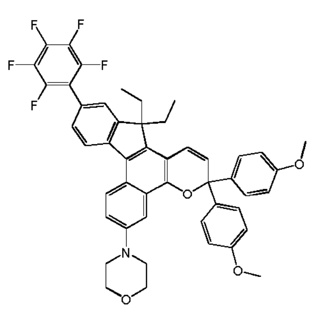
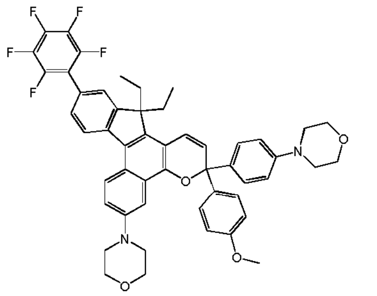
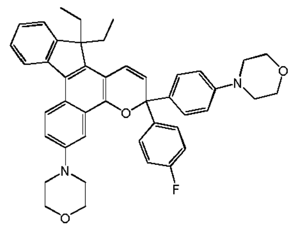

The photochromic article can have at least a substrate. In one aspect, the photochromic compound can be included in the substrate of the photochromic article. The photochromic article can have a substrate and a photochromic layer, and the substrate and/or photochromic layer can contain one or more compounds of Formula (1) or (1a) and additionally contain one or more selected from compounds of the Formulae (A), (B) (C). In the substrate and the photochromic layer, in one aspect, the photochromic compound can be contained only in the substrate, in another aspect, the photochromic compound can be contained only in the photochromic layer, and in still another aspect, the photochromic compound can be contained in the substrate and the photochromic layer. In addition, the substrate and the photochromic layer can contain, as a photochromic compound, only the above compound or one or more other photochromic compounds. Examples of other photochromic compounds include azobenzenes, spiropyrans, spirooxazines, naphthopyrans, indenonaphthopyrans, phenanthropyrans, hexaallylbisimidazoles, donor-acceptor Stenhouse adducts (DASA), salicylidene anilines, dihydropyrenes, anthracene dimers, fulgides, diarylethenes, phenoxynaphthacenequinones, and stilbenes.

<Substrate>

The photochromic article can contain a substrate selected according to the type of the photochromic article. Examples of substrates include spectacle lens substrates such as a plastic lens substrate and a glass lens substrate. The glass lens substrate can be, for example, a lens substrate made of inorganic glass. Examples of plastic lens substrates include styrene resins such as (meth)acrylic resins, allyl carbonate resins such as a polycarbonate resin, allyl resin, and diethylene glycol bisallyl carbonate resin (CR-39), vinyl resins, polyester resins, polyether resins, urethane resins obtained by reacting an isocyanate compound and a hydroxy compound such as diethylene glycol, thiourethane resins obtained by reacting an isocyanate compound and a polythiol compound, and a cured product obtained by curing a curable composition containing a (thio)epoxy compound having one or more disulfide bonds in the molecule (generally referred to as a transparent resin). As the lens substrate, an undyed substrate (colorless lens) may be used or a dyed substrate (dyed lens) may be used. The refractive index of the lens substrate may be, for example, about 1.50-1.75. However, the refractive index of the lens substrate is not limited to the above range, and may be within the above range or may be above or below

outside the above range. Here, the refractive index refers to a refractive index for light having a wavelength of 500 nm. In addition, the lens substrate may be a lens having refractive power (so-called prescription lens) or a lens having no refractive power (so-called non-prescription lens).

For example, the photochromic composition can be a polymerizable composition. In the present invention and this specification, the "polymerizable composition" is a composition containing one or more polymerizable compounds. A polymerizable composition containing at least one or more photochromic compounds and one or more polymerizable compounds can be molded by a known molding method to produce a cured product of such a polymerizable composition. Such a cured product can be included as a substrate in the photochromic article and/or can be included as a photochromic layer. The curing treatment can be light emission and/or a heat treatment. The polymerizable compound is a compound having a polymerizable group, and as the polymerization reaction of the polymerizable compound proceeds, the polymerizable composition can be cured to form a cured product. The polymerizable composition can further contain one or more additives (for example, a polymerization initiator).

The spectacle lens may include various lenses such as a single focus lens, a multifocal lens, and a progressive power lens. The type of the lens is determined by the surface shape of both sides of the lens substrate. In addition, the surface of the lens substrate may be a convex surface, a concave surface, or a flat surface. In a general lens substrate and spectacle lens, the object-side surface is a convex surface and the eyeball-side surface is a concave surface. However, the present invention is not limited thereto. The photochromic layer may be generally provided on the object-side surface of the lens substrate, or may be provided on the eyeball-side surface.

<Photochromic Layer>

The photochromic layer can be a layer that is directly provided on the surface of the substrate or indirectly provided via one or more other layers. The photochromic layer can be, for example, a cured layer obtained by curing a polymerizable composition. A photochromic layer can be formed as a cured layer obtained by curing a polymerizable composition containing at least one or more photochromic compounds and one or more polymerizable compounds. For example, when such a polymerizable composition is directly applied to the surface of the substrate or applied to the surface of the layer provided on the substrate, and a curing treatment is performed on the applied polymerizable composition, a photochromic layer can be formed as a cured layer containing one or more photochromic compounds. As the coating method, known coating methods such as a spin coating method, a dip coating method, a spray coating method, an inkjet method, a nozzle coating method, and a slit coating method can be used. The curing treatment can be light emission and/or a heat treatment. The polymerizable composition can further contain one or more additives (for example, a polymerization initiator) in addition to one or more polymerizable compounds. As the polymerization reaction of the polymerizable compound proceeds, the polymerizable composition can be cured to form a cured layer.

The thickness of the photochromic layer may be, for example, 5 μm or more, 10 μm or more or 20 μm or more, and may be, for example, 80 μm or less, 70 μm or less or 50 μm or less.

<Polymerizable Compound>

In the present invention and this specification, the term polymerizable compound refers to a compound having one or more polymerizable groups in one molecule, and the term "polymerizable group" refers to a reactive group that can undergo a polymerization reaction. Examples of polymerizable groups include acryloyl, methacryloyl, vinyl, vinyl ether, epoxy, thiol, oxetane, hydroxy, carboxy, amino and isocyanate.

Examples of polymerizable compounds that can be used to form the above substrate and the above photochromic layer include the following compounds.

(Episulfide Compound)

The episulfide compound is a compound having two or more episulfide groups in one molecule. The episulfide group is a polymerizable group that can undergo ring-opening polymerization. Specific examples of episulfide compounds include bis(1,2-epithioethyl)sulfide, bis(1,2-epithioethyl)disulfide, bis(2,3-epithiopropyl)sulfide, bis(2,3-epithiopropylthio)methane, bis(2,3-epithiopropyl)disulfide, bis(2,3-epithiopropyldithio)methane, bis(2,3-epithiopropyldithio)ethane, bis(6,7-epithio-3,4-dithiaheptyl)sulfide, bis(6,7-epithio-3,4-dithiaheptyl)disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, and 2-[1,1-bis(2,3-epithiopropyldithiomethylthio)methyl]-1,3-dithietane.

(Thietanyl Compound)

The thietanyl compound is a thietane compound having two or more thietanyl groups in one molecule. The thietanyl group is a polymerizable group that can undergo ring-opening polymerization. Some thietanyl compounds have an episulfide group together with a plurality of thietanyl groups. Such compounds are listed as examples in the above episulfide compound. Other thietanyl compounds include metal-containing thietane compounds having metal atoms in the molecule and non-metallic thietane compounds which contain no metal.

Specific examples of non-metallic thietane compounds include bis(3-thietanyl)disulfide, bis(3-thietanyl)sulfide, bis(3-thietanyl)trisulfide, bis(3-thietanyl)tetrasulfide, 1,4-bis(3-thietanyl)-1,3,4-trithibutane, 1,5-bis(3-thietanyl)-1,2,4,5-tetrathiapentane, 1,6-bis(3- thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio) methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiundecane, 1,11-bis(3-thietanylthio)-4,7-bis(3-thietanylthiomethyl)-3,6,9-trithiundecane, 1,11-bis(3-thietanylthio)-5,7-bis(3-thietanylthiomethyl)-3,6,9-trithiundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bis-thietanylsulfide, bis(thietanylthio) methane, 3-[<(thietanylthio)methylthio>methylthio]thietane, bis-thietanyl disulfide, bis-thietanyl trisulfide, bis-thietanyl tetrasulfide, bis-thietanyl pentasulfide, 1,4-bis(3-thietanyldithio)-2,3-dithibutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio) ethane.

Examples of metal-containing thietane compounds include those containing Group 14 atoms such as Sn, Si, Ge and Pb, Group 4 elements such as Zr and Ti, Group 13 atoms such as Al, and Group 12 atoms such as Zn, as metal atoms in the molecule. Specific examples thereof include alkylthio(thietanylthio)tin, bis(alkylthio)bis(thietanylthio)tin, alkylthio(alkylthio)bis(thietanylthio)tin, bis(thietanylthio)cyclic dithiotin compounds, and alkyl(thietanylthio)tin compounds.

Specific examples of alkylthio(thietanylthio)tin include methylthiotris(thietanylthio)tin, ethylthiotris(thietanylthio) tin, propylthiotris(thietanylthio)tin, and isopropylthiotris(thietanylthio)tin.

Specific examples of bis(alkylthio)bis(thietanylthio)tin include bis(methylthio)bis(thietanylthio)tin, bis(ethylthio) bis(thietanylthio)tin, bis(propylthio)bis(thietanylthio)tin, and bis(isopropylthio)bis(thietanylthio)tin.

Specific examples of alkylthio(alkylthio)bis(thietanylthio)tin include ethylthio(methylthio)bis(thietanylthio)tin, methylthio(propylthio)bis(thietanylthio)tin, isopropylthio(methylthio)bis(thietanylthio)tin, ethylthio(propylthio)bis(thietanylthio)tin, ethylthio(isopropylthio)bis(thietanylthio)tin, and isopropylthio(propylthio)bis(thietanylthio)tin.

Specific examples of bis(thietanylthio)cyclic dithiotin compounds include bis(thietanylthio)dithiastannetane, bis(thietanylthio)dithiastannolane, bis(thietanylthio)dithiastannolane, and bis(thietanylthio)trithiastannocane.

Specific examples of alkyl(thietanylthio)tin compounds include methyltris(thietanylthio)tin, dimethylbis(thietanylthio)tin, butyltris(thietanylthio)tin, tetrakis(thietanylthio)tin, tetrakis(thietanylthio)germanium, and tris(thietanylthio)bismuth.

(Polyamine Compound)

The polyamine compound is a compound having two or more $NH^2$ groups in one molecule, and can form a urea bond according to a reaction with a polyisocyanate and can form a thiourea bond according to a reaction with a polyisothiocyanate. Specific examples of polyamine compounds include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

(Epoxy Compound)

The epoxy compound is a compound having an epoxy group in the molecule. The epoxy group is a polymerizable group that can undergo ring-opening polymerization. The epoxy compounds are generally classified into aliphatic epoxy compounds, alicyclic epoxy compounds and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and tris(2-hydroxyethyl)isocyanurate triglycidyl ether.

Specific examples of alicyclic epoxy compounds include isophoronediol diglycidyl ether and bis-2,2-hydroxycyclohexylpropane diglycidyl ether.

Specific examples of aromatic epoxy compounds include resole syndiglycidyl ether, bisphenol A diglycidyl ether, bisphenol F diglycidyl ether, bisphenol S diglycidyl ether, diglycidyl orthophthalate, phenol novolac polyglycidyl ether, and cresol novolac polyglycidyl ether.

In addition, in addition to the above examples, an epoxy compound having a sulfur atom in the molecule together with an epoxy group can be used. Such epoxy compounds containing sulfur atoms include linear aliphatic compounds and cycloaliphatic compounds.

Specific examples of linear aliphatic epoxy compounds containing sulfur atoms include bis(2,3-epoxypropyl) sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithia octane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cycloaliphatic epoxy compounds containing sulfur atoms include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

(Compound Having Radically Polymerizable Group)

The compound having a radically polymerizable group has a polymerizable group that can undergo radical polymerization. Examples of radically polymerizable groups include acryloyl, methacryloyl, allyl and vinyl.

In the following, a compound having a polymerizable group selected from acryloyl groups and methacryloyl groups will be referred to as a "(meth)acrylate compound". Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl(meth)acrylate), bisphenol A di(meth)acrylate, 2,2-bis(4-(meth)acryloxyethoxyphenyl)propane, 2,2-bis(4-(meth)acryloxydiethoxyphenyl) propane, 2,2-bis(4-(meth)acryloyloxyethoxyphenyl) propane, 2,2-bis(3,5-dibromo-4-(meth)acryloyloxyethoxyphenyl)propane, 2,2-bis(4-(meth)acryloyloxydipropoxyphenyl)propane, bisphenol F di(meth) acrylate, 1,1-bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylene dithiol di(meth)acrylate, mercaptoethylsulfide di(meth)acrylate, and difunctional urethane (meth)acrylate.

Specific examples of compounds having an allyl group (allyl compound) include allyl glycidyl ether, diallyl phthalate, diallyl terephthalate, diallyl isophthalate, diallyl carbonate, diethylene glycol bisallyl carbonate, methoxy polyethylene glycol allyl ether, polyethylene glycol allyl ether, methoxy polyethylene glycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds having a vinyl group (vinyl compound) include $\alpha$-methylstyrene, $\alpha$-methylstyrene dimer, styrene, chlorostyrene, methylstyrene, bromostyrene, dibromostyrene, divinylbenzene, and 3,9-divinylspirobi (m-dioxane).

The photochromic article can include one or more layers known as functional layers of the photochromic article such as a protective layer for improving the durability of the photochromic article, an antireflection layer, a water-repellent or hydrophilic antifouling layer, a defogging layer, and a primer layer for improving adhesion between layers at any position.

The photochromic article can be an optical article. One form of the optical article is a spectacle lens. Such a spectacle lens can also be called a photochromic lens or a photochromic spectacle lens. In addition, as one form of the optical article, a goggle lens, a visor (cap) part of a sun visor, a shield member of a helmet and the like may be exemplified. The photochromic composition which is a polymerizable composition is applied to the substrate for these optical articles, a curing treatment is performed on the applied composition, a photochromic layer is formed, and thereby an optical article having an anti-glare function can be obtained.

[Eyeglasses]

One aspect of the present invention relates to eyeglasses having a spectacle lens that is one form of the photochromic article. Details of the spectacle lens included in the eyeglasses are as described above. By providing such a spectacle lens, for example, the eyeglasses can exhibit an anti-glare effect like sunglasses when the photochromic compound is colored upon receiving sunlight outdoors, and the photochromic compound can fade upon returning indoors, and thus the transmittance can be recovered. For the eyeglasses, a known technique can be applied to the configuration of the frame.

[Examples]

Hereinafter, the present invention will be described in more detail with reference to examples. However, the present invention is not limited to embodiments shown in examples.

In the following, the molecular structure was identified using a nuclear magnetic resonance device (NMR). Proton NMR of ECS-400 (commercially available from JEOL Ltd.) was used as NMR. As a measurement solvent, deuterated chloroform was mainly used, and e.g. heavy dimethylsulfoxide, heavy acetone, heavy acetonitrile, heavy benzene, heavy methanol, or heavy pyridine was appropriately used only when it was poorly soluble in deuterated chloroform.
The purity was analyzed using high-performance liquid chromatography (HPLC). LC-2040C (commercially available from Shimadzu Corporation) was used as HPLC. YMC-Triart C18 was used for the column, and the measurement temperature was set to 40°C. For the mobile phase, a mixed solvent containing water containing 0.1% of trifluoroacetic acid and acetonitrile was used and the flow rate was 0.4 mL/min.

For mass spectrometry, a device including SQD2 was used as a mass spectrometry unit in ACQUITY UPLC H-Class system (UPLC) (commercially available from Nihon Waters K.K.). ACQUITY UPLC BEH C18 was used as the column, and the measurement temperature was set to 40°C. For the mobile phase, a mixed solvent containing water to which formic acid was added and acetonitrile was used, a concentration gradient was applied and the flow rate was set to 0.61 mL/min for flowing. An electrospray ionization (ESI) method was used for ionization.
CHN (carbonhydrogennitrogen) elemental analysis was performed by a combustion method.

[Example 1]

From the reaction product shown in Table 1, a product shown in Table 1 was obtained by the following method.

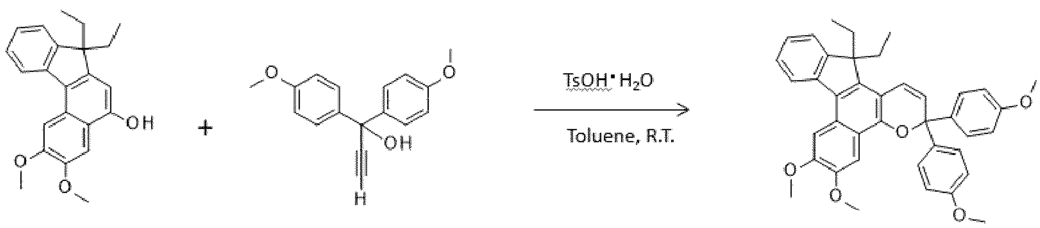


Under an argon atmosphere, p-toluenesulfonic acid monohydrate (0.15 g, 0.80 mmol) was added to a toluene solution (36 mL) containing Reaction Product 1 (1.4 g, 4 mmol) and Reaction Product 2 (2.1 g, 8 mmol) shown in Table 1, and the mixture was stirred at room temperature overnight. A sodium hydroxide aqueous solution (1.0 M, 37 mL) was added thereto and the mixture was stirred for about 20 minutes. Impurities were removed by filtration, extraction with toluene (30 mL×2) was performed, and the combined organic layer was then washed with water (20 mL×2) and concentrated. The obtained residue was purified through column chromatography ($SiO_2$: 200 g, heptane/chloroform (volume basis)=70/30 to 60/40) (1.2 g, brown solid). The obtained solid was suspended in heptane/ethyl acetate (2/1 (volume basis), 90 mL), subjected to an ultrasonic treatment for about 30 minutes, and filtered and dried, and thereby, as the final product, a product shown in Table 1 was obtained as a light yellow-green solid (0.9 g).

The obtained products were analyzed by the following method.
The structure was identified by a nuclear magnetic resonance device (NMR).
The purity was analyzed by HPLC and was 98% in terms of area ratio.
s a result of mass spectrometry, the measured value was 598.8 (M+, a relative intensity of 100) for the calculated value of the exact mass of 598.272.

As a result of CHN elemental analysis by a combustion method, C: 80.0%, H: 6.6%, N: 0% for the calculated values C: 80.2%, H: 6.4%, and N: 0%.
It was confirmed based on the above analysis results that a product shown in Table 1 as a desired compound was produced comprehensively.

[Examples 2-11, and Comparative Examples]

Products shown in Table 1 were obtained by the same operation except that reaction products shown in Table 1 were used as Reaction Product 1 and Reaction Product 2 used to synthesize the present photochromic compound.

The obtained products were analyzed by the method described above. The analysis results are shown in Table 1.

[Evaluation Method]

<Measurement of Solution Spectrum>

Compounds of Example 3 and comparative examples were dissolved in chloroform containing no stabilizer to prepare a chloroform solution containing the compound.

A 1 cm square quartz spectroscopic cell containing the prepared solution was covered, and ultraviolet rays were emitted using UV-LED (commercially available from Hamamatsu Photonics K.K.) (a combination of LIGHTNINGCURE LC-L1V5 and L14310-120, an output of 70%) as an ultraviolet light source for 15 seconds. The solution was stirred with a small stirrer during UV emission. Within 10 seconds after UV emission was completed, the absorbance was measured using a UV-visible spectrophotometer (UV-1900i, commercially available from Shimadzu Corporation, a measurement wavelength of 700-400 nm, wavelength increments of 2 nm, survey mode). The absorbance was measured at room temperature (23-28°C). Here, the concentration of the solution was adjusted so that the absorbance at the first absorption wavelength (the peak of the absorption intensity observed at the longest wavelength) was 0.95-1.05. In addition, the absorbance was measured every 10 seconds, and the attenuation of the absorbance was measured. Normalization was performed so that the peak of the first absorption wavelength in the first absorbance measurement became 1, the attenuation of the absorbance was then measured, data of fading for an initial 100 seconds (11 absorbance measurements) from the change in absorbance over time was analyzed with a first -order reaction model, and thus the reaction rate constant was obtained. If [A0] is the initial concentration of the colored component, that is, the normalized absorbance value of 1, [A] is the concentration of the colored component after a certain time, that is, the normalized absorbance value, t is time (seconds), and k is a rate constant, the first -order reaction can be expressed as in the following formula. This formula is called an integral rate formula.

[Math. 1]

$$\ln \frac{[A]}{[A_0]} = -kt$$

As an example, Fig. 1 shows solution spectrums obtained for the compound of Example 3, and Fig. 2 shows a graph plotted using the above formula for the compound of Example 3 and the compound of the comparative example. The graph shown in Fig. 2 is a graph showing the absorbance assuming an integral rate formula of a first - order reaction in order to calculate a reaction rate constant for each of the compound of Example 3 and the compound of the comparative example, that is, the change in concentration of a colored component in the system over time.

Based on the results shown in Fig. 1 and Fig. 2, it was confirmed that the compound of Example 3 exhibited photochromic properties of structural transition to a molecular structure with strong absorption in a visible range upon irradiation with ultraviolet rays and exhibited a fast fade rate.

The same measurement was performed for Examples 1, 2, and 4-11. It was confirmed that compounds of all examples exhibited photochromic properties of structural transition to a molecular structure with strong absorption in a visible range upon irradiation with ultraviolet rays and exhibited a fast fade rate as in Example 3.

Table 1 (Table 1-1 to Table 1-3) shows the reaction rate constants obtained for Examples 1-11 and comparative examples.

[Table 1-1]

| | Reaction Product 1 | Reaction Product 2 | Product | HPLC purity (%) | Calculated exact mass | Mass spectrometry measured value | CHN composition calculated value (%) | CHN elemental analysis measured value (%) | Reaction rate constant $(10^{-3}$ sec.$^{-1})$ |
|---|---|---|---|---|---|---|---|---|---|
| Example 1 | 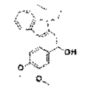 | 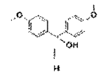 | 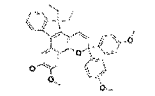 | 98 | 598.272 | 598.8 | C: 80.2% H: 6.4% | C: 80.0% H: 6.6% | 7.8 |
| Example 2 | 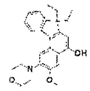 | 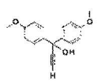 | 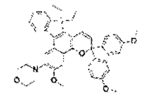 | 98 | 653.314 | 653.5 | C: 79% H: 6.6% N: 2.1% | C: 76.0% H: 6.7% N: 2.4% | 8.0 |
| Example 3 | 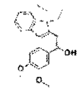 | 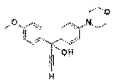 | 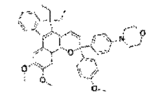 | 98 | 653.314 | 653.7 | C: 79% H: 6.6% N: 2.1% | C: 78.0% H: 6.8% N: 2.2% | 9.2 |
| Example 4 | 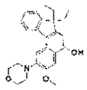 | 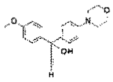 | 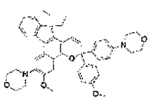 | 98 | 708.356 | 708.8 | C: 77.9% H: 6.8% N: 4% | C: 76.0% H: 6.80% N: 3.9% | 9.0 |

[Table 1-2]

| | Reaction Product 1 | Reaction Product 2 | Product | HPLC purity (%) | Calculated exact mass | Mass spectrometry measured value | CHN composition calculated value (%) | CHN elemental analysis measured value (%) | Reaction rate constant $(10^{-3}$ sec.$^{-1})$ |
|---|---|---|---|---|---|---|---|---|---|
| Example 5 | 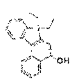 | 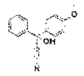 | 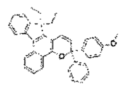 | 98 | 544.293 | 544.5 | C: 85.8% H: 6.3% | C: 84.8% H: 6.7% | 6.0 |
| Example 6 | 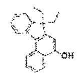 | 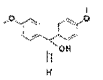 | 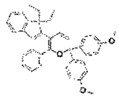 | 98 | 538.251 | 538.6 | C: 84.7% H: 6.4% | C: 83.2% H: 7.2% | 6.2 |
| Example 7 | 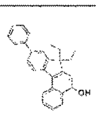 | 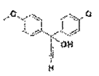 | 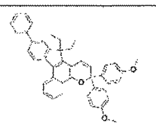 | 97 | 614.282 | 614.8 | C: 86.0% H: 6.2% | C: 85.5% H: 7.2% | 6.2 |
| Example 8 | 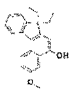 | 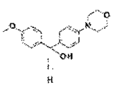 | 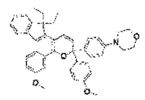 | 98 | 623.304 | 623.6 | C: 80.9% H: 6.6% N: 2.2% | C:79.8% H: 6.8% N: 2.5% | 9.0 |

[Table 1-3]

| | Reaction Product 1 | Reaction Product 2 | Product | HPLC purity (%) | Calculated exact mass | Mass spectrometry measured value | CHN composition calculated value (%) | CHN elemental analysis measured value (%) | Reaction rate constant $(10^{-5}$ sec.$^{-1})$ |
|---|---|---|---|---|---|---|---|---|---|
| Example 9 | 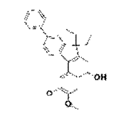 | 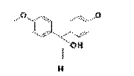 | 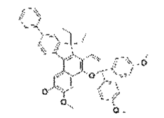 | 96 | 674.303 | 674.5 | C: 81.9% H: 6.3% | C: 82.2% H: 5.1% O: 12.0% | 7.2 |
| Example 10 | 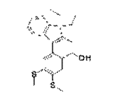 | 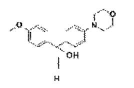 | 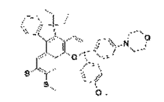 | 97 | 685.268 | 685.8 | C: 75.3% H: 6.3% N: 2% | C: 77.2% H: 7.1% N: 1.8% | 8.0 |
| Example 11 | 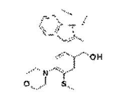 | 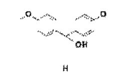 | 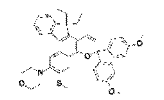 | 97 | 669.291 | 670.2 | C: 77.1% H: 6.5% N: 2.1% | C: 76.81% H: 7.0% N: 2.2% | 8.0 |
| Comparative Example 1 | 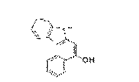 | 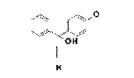 | 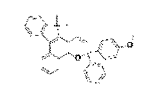 | 98 | 480.209 | 480.3 | C: 87.5% H: 5.9% O: 6.7% | C: 86.8% H: 6.3% O: 6.8% | 2.8 |

[Synthesis of Compounds]

Compounds 1-16 shown below were synthesized by the above synthesis method or with reference to the documents shown above regarding the compound synthesis method. Compounds 1-8 were embodiments of the present photochromic compounds. Compound 9 was a compound of Formula (A), Compound 13 was a compound of Formula (C), and Compounds 10-12 and 14-16 were compounds of Formula (B). Compounds 9-16 were identified in the same method as described in the above reference publication, and it was confirmed that the compounds were synthesized.

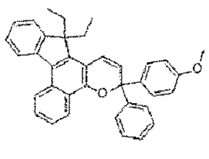

Compound 1

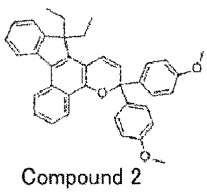

Compound 2

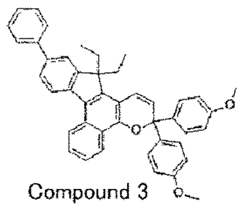

Compound 3

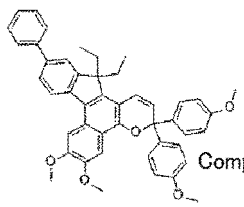

Compound 4

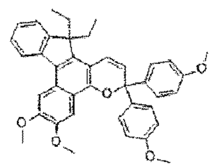

Compound 5

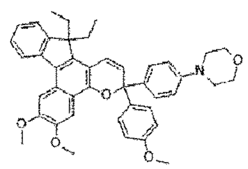

Compound 6

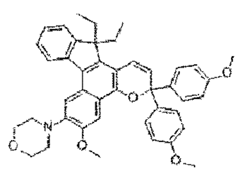

Compound 7

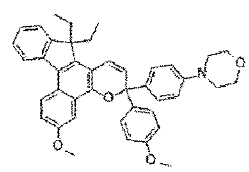

Compound 8

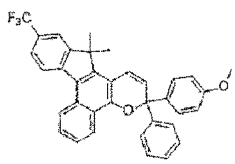

Compound 9

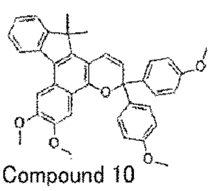

Compound 10

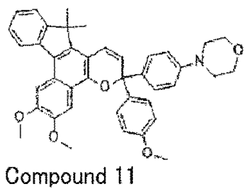

Compound 11

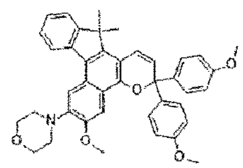

Compound 12

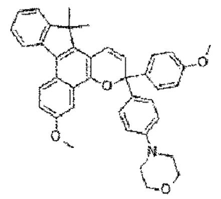
Compound 13

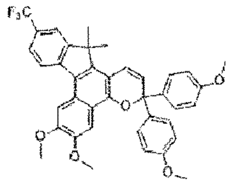
Compound 14

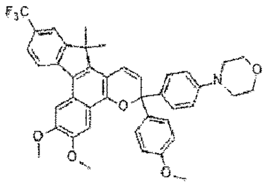
Compound 15

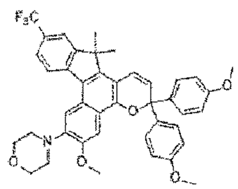
Compound 16

[Examples 21-40 and Comparative Examples 1-3]

<Preparation of Photochromic Composition (Polymerizable Composition)>

In a plastic container, with respect to a total amount of 100 parts by mass (pbm) of (meth)acrylates, 68 pbm of polyethylene glycol diacrylate, 12 pbm of trimethylolpropane trimethacrylate, and 20 pbm of neopentyl glycol dimethacrylate were mixed to prepare a (meth)acrylate mixture. 2.5 pbm of a photochromic compound was mixed with respect to 100 pbm of the (meth) acrylate mixture. For a composition containing a plurality of photochromic compounds, Table 2 (Table 2-1 and Table 2-2) shows the mass ratio of respective photochromic compounds based on a total amount of 10 of the photochromic compounds. In addition, a photopolymerization initiator (phenyl bis(2,4,6-trimethylbenzoyl)phosphine oxide), an antioxidant [bis(3-tert-butyl-4-hydroxy-5-methylphenyl)propionic acid)][ethylene bis (oxyethylene) and a light stabilizer (bis(1,2,2,6,6-pentamethyl-4-piperidyl) sebacate) were mixed and sufficiently stirred and a silane coupling agent (γ-methacryloxypropyltrimethoxysilane) was then added dropwise with stirring. Then, defoaming was performed using an automatic revolution type stirring and defoaming device.
A photochromic composition was prepared by the above method.
The comparative compound used in Comparative Example 3 was the following compound.

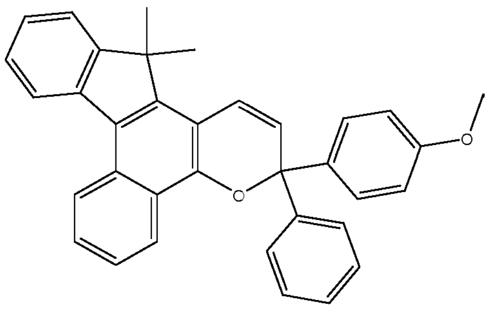

<Formation of Primer Layer>

A plastic lens substrate (commercially available from HOYA, product name EYAS: a center thickness of 2.5 mm, a diameter of 75 mm, and a spherical lens power of -4.00) was immersed in a sodium hydroxide aqueous solution having a concentration of 10 mass% (a liquid temperature of 60°C) for 5 minutes, washed with an alkali and additionally washed with pure water and dried. Then, a water-based polyurethane resin liquid (polycarbonate polyol-based polyurethane emulsion, a viscosity of 100 cPs, and a solid content concentration of 38 mass%) was applied to the convex surface of the plastic lens substrate in an environment of room temperature and a relative humidity of 40-60% using a spin coater MS-B150 (commercially available from Mikasa Corporation) at a rotational speed of 1,500 rpm for 1 minute according to a spin coating method and then dried naturally for 15 minutes, and thereby a primer layer having a thickness of 5.5 μm was formed.

<Formation of Photochromic Layer>

The photochromic composition prepared above was added dropwise to the primer layer, and applied by a spin coating method using a program in which the rotational speed was changed in a slope mode from a rotational speed of 500-1,500 rpm over 1 minute, and rotation was additionally performed at 1,500 rpm for 5 seconds using MS-B150 (commercially available from Mikasa Corporation). Then, ultraviolet rays (with a dominant wavelength of 405 nm) were emitted to the photochromic composition applied on the primer layer formed on the plastic lens substrate in a nitrogen atmosphere (with an oxygen concentration of 500 ppm or less) for 40 seconds, and the composition was cured to form a photochromic layer. The thickness of the formed photochromic layer was 45 μm. Accordingly, a photochromic article

(spectacle lens) was produced.

[Evaluation Method]

<Evaluation of Coloring Concentration>

The luminous reflectance was obtained by the following method according to JIS T 7333: 2005.

Light was emitted to the convex surface of each spectacle lens of examples and comparative examples using a xenon lamp as a light source through an air mass filter for 15 minutes, and the photochromic layer was colored. Light emission was performed so that the irradiance and irradiance tolerance were values shown in Table 2 as specified in JIS T 7333: 2005. The transmittance during the coloring was measured with a spectrophotometer (commercially available from Otsuka Electronics Co., Ltd.). Table 3 shows the luminous transmittance T(%) obtained from the measurement results in a wavelength range of 380-780 nm. A smaller value of T(%) means that the photochromic compound is colored at a higher concentration.

[Table 2]

| Wavelength range (nm) | Irradiance ($W/m^2$) | Irradiance tolerance ($W/m^2$) |
|---|---|---|
| 300~340 | <2.5 | - |
| 340~ 380 | 5.6 | ±1.5 |
| 500~ 420 | 12 | ±3.0 |
| 420~480 | 12 | ±3.0 |
| 460~500 | 26 | ±2.6 |

<Evaluation of Fade Rate>

The fade rate was evaluated by the following method.

The transmittance (measurement wavelength: 550 nm) of each spectacle lens of the examples and comparative example before light emission (uncolored state) was measured with a spectrophotometer (commercially available from Otsuka Electronics Co., Ltd.). The transmittance measured here is called an "initial transmittance".

Light was emitted to each spectacle lens using a xenon lamp as a light source through an air mass filter for 15 minutes, and the photochromic layer was colored. Light emission was performed sot that the irradiance and irradiance tolerance were values shown in Table 1 as specified in JIS T 7333: 2005. The transmittance during the coloring was measured in the same manner as the initial transmittance. The transmittance measured here is called a "transmittance during coloring".

Then, the time required for the transmittance to reach [(initial transmittance-transmittance during coloring)/2] from the time when light emission was stopped was measured. This time is called a "half-life time". It can be said that the shorter the half-life time, the higher the fade rate. Table 3 shows the obtained half-life time.

[Table 3]

| | Composition | Mass ratio | Coloring concentration T (%) | Fade rate half-life time (seconds) |
|---|---|---|---|---|
| Example 21 | Compound 1 / Compound 5 | 2/8 | 16.8 | 170 |
| Example 22 | Compound 1 / Compound 5 / Compound 6 | 1 / 5 / 4 | 16.6 | 170 |
| Example 23 | Compound 2 / Compound 5 | 1/9 | 16.2 | 175 |
| Example 24 | Compound 2 / Compound 5 / Compound 6 | 1/5/4 | 16.2 | 175 |
| Example 25 | Compound 2 / Compound 5 / Compound 7 | 1 / 5 / 4 | 16.2 | 170 |

(continued)

| | Composition | Mass ratio | Coloring concentration T (%) | Fade rate half-life time (seconds) |
|---|---|---|---|---|
| Example 26 | Compound 2 / Compound 5 / Compound 8 | 2 / 4/ 4 | 17.5 | 155 |
| Example 27 | Compound 2 / Compound 6 / Compound 7 | 1 / 5 /4 | 16.2 | 165 |
| Example 28 | Compound 3 / Compound 4 / Compound 6 | 1 / 5 / 4 | 16.0 | 165 |
| Example 29 | Compound 3 / Compound 4 / Compound 7 | 1 / 5 / 4 | 15.8 | 165 |
| Example 30 | Compound 1 / Compound 1 1 | 1 / 9 | 14.8 | 185 |
| Example 31 | Compound 1 / Compound 1 1 / Compound 1 2 | 1/ 6 / 3 | 14.2 | 190 |
| Example 32 | Compound 2 / Compound 7 / Compound 1 6 | 1/ 5 / 4 | 16.0 | 155 |
| Example 33 | Compound 2 / Compound 1 3 | 3 / 7 | 15.6 | 180 |
| Example 34 | Compound 2 / Compound 1 4 | 2 / 8 | 15.4 | 160 |
| Example 35 | Compound 2 / Compound 1 5 | 1 / 9 | 16.4 | 150 |
| Example 36 | Compound 2 / Compound 1 4 / Compound 1 6 | 1 / 5 / 4 | 16.0 | 150 |
| Example 37 | Compound 9 / Compound 5 | 1 / 9 | 16.4 | 160 |
| Example 38 | Compound 9 / Compound 5 / Compound 6 | 1 / 5 / 4 | 17.0 | 155 |
| Example 39 | Compound 9 / Compound 5 / Compound 7 | 1 / 5 / 4 | 16.4 | 160 |
| Example 40 | Compound 9 / Compound 6 / Compound 1 3 | 1 / 4 / 5 | 16.8 | 155 |
| Comparative Example 1 | Compound 5 | 1 0 | 23.0 | 160 |
| Comparative Example 2 | Compound 8 | 1 0 | 24.4 | 150 |
| Comparative Example 3 | Comparative Compound / Compound 1 0 / Compound 1 1 | 2 / 4 / 4 | 16.0 | 245 |

Based on the results shown in Table 3, it was confirmed that each spectacle lens of the examples was a photochromic article that was colored at a high concentration in a visible range and exhibited a fast fade rate.

## Claims

**1.** A compound, which is a photochromic compound of formula (1) :

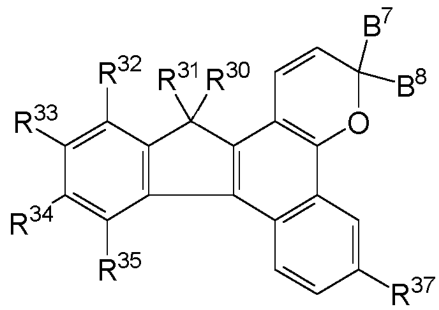

(1)

wherein

$R^{30}$ and $R^{31}$ each independently are selected from linear or branched $C_{2-10}$-alkyl, each optionally substituted,
$R^{32}$-$R^{35}$ each independently are H or a substituent other than an electron-attracting group,
$R^{37}$ is H or an electron-donating group selected from methoxy, ethoxy, phenoxy, methylsulfide, phenylsulfide, dimethylamino, pyrrolidino, piperidino, morpholino and thiomorpholino, and
$B^7$ and $B^8$ each independently are H or a substituent.

**2.** The compound of claim 1, wherein $R^{30}$ and $R^{31}$ each independently are ethyl, propyl, isopropyl, n-butyl, s-butyl or t-butyl, and preferably $R^{30}$ and $R^{31}$ both are ethyl.

**3.** The compound of claim 1 or 2, wherein $B^7$ and $B^8$ each independently are phenyl, naphthyl, fluorenyl, benzofluorenyl, fluoranthenyl, dibenzofuranyl or dibenzothiophenyl, each optionally substituted; and peferably phenyl substituted with one or more substituents selected from methoxy, methylsulfide, amino, dimethylamino, piperidino, morpholino, thiomorpholino, phenyl, F, Cl, Br, I, trifluoromethyl and cyano.

**4.** The compound of any of claims 1-3, wherein $R^{32}$-$R^{35}$ each independently are H, phenyl or methoxy, and preferably $R^{32}$-$R^{35}$ are all H.

**5.** A compound, which is a photochromic compound of formula (1a) :

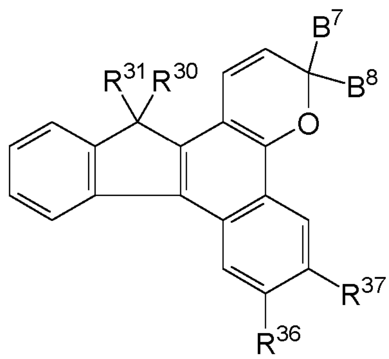

(1a)

wherein

$R^{30}$ and $R^{31}$ each independently are selected from linear or branched $C\geq_2$-alkyl, $C\geq_3$-cycoalkyl and - $(R^{100})_nR^{101}$ wherein $R^{100}$ is alkyleneoxy, $R^{101}$ is alkyl, n is an integer of ≥ 1; each optionally substituted,
$R^{36}$ and $R^{37}$ each independently are an electron-donating group, and
$B^7$ and $B^8$ each independently are H or a substituent.

**6.** A composition, which is a photochromic composition comprising one or more photochromic compounds of any of claims 1-5.

**7.** The composition of claim 6, which contains one or more photochromic compounds of formula (1) as defined in claim 1 wherein $R^{30}$ and $R^{31}$ both are ethyl, and $R^{37}$ is H, and one or more photochromic compounds selected from

compounds of the formulae (B) and (C):

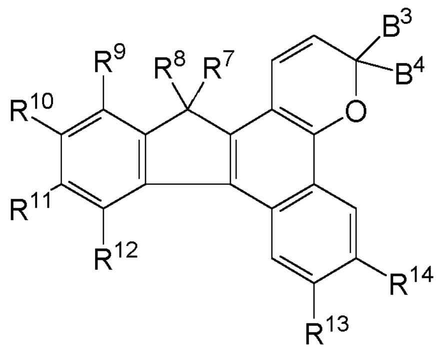

(B)

wherein $R^7$-$R^{12}$, $B^3$ and $B^4$ each independently are H or a substituent, and $R^{13}$ and $R^{24}$ each independently are an electron-donating group; and

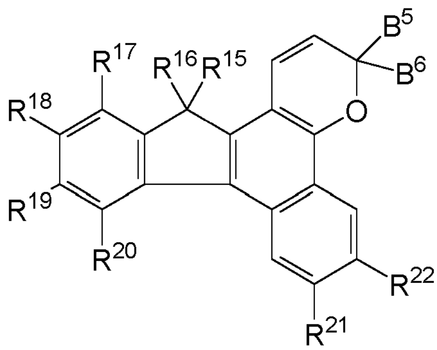

(C)

wherein $R^{15}$-$R^{20}$, $B^5$ and $B^6$ each independently are H or a substituent, and one of $R^{21}$ and $R^{22}$ is H, and the other is an electron-donating group.

**8.** A composition, which is a photochromic composition comprising one or more photochromic compounds of formula (1b):

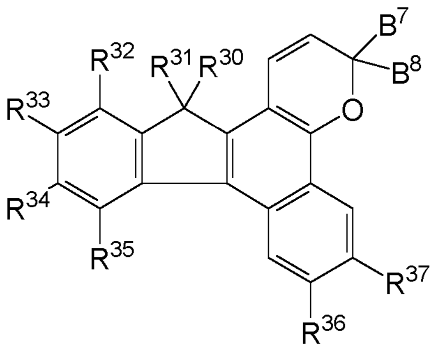

(1b)

wherein $R^{30}$ and $R^{31}$ both are ethyl, $R^{32}$-$R^{35}$ each independently are H or a substituent other than an electron-attracting group, and one of $R^{36}$ and $R^{37}$ is an electron-donating group, and the other is H or an electron-donating group, and one or more photochromic compounds of Formula (A):

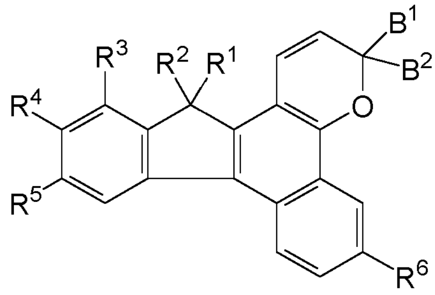

(A)

wherein $R^1$-$R^6$, $B^1$ and $B^2$ each independently are H or a substituent.

**9.** The composition of any of claims 6-8, further comprising a polymerizable compound.

**10.** A photochromic article comprising a cured product obtained by curing the composition of claim 9.

**11.** The photochromic article of claim 10, comprising a substrate and a photochromic layer which is the cured product.

**12.** The photochromic article of claim 10 or 11, which is a spectacle lens, a goggle lens, a visor part of a sun visor or a shield member of a helmet.

**13.** Eyeglasses comprising the spectacle lens of claim 12.

**Patentansprüche**

**1.** Eine Verbindung, die eine photochrome Verbindung der Formel (1) ist:

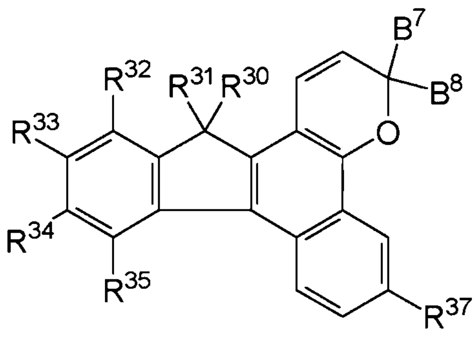

(1)

wobei

$R^{30}$ und $R^{31}$ jeweils unabhängig voneinander aus linearem oder verzweigtem $C_{2-10}$-Alkyl ausgewählt sind, die jeweils optional substituiert sind,
$R^{32}$-$R^{35}$ jeweils unabhängig voneinander H oder ein Substituent außer einer elektronenanziehenden Gruppe sind,
$R^{37}$ H oder eine elektronenabgebende Gruppe ist, ausgewählt aus Methoxy, Ethoxy, Phenoxy, Methylsulfid, Phenylsulfid, Dimethylamino, Pyrrolidino, Piperidino, Morpholino und Thiomorpholino, und
$B^7$ und $B^8$ jeweils unabhängig voneinander H oder ein Substituent sind.

**2.** Die Verbindung gemäß Anspruch 1, wobei $R^{30}$ und $R^{31}$ jeweils unabhängig voneinander Ethyl, Propyl, Isopropyl, n-Butyl, s-Butyl oder t-Butyl sind, und vorzugsweise $R^{30}$ und $R^{31}$ beide Ethyl sind.

**3.** Die Verbindung gemäß Anspruch 1 oder 2, wobei $B^7$ und $B^8$ jeweils unabhängig voneinander Phenyl, Naphthyl, Fluorenyl, Benzofluorenyl, Fluoranthenyl, Dibenzofuranyl oder Dibenzothiophenyl sind, die jeweils optional substituiert sind; und vorzugsweise Phenyl, das mit einem oder mehreren Substituenten substituiert ist, ausgewählt aus Methoxy, Methylsulfid, Amino, Dimethylamino, Piperidino, Morpholino, Thiomorpholino, Phenyl, F, Cl, Br, I, Trifluormethyl und Cyano.

4. Die Verbindung gemäß mindestens einem der Ansprüche 1 bis 3, wobei $R^{32}$ bis $R^{35}$ jeweils unabhängig voneinander H, Phenyl oder Methoxy sind, und vorzugsweise $R^{32}$ bis $R^{35}$ alle H sind.

5. Eine Verbindung, die eine photochrome Verbindung der Formel (1a) ist:

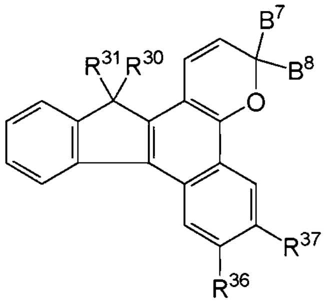

(1a)

wobei

$R^{30}$ und $R^{31}$ jeweils unabhängig voneinander ausgewählt sind aus linearem oder verzweigtem $C_{\geq 2}$-Alkyl, $C_{\geq 3}$-Cycloalkyl und $-(R^{100})_nR^{101}$, wobei $R^{100}$ Alkylenoxy ist, $R^{101}$ Alkyl ist, n eine ganze Zahl von ≥ 1 ist; jeweils optional substituiert,
$R^{36}$ und $R^{37}$ jeweils unabhängig voneinander eine elektronenabgebende Gruppe sind und
$B^7$ und $B^8$ jeweils unabhängig voneinander H oder ein Substituent sind.

6. Eine Zusammensetzung, die eine photochrome Zusammensetzung ist, die eine oder mehrere photochrome Verbindungen gemäß mindestens einem der Ansprüche 1 bis 5 umfasst.

7. Die Zusammensetzung gemäß Anspruch 6, die enthält eine oder mehrere photochrome Verbindungen der Formel (1) gemäß Anspruch 1, wobei $R^{30}$ und $R^{31}$ beide Ethyl sind, und $R^{37}$ H ist, und eine oder mehrere photochrome Verbindungen, ausgewählt aus Verbindungen der Formeln (B) und (C):

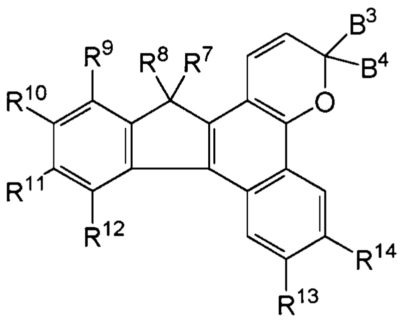

(B)

wobei $R^7$-$R^{12}$, $B^3$ und $B^4$ jeweils unabhängig voneinander H oder ein Substituent sind, und $R^{13}$ und $R^{14}$ jeweils unabhängig voneinander eine elektronenabgebende Gruppe sind; und

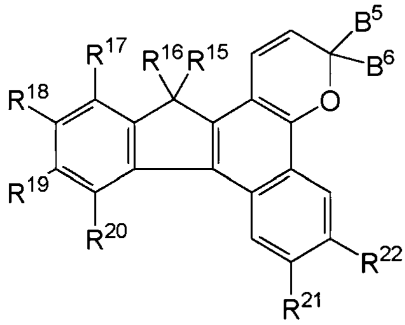

(C)

wobei $R^{15}$-$R^{20}$, $B^5$ und $B^6$ jeweils unabhängig voneinander H oder ein Substituent sind, und eines von $R^{21}$ und $R^{22}$ H ist, und das andere eine elektronenabgebende Gruppe ist.

**8.** Eine Zusammensetzung, die eine photochrome Zusammensetzung ist, umfassend eine oder mehrere photochrome Verbindungen der Formel (1b):

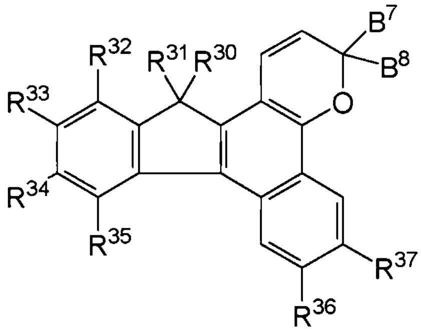

(1b)

wobei $R^{30}$ und $R^{31}$ beide Ethyl sind, $R^{32}$-$R^{35}$ jeweils unabhängig voneinander H oder ein Substituent außer einer elektronenanziehenden Gruppe sind, und einer von $R^{36}$ und $R^{37}$ eine elektronenabgebende Gruppe ist, und der andere H oder eine elektronenabgebende Gruppe ist, und eine oder mehrere photochrome Verbindungen der Formel (A) :

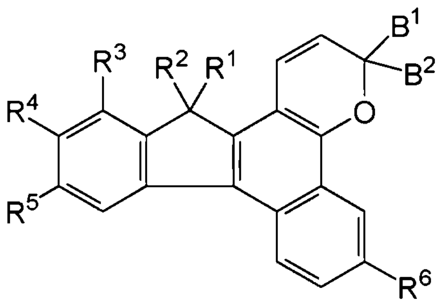

(A)

wobei $R^1$-$R^6$, $B^1$ und $B^2$ jeweils unabhängig voneinander H oder ein Substituent sind.

**9.** Die Zusammensetzung gemäß mindestens einem der Ansprüche 6-8, die ferner eine polymerisierbare Verbindung umfasst.

**10.** Ein photochromer Gegenstand, umfassend ein gehärtetes Produkt, das durch Härten der Zusammensetzung gemäß Anspruch 9 erhalten wird.

**11.** Der photochrome Gegenstand gemäß Anspruch 10, umfassend ein Substrat und eine photochrome Schicht, die das gehärtete Produkt ist.

**12.** Der photochrome Gegenstand gemäß Anspruch 10 oder 11, der ein Brillenglas, ein Schutzbrillenglas, ein Visierteil

einer Sonnenblende oder ein Schutzschildelement eines Helms ist.

**13.** Brille, umfassend das Brillenglas gemäß Anspruch 12.

## Revendications

**1.** Composé, qui est un composé photochromique de formule (1) :

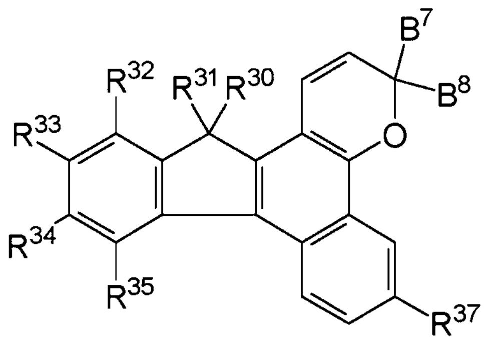

(1)

dans laquelle

$R^{30}$ et $R^{31}$ sont chacun indépendamment sélectionnés parmi un alkyle en $C_{2-10}$ linéaire ou ramifié, chacun facultativement substitué,
$R^{32}$-$R^{35}$ sont chacun indépendamment H ou un substituant autre qu'un groupe attracteur d'électrons,
$R^{37}$ est H ou un groupe donneur d'électrons sélectionné parmi méthoxy, éthoxy, phénoxy, méthylsulfure, phénylsulfure, diméthylamino, pyrrolidino, pipéridino, morpholino et thiomorpholino, et
$B^7$ et $B^8$ sont chacun indépendamment H ou un substituant.

**2.** Composé selon la revendication 1, dans lequel $R^{30}$ et $R^{31}$ sont chacun indépendamment éthyle, propyle, isopropyle, n-butyle, s-butyle ou t-butyle, et de préférence $R^{30}$ et $R^{31}$ sont tous deux éthyle.

**3.** Composé selon la revendication 1 ou la revendication 2, dans lequel $B^7$ et $B^8$ sont chacun indépendamment phényle, naphtyle, fluorényle, benzofluorényle, fluoranthényle, dibenzofuranyle ou dibenzothiophényle, chacun facultativement substitué ; et de préférence phényl substitué par un ou plusieurs substituants sélectionnés parmi méthoxy, sulfure de méthyle, amino, diméthylamino, pipéridino, morpholino, thiomorpholino, phényle, F, Cl, Br, I, trifluorométhyle et cyano.

**4.** Composé selon l'une quelconque des revendications 1 à 3, dans lequel $R^{32}$-$R^{35}$ sont chacun indépendamment H, phényl ou méthoxy, et de préférence $R^{32}$-$R^{35}$ sont tous H.

**5.** Composé, qui est un composé photochromique de formule (1a) :

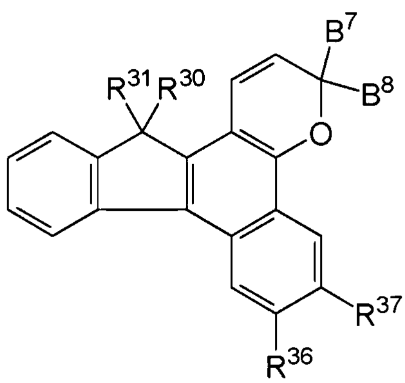

(1a)

dans laquelle

$R^{30}$ et $R^{31}$ sont chacun indépendamment sélectionnés parmi un alkyle en $C_{\geq 2}$- linéaire ou ramifié, un cycloalkyle en $C_{\geq 3}$- et $-(R^{100})_nR^{101}$, $R^{100}$ étant alkylèneoxy, $R^{101}$ étant alkyle, n étant un nombre entier de ≥ 1 ; chacun facultativement substitué,
$R^{36}$ et $R^{37}$ sont chacun indépendamment un groupe donneur d'électrons, et
$B^7$ et $B^8$ sont chacun indépendamment H ou un substituant.

**6.** Composition, qui est une composition photochromique comprenant un ou plusieurs composés photochromiques selon l'une quelconque des revendications 1 à 5.

**7.** Composition selon la revendication 6, qui contient un ou plusieurs composés photochromiques de formule (1) telle que définie dans la revendication 1 dans lesquels $R^{30}$ et $R^{31}$ sont tous deux éthyle, et $R^{37}$ est H, et un ou plusieurs composés photochromiques sélectionnés parmi des composés des formules (B) et (C) :

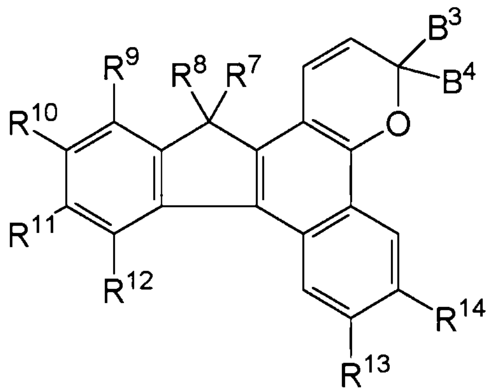

(B)

dans lesquelles $R^7$-$R^{12}$, $B^3$ et $B^4$ sont chacun indépendamment H ou un substituant, et $R^{13}$ et $R^{14}$ sont chacun indépendamment un groupe donneur d'électrons ; et

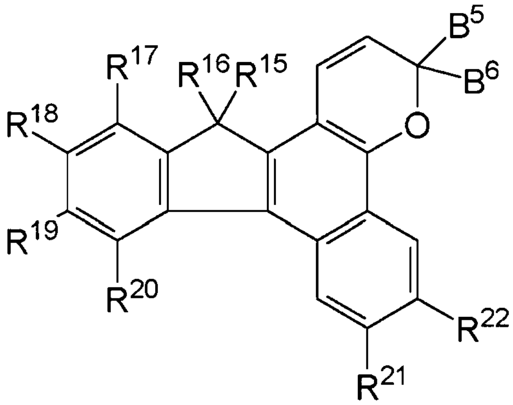

(C)

dans lesquelles $R^{15}$-$R^{20}$, $B^5$ et $B^5$ sont chacun indépendamment H ou un substituant, et l'un de $R^{21}$ et $R^{22}$ est H, et l'autre est un groupe donneur d'électrons.

**8.** Composition, qui est une composition photochromique comprenant un ou plusieurs composés photochromiques de formule (1b) :

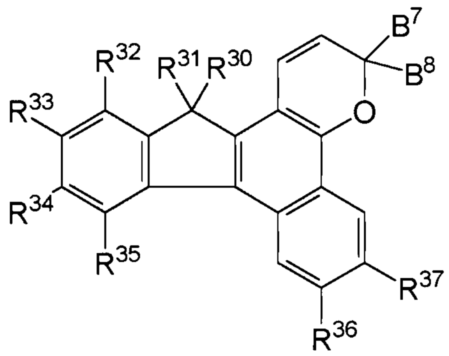

(1b)

dans laquelle $R^{30}$ et $R^{31}$ sont tous deux éthyle, $R^{32}$-$R^{35}$ sont chacun indépendamment H ou un substituant autre qu'un groupe attracteur d'électrons, et l'un de $R^{36}$ et $R^{37}$ est un groupe donneur d'électrons, et l'autre est H ou un groupe donneur d'électrons, et un ou plusieurs composés photochromiques de formule (A) :

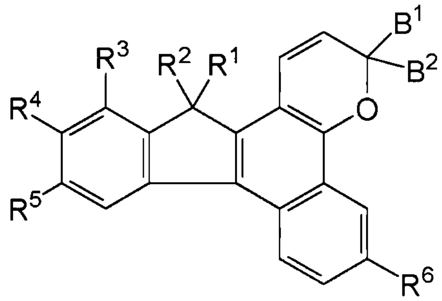

(A)

dans laquelle $R^1$-$R^6$, $B^1$ et $B^2$ sont chacun indépendamment H ou un substituant.

**9.** Composition selon l'une quelconque des revendications 6 à 8, comprenant en outre un composé polymérisable.

**10.** Article photochromique comprenant un produit durci obtenu par durcissement de la composition selon la revendication 9.

**11.** Article photochromique selon la revendication 10, comprenant un substrat et une couche photochromique qui est le produit durci.

**12.** Article photochromique selon la revendication 10 ou la revendication 11, qui est un verre de lunettes, un verre de lunettes de protection, une partie de visière d'un pare-soleil ou un élément de protection d'un casque.

**13.** Lunettes comprenant le verre de lunettes selon la revendication 12.

Fig. 1

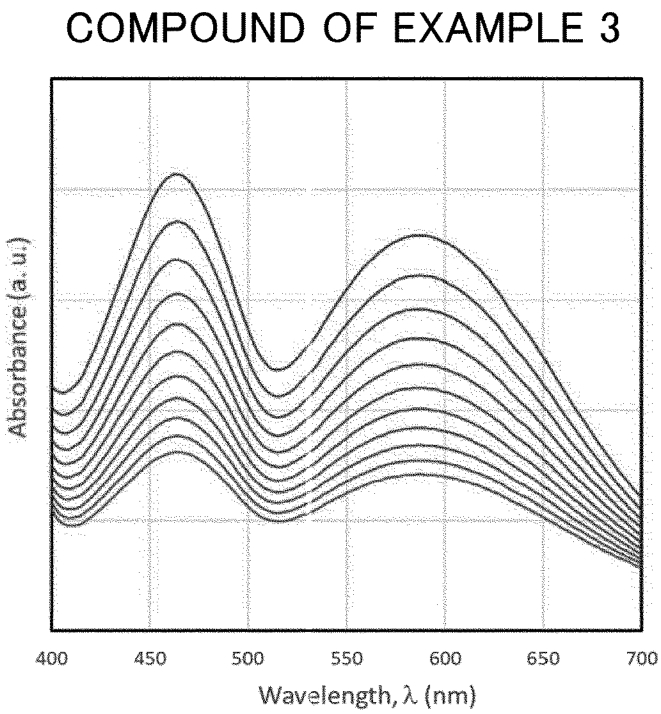
COMPOUND OF EXAMPLE 3
Absorbance (a. u.)
400
450
500
550
600
650
700
Wavelength, λ (nm)

Fig. 2

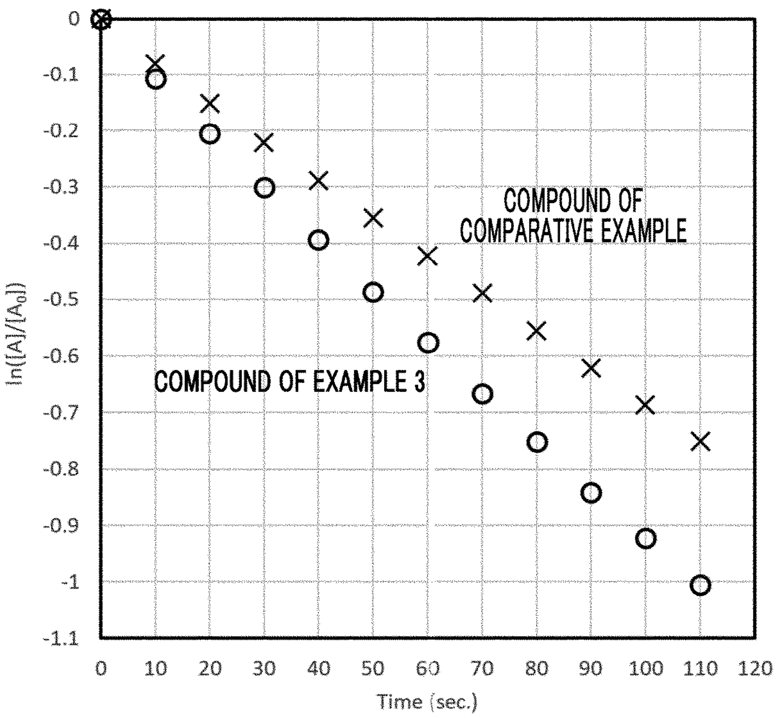
0
-0.1
-0.2
-0.3
-0.4
-0.5
-0.6
-0.7
-0.8
-0.9
-1
-1.1
ln([A]/[A0])
COMPOUND OF COMPARATIVE EXAMPLE
COMPOUND OF EXAMPLE 3
0
10
20
30
40
50
60
70
80
90
100
110
120
Time (sec.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200015631 A
- EP 2479171 A
- WO 2013078086 A
- JP 4884578 B
- US 20060226402 A
- US 20060228557 A
- US 20080103301 A
- US 20110108781 A
- US 7527754 B
- US 7556751 B
- WO 200160811 A
- WO 2013086248 A
- WO 1996014596 A
- WO 2001019813 A

### Non-patent literature cited in the description

- **SHU IWAMURA** ; **RYOJI NOYORI** ; **TAKESHI NAKAI** ; **ISAO KITAGAWA**. *Graduate School of Organic Chemistry (Part 1)*, 1988